# EUROPEAN PATENT APPLICATION

(11) **EP 4 603 583 A2**
(43) Date of publication of application: **20.08.2025**
(21) Application number: 25173019.8
(22) Date of filing: 29.08.2022
(51) Int. Cl.: C12N 15/10

(54) **TEMPLATE SWITCH OLIGONUCLEOTIDE (TSO) FOR MRNA 5' ANALYSIS**

(30) Priority: 30.08.2021 US 202163238748 P
(62) Divisional of application: 22789792.3
(71) Applicant: Becton, Dickinson and Company, Franklin Lakes, NJ 07417-1880 (US)
(72) Inventor: PROSEN, Dennis, San Jose, California 95131 (US); ACOB, Ricelle Agbayani, San Jose, California 95131 (US)
(74) Representative: Carpmaels & Ransford LLP

(57) **Abstract**

Disclosed herein include systems, methods, compositions, and kits for 5'-based gene expression profiling and for whole transcriptome analysis (WTA) with random priming and extension (RPE). There are provided, in some embodiments, template switch oligonucleotides (TSO) and methods of using which reduce the generation of undesirable extension products during library preparation. In some embodiments, the TSO comprises a blocking sequence. In some embodiments, the blocking sequence comprises a blocking nucleotide. Immune repertoire profiling methods are also provided in some embodiments.

## Description

### RELATED APPLICATIONS

This application claims the benefit under 35 U.S.C. §119(e) of U.S. Provisional Patent Application Ser. No. 63/238,748, filed August 30, 2021, the content of this related application is incorporated herein by reference in its entirety for all purposes.

### REFERENCE TO SEQUENCE LISTING

The present application is being filed along with a Sequence Listing in electronic format. The Sequence Listing is provided as a file entitled 68EB-317320-WO, created August 27, 2022, which is 24.0 kilobytes in size. The information in the electronic format of the Sequence Listing is incorporated herein by reference in its entirety.

### BACKGROUND

### Field

The present disclosure relates generally to the field of molecular biology, and for particular to multiomics analyses using molecular barcoding.

### Description of the Related Art

Methods and techniques of molecular barcoding are useful for single cell transcriptomics analysis, including deciphering gene expression profiles to determine the states of cells using, for example, reverse transcription, polymerase chain reaction (PCR) amplification, and next generation sequencing (NGS). Molecular barcoding is also useful for single cell proteomics analysis. There is a need for methods and techniques for molecular barcoding of nucleic acid targets on one or both the 5' ends and the 3' ends. There is a need for systems and methods that can quantitatively analyze gene expression of cells efficiently.

### SUMMARY

Disclosed herein include methods for labeling nucleic acid targets in a sample. In some embodiments, the method comprises: contacting copies of a nucleic acid target with a first plurality of oligonucleotide barcodes, wherein each oligonucleotide barcode comprises a first universal sequence, a molecular label, and a target-binding region capable of hybridizing to the nucleic acid target; extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region; hybridizing the complement of the target-binding region of each barcoded nucleic acid molecule with the target-binding region of an oligonucleotide barcode of the first plurality of oligonucleotide barcodes; and extending the 3' ends of oligonucleotide barcodes hybridized to the complement of the target-binding region of the barcoded nucleic acid molecule to generate a first plurality of extended barcoded nucleic acid molecules each comprising a complement of the first molecular label and a second molecular label. The method can comprise: determining the copy number of the nucleic acid target in the sample based on the number of second molecular labels with distinct sequences associated with the first plurality of extended barcoded nucleic acid molecules, or products thereof.

Disclosed herein include methods for determining the numbers of nucleic acid targets in a sample. In some embodiments, the method comprises: contacting copies of a nucleic acid target with a first plurality of oligonucleotide barcodes, wherein each oligonucleotide barcode comprises a first universal sequence, a molecular label, and a target-binding region capable of hybridizing to the nucleic acid target; extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region; hybridizing the complement of the target-binding region of each barcoded nucleic acid molecule with the target-binding region of an oligonucleotide barcode of the first plurality of oligonucleotide barcodes; extending the 3' ends of the oligonucleotide barcodes hybridized to the complement of the target-binding region of the barcoded nucleic acid molecule to generate a first plurality of extended barcoded nucleic acid molecules each comprising a complement of the first molecular label and a second molecular label; and determining the copy number of the nucleic acid target in the sample based on the number of second molecular labels with distinct sequences associated with the first plurality of extended barcoded nucleic acid molecules, or products thereof.

The method can comprise: amplifying the first plurality of extended barcoded nucleic acid molecules to generate a first plurality of single-labeled nucleic acid molecules each comprising the second molecular label, wherein determining the copy number of the nucleic acid target in the sample comprises: determining the copy number of the nucleic acid target in the sample based on the number of second molecular labels with distinct sequences associated with the first plurality of single-labeled nucleic acid molecules.

Disclosed herein include methods for labeling nucleic acid targets in a sample. In some embodiments, the method comprises: contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target; extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target; contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence; and extending: the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence. The method can comprise: determining the copy number of the nucleic acid target in the sample based on: the number of first molecular labels with distinct sequences, second molecular labels with distinct sequences, or a combination thereof, associated with the second plurality of extended barcoded nucleic acid molecules, or products thereof.

Disclosed herein include methods for the copy number of a nucleic acid target in a sample. In some embodiments, the method comprises: contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target; extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target; contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence; extending: the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence; and determining the copy number of the nucleic acid target in the sample based on: the number of first molecular labels with distinct sequences, second molecular labels with distinct sequences, or a combination thereof, associated with the second plurality of extended barcoded nucleic acid molecules, or products thereof.

In some embodiments, determining the copy number of the nucleic acid target comprises determining the copy number of each of a plurality of nucleic acid targets in the sample based on the number of first molecular labels with distinct sequences, second molecular labels with distinct sequences, or a combination thereof, associated with extended barcoded nucleic acid molecules of the second plurality of extended barcoded nucleic acid molecules comprising a sequence of the each of the plurality of nucleic acid targets. In some embodiments, the first universal sequence of each oligonucleotide barcode of the first plurality of oligonucleotide barcodes is 5' of the first molecular label and the target-binding region. In some embodiments, the second universal sequence of each oligonucleotide barcode of the second plurality of oligonucleotide barcodes is 5' of the second molecular label and the bait sequence. In some embodiments, the bait sequence comprises at least 6 nucleotides. In some embodiments, the bait sequence comprises a GC content of about 20% to about 80%.

The method can comprise: amplifying the second plurality of extended barcoded nucleic acid molecules using an amplification primer and a primer comprising the second universal sequence, or a portion thereof, thereby generating a second plurality of single-labeled nucleic acid molecules comprising the sequence of the nucleic acid target, or a portion thereof, wherein determining the copy number of the nucleic acid target in the sample comprises: determining the copy number of the nucleic acid target in the sample based on the number of second molecular labels with distinct sequences associated with the second plurality of single-labeled nucleic acid molecules, or products thereof.

The method can comprise: amplifying the second plurality of extended barcoded nucleic acid molecules using an amplification primer and a primer comprising the first universal sequence, or a portion thereof, thereby generating a third plurality of single-labeled nucleic acid molecules comprising the sequence of the nucleic acid target, or a portion thereof, wherein determining the copy number of the nucleic acid target in the sample comprises: determining the copy number of the nucleic acid target in the sample based on the number of first molecular labels with distinct sequences associated with the third plurality of single-labeled nucleic acid molecules, or products thereof.

In some embodiments, the oligonucleotide barcodes of the first and/or second pluralities of oligonucleotide barcodes comprise a cell label, optionally the cell label is located 5' of the molecular label. In some embodiments, at least a portion of the oligonucleotide barcode the first and/or second pluralities of oligonucleotide barcodes comprises at least one variable sequence, optionally at least 10 of the first and/or second pluralities of oligonucleotide barcodes comprise different sequences at the invariable sequence, optionally at least a portion of the cell label and/or molecular label comprises a variable sequence. In some embodiments, the blocking sequence is located at the 3' end of the first and/or second pluralities of barcoded nucleic acid molecules. In some embodiments, the blocking sequence is configured to be non-complementary to: (i) the region of the oligonucleotide barcode of the first plurality of oligonucleotide barcodes 5' of the target-binding region; and/or (ii) the region of the oligonucleotide barcode of the second plurality of oligonucleotide barcodes 5' of the bait sequence. In some embodiments, the blocking sequence is less than about 50% complementary to: (i) the region of at least 50% of oligonucleotide barcodes of the first plurality of oligonucleotide barcodes 5' of the target-binding region; and/or (ii) the region of at least 50% of the oligonucleotide barcodes of the second plurality of oligonucleotide barcodes 5' of the bait sequence. In some embodiments, the blocking sequence is configured to prevent extension of the 3' ends of the first and/or second pluralities of barcoded nucleic acid molecules. In some embodiments, the blocking sequence reduces the generation of extended barcoded nucleic acid molecules comprising a complement of the first universal sequence.

In some embodiments, in the absence of the blocking sequence, (i) the 3' ends of the second plurality of barcoded nucleic acid molecules hybridized to the bait sequence of the second plurality of oligonucleotide barcodes; and/or (ii) the 3' ends of the first plurality of barcoded nucleic acid molecules hybridized to the target-binding region of the first plurality of oligonucleotide barcodes; are capable of being extended to generate a third plurality of extended barcoded nucleic acid molecules. In some embodiments, the generation of the third plurality of extended barcoded nucleic acid molecules is reduced by at least 10%, by at least 25%, by at least 50%, by at least 80%. by at least 90%, by at least 95%, or by at least 99%, as compared to a comparable method wherein the TSO does not comprise a complement of a blocking sequence.

In some embodiments, at least a portion of an oligonucleotide barcode of the first and/or second pluralities of oligonucleotide barcodes comprises an invariable sequence, optionally each of the first and/or second pluralities of oligonucleotide barcodes comprise the same sequence at the invariable sequence. In some embodiments, the invariable sequence is between about 1-15 nucleotides in length, optionally the invariable sequence is 1 nucleotide in length. In some embodiments, the invariable sequence is located in the cell label, optionally the cell label comprises a first portion of the cell label, a first linker, a second portion of the cell label, a second linker, and a third portion of the cell label, further optionally the invariable sequence is located in the third portion of the cell label. In some embodiments, the blocking sequence comprises a blocker nucleotide, wherein the blocker nucleotide is located at the 3' end of the blocking sequence. In some embodiments, the blocker nucleotide is configured to be non-complementary to the invariable sequence. In some embodiments, the distance between the invariable sequence and the bait sequence is equidistant to the distance between the complement of the bait sequence and the blocker nucleotide. In some embodiments, the distance between the invariable sequence and the target-binding region is equidistant to the distance between the complement of the target-binding region and the blocker nucleotide. In some embodiments, the TSO comprises a sequence selected from SEQ ID NOs: 1-7, or a sequence that exhibits at least about 50% identity to a sequence selected from SEQ ID NOs: 1-7. In some embodiments, the blocking sequence comprises at least 4 contiguous bases of any one of SEQ ID NOs: 1-7. In some embodiments, the complement of the blocking sequence is 5' of the bait sequence in the TSO. In some embodiments, the complement of the blocking sequence is 5' of the target-binding region, or portion thereof, in the TSO.

In some embodiments, Template Switch Efficiency is reduced less than 40%, less than 20%, less than 10%, or less than 5%, as compared to a comparable method wherein the TSO does not comprise a complement of a blocking sequence. In some embodiments, the method further comprises the addition of blocker oligonucleotides before or during one or more extension steps. In some embodiments, the method does not comprise the addition of blocker oligonucleotides. In some embodiments, the complement of the blocking sequence comprises a reverse complementary sequence of the blocking sequence, and/or a complementary sequence of the blocking sequence.

In some embodiments, hybridizing the complement of the target-binding region of a barcoded nucleic acid molecule with the target-binding region of an oligonucleotide barcode of the first plurality of oligonucleotide barcodes comprises intermolecular hybridization of the complement of the target-binding region of a barcoded nucleic acid molecule with the target-binding region of an oligonucleotide barcode of the first plurality of oligonucleotide barcodes. The method can comprise: denaturing the first plurality of barcoded nucleic acid molecules prior to hybridizing the complement of the target-binding region of each barcoded nucleic acid molecule with the target-binding region of an oligonucleotide barcode of the first plurality of oligonucleotide barcodes. The method can comprise: denaturing the first and/or second plurality of extended barcoded nucleic acid molecules prior to amplifying the first and/or second plurality of extended barcoded nucleic acid molecules.

In some embodiments, determining the copy number of the nucleic acid target comprises determining the copy number of each of the plurality of nucleic acid targets in the sample based on the number of second molecular labels with distinct sequences associated with single-labeled nucleic acid molecules of the first plurality of single-labeled nucleic acid molecules comprising a sequence of the each of the plurality of nucleic acid targets. In some embodiments, the sequence of the each of the plurality of nucleic acid targets comprises a subsequence of the each of the plurality of nucleic acid targets. In some embodiments, the sequence of the nucleic acid target in the first plurality of barcoded nucleic acid molecules and/or second plurality of barcoded nucleic acid molecules comprises a subsequence of the nucleic acid target.

In some embodiments, the complement of the target-binding region is complementary to a portion of the target-binding region. In some embodiments, the target-binding region comprises a gene-specific sequence, and/or a poly(dT) sequence. In some embodiments, the second molecular label is a different from the first molecular label, and wherein the second molecular label is not a complement of the first molecular label. In some embodiments, the first and/or second plurality of extended barcoded nucleic acid molecules each comprise the sequence of the nucleic acid target. In some embodiments, the nucleic acid target comprises mRNA, and wherein the first and/or second plurality of extended barcoded nucleic acid molecules each comprise the sequence of the sense strand of the nucleic acid target. In some embodiments, the reverse transcriptase is capable of terminal transferase activity. In some embodiments, the template switch oligonucleotide comprises one or more 3' ribonucleotides, optionally three 3' ribonucleotides, and further optionally the 3' ribonucleotides comprise guanine. In some embodiments, the reverse transcriptase comprises a viral reverse transcriptase, optionally the viral reverse transcriptase is a murine leukemia virus (MLV) reverse transcriptase or a Moloney murine leukemia virus (MMLV) reverse transcriptase.

The sample can comprise a single cell, for example an immune cell, and further optionally a B cell or a T cell. In some embodiments, the sample comprises a plurality of cells, a plurality of single cells, a tissue, a tumor sample, or any combination thereof. In some embodiments, the single cell comprises a circulating tumor cell. In some embodiments, the first universal sequence is 5' of the molecular label and the target-binding region. In some embodiments, amplifying the first plurality of extended barcoded nucleic acid molecules to generate a first plurality of single-labeled nucleic acid molecules comprises using a primer capable of hybridizing to the first universal sequence, and an amplification primer.

In some embodiments, the amplification primer is a target-specific primer, and optionally the target-specific primer specifically hybridizes to an immune receptor, a constant region of an immune receptor, a variable region of an immune receptor, a diversity region of an immune receptor, and/or the junction of a variable region and diversity region of an immune receptor. The immune receptor can be a T cell receptor (TCR) and/or a B cell receptor (BCR) receptor, and optionally the TCR comprises TCR alpha chain, TCR beta chain, TCR gamma chain, TCR delta chain, or any combination thereof; and the BCR receptor comprises BCR heavy chain and/or BCR light chain. In some embodiments, the amplification primer specifically binds the extended barcoded nucleic acid molecules each comprising a complement of the first molecular label and a second molecular label. In some embodiments, the amplification primer does not bind extended barcoded nucleic acid molecules comprising the first molecular label. In some embodiments, the amplification primer comprises the complement of the nucleic acid target. In some embodiments, the nucleic acid target comprises mRNA, and wherein the amplification primer comprises the sequence of the anti-sense strand of the nucleic acid target. In some embodiments, extending the 3' ends of the oligonucleotide barcodes comprises extending the 3' ends of the oligonucleotide barcodes using a mesophilic DNA polymerase, a thermophilic DNA polymerase, a psychrophilic DNA polymerase, or any combination thereof. In some embodiments, extending the 3' ends of the oligonucleotide barcodes comprises extending the 3' ends of the oligonucleotide barcodes using a DNA polymerase lacking at least one of 5' to 3' exonuclease activity and 3' to 5' exonuclease activity, and optionally the DNA polymerase comprises a Klenow Fragment.

The method can comprise: obtaining sequence information of the first and/or second plurality of extended barcoded nucleic acid molecules, or products thereof. The method can comprise: obtaining sequence information of one or more of the first, second, and third pluralities of single-labeled nucleic acid molecules, or products thereof, In some embodiments, obtaining the sequence information comprises attaching sequencing adaptors to the first and/or second plurality of extended barcoded nucleic acid molecules, or products thereof. In some embodiments, obtaining the sequence information comprises attaching sequencing adaptors to the first, second, and/or third pluralities of single-labeled nucleic acid molecules, or products thereof. In some embodiments, obtaining sequence information comprises: obtaining sequencing data comprising a plurality of sequencing reads of one or more of the first and/or second plurality of extended barcoded nucleic acid molecules, or products thereof, and/or the first, second, and third pluralities of single-labeled nucleic acid molecules, or products thereof, wherein each of the plurality of sequencing reads comprise (1) a cell label sequence, (2) a molecular label sequence, and/or (3) a subsequence of the nucleic acid target. In some embodiments, less than 40%, less than 20%, less than 10%, or less than 5%, of the total sequencing reads are derived from the third plurality of extended barcoded nucleic acid molecules, or products thereof. In some embodiments, the number of sequencing reads derived from the third plurality of extended barcoded nucleic acid molecules, or products thereof, is reduced at least about 2-fold as compared to a comparable method wherein the TSO does not comprise a complement of a blocking sequence.

In some embodiments, obtaining the sequence information comprises obtaining the sequence information of the BCR light chain and the BCR heavy chain of a single cell. In some embodiments, the sequence information of the BCR light chain and the BCR heavy chain comprises the sequence of the complementarity determining region 1 (CDR1), the CDR2, the CDR3, or any combination thereof, of the BCR light chain and/or the BCR heavy chain. The method can comprise: pairing the BCR light chain and the BCR heavy chain of the single cell based on the obtained sequence information. In some embodiments, the sample comprises a plurality of single cells, the method comprising pairing the BCR light chain and the BCR heavy chain of at least 50% of said single cells based on the obtained sequence information.

In some embodiments, obtaining the sequence information comprises obtaining the sequence information of the TCR alpha chain and the TCR beta chain of a single cell. In some embodiments, the sequence information of the TCR alpha chain and the TCR beta chain comprises the sequence of the complementarity determining region 1 (CDR1), the CDR2, the CDR3, or any combination thereof, of the TCR alpha chain and/or the TCR beta chain. The method can comprise: pairing the TCR alpha chain and the TCR beta chain of the single cell based on the obtained sequence information. In some embodiments, the sample comprises a plurality of single cells, the method comprising pairing the TCR alpha chain and the TCR beta chain of at least 50% of said single cells based on the obtained sequence information.

Obtaining the sequence information can comprise obtaining the sequence information of the TCR gamma chain and the TCR delta chain of a single cell. In some embodiments, the sequence information of the TCR gamma chain and the TCR delta chain comprises the sequence of the complementarity determining region 1 (CDR1), the CDR2, the CDR3, or any combination thereof, of the TCR gamma chain and/or the TCR delta chain. The method can comprise: pairing the TCR gamma chain and the TCR delta chain of the single cell based on the obtained sequence information. In some embodiments, the sample comprises a plurality of single cells, the method comprising pairing the TCR gamma chain and the TCR delta chain of at least 50% of said single cells based on the obtained sequence information.

In some embodiments, the complement of the target-binding region comprises the reverse complementary sequence of the target-binding region and/or the complementary sequence of the target-binding region. In some embodiments, the complement of the molecular label comprises a reverse complementary sequence of the molecular label, and/or a complementary sequence of the molecular label. In some embodiments, the first and/or second plurality of barcoded nucleic acid molecules comprises barcoded deoxyribonucleic acid (DNA) molecules and/or barcoded ribonucleic acid (RNA) molecules. In some embodiments, the target-binding region comprises an oligo dT sequence, a random sequence, a target-specific sequence, or a combination thereof. In some embodiments, the target-binding region comprises a poly(dT) region, and wherein the nucleic acid target comprises a poly(dA) region. In some embodiments, the sample comprises a plurality of cells, a plurality of single cells, a tissue, a tumor sample, or any combination thereof. In some embodiments, the nucleic acid target comprises a nucleic acid molecule, and optionally the nucleic acid molecule comprises ribonucleic acid (RNA), messenger RNA (mRNA), microRNA, small interfering RNA (siRNA), RNA degradation product, RNA comprising a poly(A) tail, or any combination thereof. In some embodiments, the mRNA encodes an immune receptor. In some embodiments, the nucleic acid target comprises a cellular component binding reagent. In some embodiments, the nucleic acid molecule is associated with the cellular component binding reagent. The method can comprise: dissociating the nucleic acid molecule and the cellular component binding reagent.

In some embodiments, at least 10 of the first plurality of oligonucleotide barcodes comprise different molecular label sequences. In some embodiments, each molecular label of the plurality of oligonucleotide barcodes comprises at least 6 nucleotides. In some embodiments, the first plurality of oligonucleotide barcodes are associated with a solid support, and optionally the first plurality of oligonucleotide barcodes associated with the same solid support each comprise an identical sample label, and further optionally each sample label of the first plurality of oligonucleotide barcodes comprises at least 6 nucleotides. In some embodiments, the first plurality of oligonucleotide barcodes each comprise a cell label, and optionally each cell label of the first plurality of oligonucleotide barcodes comprises at least 6 nucleotides. In some embodiments, oligonucleotide barcodes associated with the same solid support comprise the same cell label. In some embodiments, oligonucleotide barcodes associated with different solid supports comprise different cell labels. In some embodiments, the first and/or second plurality of extended barcoded nucleic acid molecules each comprises a cell label and a complement of the cell label, and optionally the complement of the cell label comprises a reverse complementary sequence of the cell label and/or a complementary sequence of the cell label.

The method can comprise: extending the first plurality of oligonucleotide barcodes hybridized to the copies of the nucleic acid target in the presence of one or more of ethylene glycol, polyethylene glycol, 1,2- propanediol, dimethyl sulfoxide (DMSO), glycerol, formamide, 7-deaza-GTP, acetamide, tetramethylammonium chloride salt, betaine, or any combination thereof. In some embodiments, at least 10 of the first and second pluralities of oligonucleotide barcodes comprise different molecular label sequences, optionally each molecular label of the first and second pluralities of oligonucleotide barcodes comprises at least 6 nucleotides. In some embodiments, the first and second pluralities of oligonucleotide barcodes are associated with a solid support. In some embodiments, the first and second pluralities of oligonucleotide barcodes associated with the same solid support each comprise an identical sample label, optionally each sample label of the first and second pluralities of oligonucleotide barcodes comprises at least 6 nucleotides. In some embodiments, the first and second pluralities of oligonucleotide barcodes each comprise a cell label, optionally each cell label of the first and second pluralities of oligonucleotide barcodes comprises at least 6 nucleotides. In some embodiments, (a) oligonucleotide barcodes of the first and second pluralities of oligonucleotide barcodes associated with the same solid support comprise the same cell label; and/or (b) oligonucleotide barcodes of the first and second pluralities of oligonucleotide barcodes associated with different solid supports comprise different cell labels.

In some embodiments, the extended barcoded nucleic acid molecules of the first and second pluralities of extended barcoded nucleic acid molecules each comprises a cell label and a complement of the cell label, optionally the complement of the cell label comprises a reverse complementary sequence of the cell label, or a complementary sequence of the cell label.. In some embodiments, at least one oligonucleotide barcode of the first and second pluralities of oligonucleotide barcodes is immobilized or partially immobilized on the synthetic particle, or at least one oligonucleotide barcode of the first and second pluralities of oligonucleotide barcodes is enclosed or partially enclosed in the synthetic particle. In some embodiments, the solid support comprises a synthetic particle or a planar surface.

In some embodiments, the sample comprises a single cell, the method comprising associating a synthetic particle comprising the plurality of the oligonucleotide barcodes with the single cell in the sample. The method can comprise: lysing the single cell after associating the synthetic particle with the single cell, and optionally lysing the single cell comprises heating the sample, contacting the sample with a detergent, changing the pH of the sample, or any combination thereof. In some embodiments, the synthetic particle and the single cell are in the same well. In some embodiments, the synthetic particle and the single cell are in the same droplet. In some embodiments, at least one of the first plurality of oligonucleotide barcodes is immobilized or partially immobilized on the synthetic particle, or the at least one of the first plurality of oligonucleotide barcodes is enclosed or partially enclosed in the synthetic particle.

The synthetic particle can be disruptable. In some embodiments, the synthetic particle comprises a bead, for example a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof; a material selected from polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof; and a disruptable hydrogel particle.

In some embodiments, each oligonucleotide barcode of the first and second pluralities of oligonucleotide barcodes comprises a linker functional group, the synthetic particle comprises a solid support functional group, and the support functional group and the linker functional group are associated with each other, and optionally the linker functional group and the support functional group are individually selected from C6, biotin, streptavidin, primary amine(s), aldehyde(s), ketone(s), and any combination thereof. In some embodiments, each of the first plurality of oligonucleotide barcodes comprises a linker functional group, the synthetic particle comprises a solid support functional group, and the support functional group and the linker functional group are associated with each other, and optionally the linker functional group and the support functional group are individually selected from C6, biotin, streptavidin, primary amine(s), aldehyde(s), ketone(s), and any combination thereof.

Disclosed herein include kits. In some embodiments, the kit comprises: a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences; a reverse transcriptase; a template switch oligonucleotide (TSO) comprising (i) a complement of a blocking sequence and (ii) the target-binding region, or a portion thereof; and/or a DNA polymerase lacking at least one of 5' to 3' exonuclease activity and 3' to 5' exonuclease activity.

Disclosed herein include kits. In some embodiments, the kit comprises: a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences; a second plurality of oligonucleotide barcodes, wherein each oligonucleotide barcode of the second plurality of oligonucleotide barcodes comprises a second universal sequence, a second molecular label, and a bait sequence, wherein at least 10 of the second plurality of oligonucleotide barcodes comprise different molecular label sequences; a template switch oligonucleotide (TSO) comprising (i) a complement of a blocking sequence and (ii) a bait sequence; a DNA polymerase lacking at least one of 5' to 3' exonuclease activity and 3' to 5' exonuclease activity; and/or a reverse transcriptase.

In some embodiments, the oligonucleotide barcodes of the first and/or second pluralities of oligonucleotide barcodes comprise a cell label, optionally the cell label is located 5' of the molecular label. In some embodiments, at least a portion of the oligonucleotide barcode the first and/or second pluralities of oligonucleotide barcodes comprises at least one variable sequence, optionally at least 10 of the first and/or second pluralities of oligonucleotide barcodes comprise different sequences at the invariable sequence, optionally at least a portion of the cell label and/or molecular label comprises a variable sequence.

In some embodiments, the blocking sequence is located at the 3' end of the first and/or second pluralities of barcoded nucleic acid molecules. In some embodiments, the blocking sequence is configured to be non-complementary to: (i) the region of the oligonucleotide barcode of the first plurality of oligonucleotide barcodes 5' of the target-binding region; and/or (ii) the region of the oligonucleotide barcode of the second plurality of oligonucleotide barcodes 5' of the bait sequence. In some embodiments, the blocking sequence is less than about 50% complementary to: (i) the region of at least 50% of oligonucleotide barcodes of the first plurality of oligonucleotide barcodes 5' of the target-binding region; and/or (ii) the region of at least 50% of the oligonucleotide barcodes of the second plurality of oligonucleotide barcodes 5' of the bait sequence.

In some embodiments, at least a portion of an oligonucleotide barcode of the first and/or second pluralities of oligonucleotide barcodes comprises an invariable sequence, optionally each of the first and/or second pluralities of oligonucleotide barcodes comprise the same sequence at the invariable sequence. In some embodiments, the invariable sequence is between about 1-15 nucleotides in length, optionally the invariable sequence is 1 nucleotide in length. In some embodiments, the invariable sequence is located in the cell label, optionally the cell label comprises a first portion of the cell label, a first linker, a second portion of the cell label, a second linker, and a third portion of the cell label, further optionally the invariable sequence is located in the third portion of the cell label. In some embodiments, the blocking sequence comprises a blocker nucleotide, wherein the blocker nucleotide is located at the 3' end of the blocking sequence. In some embodiments, the blocker nucleotide is configured to be non-complementary to the invariable sequence. In some embodiments, the distance between the invariable sequence and the bait sequence is equidistant to the distance between the complement of the bait sequence and the blocker nucleotide. In some embodiments, the distance between the invariable sequence and the target-binding region is equidistant to the distance between the complement of the target-binding region and the blocker nucleotide.

**In** some embodiments, the TSO comprises a sequence selected from SEQ ID NOs: 1-7, or a sequence that exhibits at least about 50% identity to a sequence selected from SEQ ID NOs: 1-7. In some embodiments, the blocking sequence comprises at least 4 contiguous bases of any one of SEQ ID NOs: 1-7. In some embodiments, the complement of the blocking sequence is 5' of the bait sequence in the TSO. In some embodiments, the complement of the blocking sequence is 5' of the target-binding region, or portion thereof, in the TSO. In some embodiments, the complement of the blocking sequence comprises a reverse complementary sequence of the blocking sequence, and/or a complementary sequence of the blocking sequence.

In some embodiments, the DNA polymerase lacking at least one of 5' to 3' exonuclease activity and 3' to 5' exonuclease activity comprises a mesophilic DNA polymerase, a thermophilic DNA polymerase, a psychrophilic DNA polymerase, or any combination thereof. In some embodiments, the DNA polymerase comprises a Klenow Fragment. In some embodiments, the reverse transcriptase comprises a viral reverse transcriptase, optionally a murine leukemia virus (MLV) reverse transcriptase or a Moloney murine leukemia virus (MMLV) reverse transcriptase. In some embodiments, the template switch oligonucleotide comprises one or more 3' ribonucleotides, optionally three 3' ribonucleotides, and further optionally the 3' ribonucleotides comprise guanine. The kit can comprise: one or more of ethylene glycol, polyethylene glycol, 1,2- propanediol, dimethyl sulfoxide (DMSO), glycerol, formamide, 7-deaza-GTP, acetamide, tetramethylammonium chloride salt, betaine, or any combination thereof. The kit can comprise: a buffer, a cartridge, one or more reagents for a reverse transcription reaction, one or more reagents for an amplification reaction, or a combination thereof. In some embodiments, the target-binding region comprises a gene-specific sequence, an oligo(dT) sequence, a random multimer, or any combination thereof.

### BRIEF DESCRIPTION OF THE DRAWINGS

FIG. 1 illustrates a non-limiting exemplary barcode.
FIG. 2 shows a non-limiting exemplary workflow of barcoding and digital counting.
FIG. 3 is a schematic illustration showing a non-limiting exemplary process for generating an indexed library of targets barcoded at the 3'-ends from a plurality of targets.
FIGS. 4A-4E show schematic illustrations of non-limiting exemplary workflows of determining the sequences of a nucleic acid target (e.g., the V(D)J region of an immune receptor) using 5' barcoding and the template switching oligonucleotides disclosed herein.
FIG. 5 shows non-limiting exemplary workflows for the determination of the sequences of a nucleic acid target (e.g., the V(D)J region of an immune receptor) using the compositions and methods provided herein.
FIG. 6 depicts a non-limiting schematic illustration of a library preparation method employing the template switch oligonucleotide TSO1 (TATGCGTAGTAGGTATG rGrGrG; SEQ ID NO: 1).
FIG. 7 depicts a non-limiting schematic illustration of a library preparation method employing the template switch oligonucleotide TSO21 (GTGGAGTCGTTATGCGTAGTAGGTATG rGrGrG; SEQ ID NO: 4).
FIG. 8 depicts a non-limiting schematic illustration of a library preparation method employing the template switch oligonucleotide TSO t21 (TGTGGAGTCGTTATGCGTAGTAGGTATG rGrGrG; SEQ ID NO: 5).
FIG. 9 depicts a non-limiting schematic illustration of a library preparation method employing the template switch oligonucleotide TSO12 (TATGCGTAGTAGGTATGGTGGAGTCGT rGrGrG; SEQ ID NO: 3).
FIG. 10 depicts a non-limiting schematic illustration of a library preparation method employing the template switch oligonucleotide TSO t2dT₂₅ (TGTGGAGTCGTTTTTTTTTTTTTTTTTTTTTTTTTT rGrGrG; SEQ ID NO: 6).
FIG. 11 depicts a non-limiting schematic illustration of a library preparation method employing the template switch oligonucleotide TSO1 (TATGCGTAGTAGGTATG rGrGrG; SEQ ID NO: 1).
FIG. 12 depicts a non-limiting schematic illustration of a library preparation method employing the template switch oligonucleotide TSO12 (TATGCGTAGTAGGTATGGTGGAGTCGT rGrGrG; SEQ ID NO: 3).
FIG. 13 depicts a non-limiting schematic illustration of a library preparation method employing the template switch oligonucleotide TSO21 (GTGGAGTCGTTATGCGTAGTAGGTATG rGrGrG; SEQ ID NO: 4).
FIG. 14 depicts a non-limiting schematic illustration of a library preparation method employing the template switch oligonucleotide TSO t21 (TGTGGAGTCGTTATGCGTAGTAGGTATG rGrGrG; SEQ ID NO: 5)
FIG. 15 depicts a non-limiting schematic illustration of a library preparation method employing the template switch oligonucleotide TSO t2dT₂₅ (TGTGGAGTCGTTTTTTTTTTTTTTTTTTTTTTTTTT rGrGrG; SEQ ID NO: 6).

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

All patents, published patent applications, other publications, and sequences from GenBank, and other databases referred to herein are incorporated by reference in their entirety with respect to the related technology.

Quantifying small numbers of nucleic acids, for example messenger ribonucleotide acid (mRNA) molecules, is clinically important for determining, for example, the genes that are expressed in a cell at different stages of development or under different environmental conditions. However, it can also be very challenging to determine the absolute number of nucleic acid molecules (e.g., mRNA molecules), especially when the number of molecules is very small. One method to determine the absolute number of molecules in a sample is digital polymerase chain reaction (PCR). Ideally, PCR produces an identical copy of a molecule at each cycle. However, PCR can have disadvantages such that each molecule replicates with a stochastic probability, and this probability varies by PCR cycle and gene sequence, resulting in amplification bias and inaccurate gene expression measurements. Stochastic barcodes with unique molecular labels (also referred to as molecular indexes (MIs)) can be used to count the number of molecules and correct for amplification bias. Stochastic barcoding, such as the Precise^{™} assay (Cellular Research, Inc. (Palo Alto, CA)) and Rhapsody^{™} assay (Becton, Dickinson and Company (Franklin Lakes, NJ)), can correct for bias induced by PCR and library preparation steps by using molecular labels (MLs) to label mRNAs during reverse transcription (RT).

The Precise^{™} assay can utilize a non-depleting pool of stochastic barcodes with large number, for example 6561 to 65536, unique molecular label sequences on poly(T) oligonucleotides to hybridize to all poly(A)-mRNAs in a sample during the RT step. A stochastic barcode can comprise a universal PCR priming site. During RT, target gene molecules react randomly with stochastic barcodes. Each target molecule can hybridize to a stochastic barcode resulting to generate stochastically barcoded complementary ribonucleotide acid (cDNA) molecules). After labeling, stochastically barcoded cDNA molecules from microwells of a microwell plate can be pooled into a single tube for PCR amplification and sequencing. Raw sequencing data can be analyzed to produce the number of reads, the number of stochastic barcodes with unique molecular label sequences, and the numbers of mRNA molecules.

Disclosed herein include methods for labeling nucleic acid targets in a sample. In some embodiments, the method comprises: contacting copies of a nucleic acid target with a first plurality of oligonucleotide barcodes, wherein each oligonucleotide barcode comprises a first universal sequence, a molecular label, and a target-binding region capable of hybridizing to the nucleic acid target; extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region; hybridizing the complement of the target-binding region of each barcoded nucleic acid molecule with the target-binding region of an oligonucleotide barcode of the first plurality of oligonucleotide barcodes; and extending the 3' ends of oligonucleotide barcodes hybridized to the complement of the target-binding region of the barcoded nucleic acid molecule to generate a first plurality of extended barcoded nucleic acid molecules each comprising a complement of the first molecular label and a second molecular label. The method can comprise: determining the copy number of the nucleic acid target in the sample based on the number of second molecular labels with distinct sequences associated with the first plurality of extended barcoded nucleic acid molecules, or products thereof.

Disclosed herein include methods for determining the numbers of nucleic acid targets in a sample. In some embodiments, the method comprises: contacting copies of a nucleic acid target with a first plurality of oligonucleotide barcodes, wherein each oligonucleotide barcode comprises a first universal sequence, a molecular label, and a target-binding region capable of hybridizing to the nucleic acid target; extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region; hybridizing the complement of the target-binding region of each barcoded nucleic acid molecule with the target-binding region of an oligonucleotide barcode of the first plurality of oligonucleotide barcodes; extending the 3' ends of the oligonucleotide barcodes hybridized to the complement of the target-binding region of the barcoded nucleic acid molecule to generate a first plurality of extended barcoded nucleic acid molecules each comprising a complement of the first molecular label and a second molecular label; and determining the copy number of the nucleic acid target in the sample based on the number of second molecular labels with distinct sequences associated with the first plurality of extended barcoded nucleic acid molecules, or products thereof.

The method can comprise: amplifying the first plurality of extended barcoded nucleic acid molecules to generate a first plurality of single-labeled nucleic acid molecules each comprising the second molecular label, wherein determining the copy number of the nucleic acid target in the sample comprises: determining the copy number of the nucleic acid target in the sample based on the number of second molecular labels with distinct sequences associated with the first plurality of single-labeled nucleic acid molecules.

Disclosed herein include methods for labeling nucleic acid targets in a sample. In some embodiments, the method comprises: contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target; extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target; contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence; and extending: the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence. The method can comprise: determining the copy number of the nucleic acid target in the sample based on: the number of first molecular labels with distinct sequences, second molecular labels with distinct sequences, or a combination thereof, associated with the second plurality of extended barcoded nucleic acid molecules, or products thereof.

Disclosed herein include methods for the copy number of a nucleic acid target in a sample. In some embodiments, the method comprises: contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target; extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target; contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence; extending: the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence; and determining the copy number of the nucleic acid target in the sample based on: the number of first molecular labels with distinct sequences, second molecular labels with distinct sequences, or a combination thereof, associated with the second plurality of extended barcoded nucleic acid molecules, or products thereof.

Disclosed herein include kits. In some embodiments, the kit comprises: a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences; a reverse transcriptase; a template switch oligonucleotide (TSO) comprising (i) a complement of a blocking sequence and (ii) the target-binding region, or a portion thereof; and/or a DNA polymerase lacking at least one of 5' to 3' exonuclease activity and 3' to 5' exonuclease activity.

Disclosed herein include kits. In some embodiments, the kit comprises: a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences; a second plurality of oligonucleotide barcodes, wherein each oligonucleotide barcode of the second plurality of oligonucleotide barcodes comprises a second universal sequence, a second molecular label, and a bait sequence, wherein at least 10 of the second plurality of oligonucleotide barcodes comprise different molecular label sequences; a template switch oligonucleotide (TSO) comprising (i) a complement of a blocking sequence and (ii) a bait sequence; a DNA polymerase lacking at least one of 5' to 3' exonuclease activity and 3' to 5' exonuclease activity; and/or a reverse transcriptase.

### Definitions

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence to facilitate amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adaptors can be linear. The adaptors can be pre-adenylated adaptors. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adaptor can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adaptors can comprise identical and/or universal nucleic acid sequences and the 3' adaptors can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adaptors (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, a "complementary" sequence can refer to a "complement" or a "reverse complement" of a sequence. It is understood from the disclosure that if a molecule can hybridize to another molecule it may be complementary, or partially complementary, to the molecule that is hybridizing.

As used herein, the term "digital counting" can refer to a method for estimating a number of target molecules in a sample. Digital counting can include the step of determining a number of unique labels that have been associated with targets in a sample. This methodology, which can be stochastic in nature, transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode. The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH₂), group bridging the 2' oxygen atom and the 4' carbon atom wherein *n* is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semisolid surfaces to which a plurality of barcodes (e.g., stochastic barcodes) may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

As used herein, the term "stochastic barcode" can refer to a polynucleotide sequence comprising labels of the present disclosure. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "stochastic labeling."

As used here, the term "target" can refer to a composition which can be associated with a barcode (e.g., a stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transcriptases, and group II intron reverse transcriptases. Examples of group II intron reverse transcriptases include the *Lactococcus lactis* LI.LtrB intron reverse transcriptase, the *Thermosynechococcus* elongatus TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

The terms "universal adaptor primer," "universal primer adaptor" or "universal adaptor sequence" are used interchangeably to refer to a nucleotide sequence that can be used to hybridize to barcodes (e.g., stochastic barcodes) to generate gene-specific barcodes. A universal adaptor sequence can, for example, be a known sequence that is universal across all barcodes used in methods of the disclosure. For example, when multiple targets are being labeled using the methods disclosed herein, each of the target-specific sequences may be linked to the same universal adaptor sequence. In some embodiments, more than one universal adaptor sequences may be used in the methods disclosed herein. For example, when multiple targets are being labeled using the methods disclosed herein, at least two of the target-specific sequences are linked to different universal adaptor sequences. A universal adaptor primer and its complement may be included in two oligonucleotides, one of which comprises a target-specific sequence and the other comprises a barcode. For example, a universal adaptor sequence may be part of an oligonucleotide comprising a target-specific sequence to generate a nucleotide sequence that is complementary to a target nucleic acid. A second oligonucleotide comprising a barcode and a complementary sequence of the universal adaptor sequence may hybridize with the nucleotide sequence and generate a target-specific barcode (e.g., a target-specific stochastic barcode). In some embodiments, a universal adaptor primer has a sequence that is different from a universal PCR primer used in the methods of this disclosure.

### Barcodes

Barcoding, such as stochastic barcoding, has been described in, for example, US 2015/0299784, WO 2015/031691, and Fu et al, Proc Natl Acad Sci U.S.A. 2011 May 31;108(22):9026-31, the content of these publications is incorporated hereby in its entirety. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

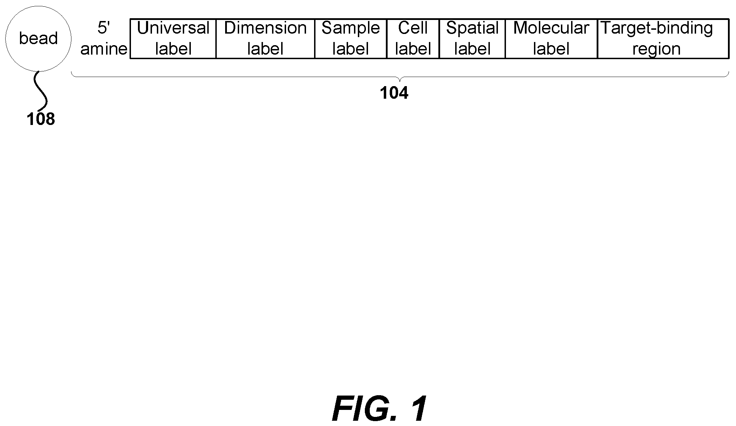

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. FIG. 1 illustrates an exemplary barcode 104 with a spatial label. The barcode 104 can comprise a 5'amine that may link the barcode to a solid support 105. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, as shown in FIG. 1, the universal label may be the 5'-most label, and the molecular label may be the 3'-most label. The spatial label, dimension label, and the cell label may be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs. In some instances, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

The barcode can comprise a target-binding region. The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, or any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the barcode (e.g., the universal label, the dimension label, the spatial label, the cell label, and the barcode sequences (e.g., molecular label)) can be separated by a spacer from another one or two of the remaining labels of the barcode. The spacer can be, for example, 1, 2, 3, 4, 5, 6, 7, 8, 9, 10, 11, 12, 13, 14, 15, 16, 17, 18, 19, or 20, or more nucleotides. In some embodiments, none of the labels of the barcode is separated by spacer.

### Universal Labels

A barcode can comprise one or more universal labels. In some embodiments, the one or more universal labels can be the same for all barcodes in the set of barcodes attached to a given solid support. In some embodiments, the one or more universal labels can be the same for all barcodes attached to a plurality of beads. In some embodiments, a universal label can comprise a nucleic acid sequence that is capable of hybridizing to a sequencing primer. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or be at most, 1, 2, 3, 4, 5, 10, 15, 20, 25, 30, 35, 40, 45, 50, 100, 200, or 300 nucleotides in length. In some embodiments, a cleavable linker or modified nucleotide can be part of the universal label sequence to enable the barcode to be cleaved off from the support.

### Dimension Labels

A barcode can comprise one or more dimension labels. In some embodiments, a dimension label can comprise a nucleic acid sequence that provides information about a dimension in which the labeling (e.g., stochastic labeling) occurred. For example, a dimension label can provide information about the time at which a target was barcoded. A dimension label can be associated with a time of barcoding (e.g., stochastic barcoding) in a sample. A dimension label can be activated at the time of labeling. Different dimension labels can be activated at different times. The dimension label provides information about the order in which targets, groups of targets, and/or samples were barcoded. For example, a population of cells can be barcoded at the G0 phase of the cell cycle. The cells can be pulsed again with barcodes (e.g., stochastic barcodes) at the G1 phase of the cell cycle. The cells can be pulsed again with barcodes at the S phase of the cell cycle, and so on. Barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, 10, or more times. The dimension label can be reversibly activatable, for example, at least 1, 2, 3, 4, 5, 6, 7, 8, 9,, 10 or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all barcodes (e.g., stochastic barcodes) attached to a given solid support (e.g., a bead), but different for different solid supports (e.g., beads). In some embodiments, at least 60%, 70%, 80%, 85%, 90%, 95%, 97%, 99% or 100%, of barcodes on the same solid support can comprise the same dimension label. In some embodiments, at least 60% of barcodes on the same solid support can comprise the same dimension label. In some embodiments, at least 95% of barcodes on the same solid support can comprise the same dimension label.

There can be as many as 10⁶ or more unique dimension label sequences represented in a plurality of solid supports (e.g., beads). A dimension label can be, be about, be at least, or be at most, 1, 2, 3, 4, 5, 10, 15, 20, 25, 30, 35, 40, 45, 50, 100, 125, 150, 200, or 300, or a number or a range between any two of these values, nucleotides in length.

### Spatial Labels

A barcode can comprise one or more spatial labels. In some embodiments, a spatial label can comprise a nucleic acid sequence that provides information about the spatial orientation of a target molecule which is associated with the barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g., a well, a container, or a droplet). In some embodiments, multiple spatial labels are used together to encode one or more positions in space.

The spatial label can be identical for all barcodes attached to a given solid support (e.g., a bead), but different for different solid supports (e.g., beads). In some embodiments, the percentage of barcodes on the same solid support comprising the same spatial label can be, or be about, 60%, 70%, 80%, 85%, 90%, 95%, 97%, 99%, 100%, or a number or a range between any two of these values. In some embodiments, the percentage of barcodes on the same solid support comprising the same spatial label can be at least, or be at most, 60%, 70%, 80%, 85%, 90%, 95%, 97%, 99%, or 100%. In some embodiments, at least 60% of barcodes on the same solid support can comprise the same spatial label. In some embodiments, at least 95% of barcodes on the same solid support can comprise the same spatial label.

There can be as many as 10⁶ or more unique spatial label sequences represented in a plurality of solid supports (e.g., beads). A spatial label can be, be about, at least or at most, 1, 2, 3, 4, 5, 10, 15, 20, 25, 30, 35, 40, 45, 50, 100, 150, 200, 250, or 300, or a number or a range between any two of these values, nucleotides in length. A spatial label can comprise between about 20 to about 125 nucleotides in length.

### Cell labels

A barcode (e.g., a stochastic barcode) can comprise one or more cell labels. In some embodiments, a cell label can comprise a nucleic acid sequence that provides information for determining which target nucleic acid originated from which cell. In some embodiments, the cell label is identical for all barcodes attached to a given solid support (e.g., a bead), but different for different solid supports (e.g., beads). In some embodiments, the percentage of barcodes on the same solid support comprising the same cell label can be, be about, be at least about, be at most about, 60%, 70%, 80%, 85%, 90%, 95%, 97%, 99%, 100%, or a number or a range between any two of these values.

There can be as many as 10⁶ or more unique cell label sequences represented in a plurality of solid supports (e.g., beads). A cell label can be, or be about, 1, 2, 3, 4, 5, 10, 15, 20, 25, 30, 35, 40, 45, 50, or a number or a range between any two of these values, nucleotides in length. A cell label can be at least, or be at most, 1, 2, 3, 4, 5, 10, 15, 20, 25, 30, 35, 40, 45, 50, 100, 125, 150, 200, or 300 nucleotides in length.

### Barcode Sequences

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., a bead). In some embodiments, there can be, or be about, 10², 10³, 10⁴, 10⁵, 10⁶, 10⁷, 10⁸, 10⁹, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or be at most, 10², 10³, 10⁴, 10⁵, 10⁶, 10⁷, 10⁸, or 10⁹, unique barcode sequences. The unique molecular label sequences can be attached to a given solid support (e.g., a bead). In some embodiments, the unique molecular label sequence is partially or entirely encompassed by a particle (e.g., a hydrogel bead).

The length of a barcode can be different in different implementations. For example, a barcode can be, or be about, 1, 2, 3, 4, 5, 10, 15, 20, 25, 30, 35, 40, 45, 50, or a number or a range between any two of these values, nucleotides in length. As another example, a barcode can be at least, or be at most, 1, 2, 3, 4, 5, 10, 15, 20, 25, 30, 35, 40, 45, 50, 100, 200, or 300 nucleotides in length.

### Molecular Labels

A barcode (e.g., a stochastic barcode) can comprise one or more molecular labels. Molecular labels can include barcode sequences. In some embodiments, a molecular label can comprise a nucleic acid sequence that provides identifying information for the specific type of target nucleic acid species hybridized to the barcode. A molecular label can comprise a nucleic acid sequence that provides a counter for the specific occurrence of the target nucleic acid species hybridized to the barcode (e.g., target-binding region).

In some embodiments, a diverse set of molecular labels are attached to a given solid support (e.g., a bead). In some embodiments, there can be, or be about, 10², 10³, 10⁴, 10⁵, 10⁶, 10⁷, 10⁸, 10⁹, or a number or a range between any two of these values, of unique molecular label sequences. For example, a plurality of barcodes can comprise about 6561 molecular labels with distinct sequences. As another example, a plurality of barcodes can comprise about 65536 molecular labels with distinct sequences. In some embodiments, there can be at least, or be at most, 10², 10³, 10⁴, 10⁵, 10⁶, 10⁷, 10⁸, or 10⁹, unique molecular label sequences. Barcodes with unique molecular label sequences can be attached to a given solid support (e.g., a bead).

For barcoding (e.g., stochastic barcoding) using a plurality of stochastic barcodes, the ratio of the number of different molecular label sequences and the number of occurrence of any of the targets can be, be about, be at least, or be at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100: 1, or a number or a range between any two of these values. A target can be an mRNA species comprising mRNA molecules with identical or nearly identical sequences.

A molecular label can be, or be about, 1, 2, 3, 4, 5, 10, 15, 20, 25, 30, 35, 40, 45, 50, or a number or a range between any two of these values, nucleotides in length. A molecular label can be at least, or be at most, 1, 2, 3, 4, 5, 10, 15, 20, 25, 30, 35, 40, 45, 50, 100, 200, or 300 nucleotides in length.

### Target-Binding Region

A barcode can comprise one or more target binding regions, such as capture probes. In some embodiments, a target-binding region can hybridize with a target of interest. In some embodiments, the target binding regions can comprise a nucleic acid sequence that hybridizes specifically to a target (e.g., target nucleic acid, target molecule, e.g., a cellular nucleic acid to be analyzed), for example to a specific gene sequence. In some embodiments, a target binding region can comprise a nucleic acid sequence that can attach (e.g., hybridize) to a specific location of a specific target nucleic acid. In some embodiments, the target binding region can comprise a nucleic acid sequence that is capable of specific hybridization to a restriction enzyme site overhang (e.g., an EcoRI sticky-end overhang). The barcode can then ligate to any nucleic acid molecule comprising a sequence complementary to the restriction site overhang.

In some embodiments, a target binding region can comprise a non-specific target nucleic acid sequence. A non-specific target nucleic acid sequence can refer to a sequence that can bind to multiple target nucleic acids, independent of the specific sequence of the target nucleic acid. For example, target binding region can comprise a random multimer sequence, or an oligo(dT) sequence that hybridizes to the poly(A) tail on mRNA molecules. A random multimer sequence can be, for example, a random dimer, trimer, quatramer, pentamer, hexamer, septamer, octamer, nonamer, decamer, or higher multimer sequence of any length. In some embodiments, the target binding region is the same for all barcodes attached to a given bead. In some embodiments, the target binding regions for the plurality of barcodes attached to a given bead can comprise two or more different target binding sequences. A target binding region can be, or be about, 5, 10, 15, 20, 25, 30, 35, 40, 45, 50, or a number or a range between any two of these values, nucleotides in length. A target binding region can be at most about 5, 10, 15, 20, 25, 30, 35, 40, 45, 50 or more nucleotides in length.

In some embodiments, a target-binding region can comprise an oligo(dT) which can hybridize with mRNAs comprising polyadenylated ends. A target-binding region can be gene-specific. For example, a target-binding region can be configured to hybridize to a specific region of a target. A target-binding region can be, be about, be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. When a barcode comprises a gene-specific target-binding region, the barcode can be referred to herein as a gene-specific barcode.

### Orientation Property

A stochastic barcode (e.g., a stochastic barcode) can comprise one or more orientation properties which can be used to orient (e.g., align) the barcodes. A barcode can comprise a moiety for isoelectric focusing. Different barcodes can comprise different isoelectric focusing points. When these barcodes are introduced to a sample, the sample can undergo isoelectric focusing in order to orient the barcodes into a known way. In this way, the orientation property can be used to develop a known map of barcodes in a sample. Exemplary orientation properties can include, electrophoretic mobility (e.g., based on size of the barcode), isoelectric point, spin, conductivity, and/or self-assembly. For example, barcodes with an orientation property of self-assembly, can self-assemble into a specific orientation (e.g., nucleic acid nanostructure) upon activation.

### Affinity Property

A barcode (e.g., a stochastic barcode) can comprise one or more affinity properties. For example, a spatial label can comprise an affinity property. An affinity property can include a chemical and/or biological moiety that can facilitate binding of the barcode to another entity (e.g., cell receptor). For example, an affinity property can comprise an antibody, for example, an antibody specific for a specific moiety (e.g., receptor) on a sample. In some embodiments, the antibody can guide the barcode to a specific cell type or molecule. Targets at and/or near the specific cell type or molecule can be labeled (e.g., stochastically labeled). The affinity property can, in some embodiments, provide spatial information in addition to the nucleotide sequence of the spatial label because the antibody can guide the barcode to a specific location. The antibody can be a therapeutic antibody, for example a monoclonal antibody or a polyclonal antibody. The antibody can be humanized or chimeric. The antibody can be a naked antibody or a fusion antibody.

The antibody can be a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

The antibody fragment can be, for example, a portion of an antibody such as F(ab')2, Fab', Fab, Fv, sFv and the like. In some embodiments, the antibody fragment can bind with the same antigen that is recognized by the full-length antibody. The antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (CD8, CD34, CD45), and therapeutic antibodies.

### Universal Adaptor Primer

A barcode can comprise one or more universal adaptor primers. For example, a gene-specific barcode, such as a gene-specific stochastic barcode, can comprise a universal adaptor primer. A universal adaptor primer can refer to a nucleotide sequence that is universal across all barcodes. A universal adaptor primer can be used for building gene-specific barcodes. A universal adaptor primer can be, be about, at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length.

### Linker

When a barcode comprises more than one of a type of label (e.g., more than one cell label or more than one barcode sequence, such as one molecular label), the labels may be interspersed with a linker label sequence. A linker label sequence can be at least about, or at most about, 5, 10, 15, 20, 25, 30, 35, 40, 45, 50 or more nucleotides in length. In some instances, a linker label sequence is 12 nucleotides in length. A linker label sequence can be used to facilitate the synthesis of the barcode. The linker label can comprise an error-correcting (e.g., Hamming) code.

### Solid Supports

Barcodes, such as stochastic barcodes, disclosed herein can, in some embodiments, be associated with a solid support. The solid support can be, for example, a synthetic particle. In some embodiments, some or all of the barcode sequences, such as molecular labels for stochastic barcodes (e.g., the first barcode sequences) of a plurality of barcodes (e.g., the first plurality of barcodes) on a solid support differ by at least one nucleotide. The cell labels of the barcodes on the same solid support can be the same. The cell labels of the barcodes on different solid supports can differ by at least one nucleotide. For example, first cell labels of a first plurality of barcodes on a first solid support can have the same sequence, and second cell labels of a second plurality of barcodes on a second solid support can have the same sequence. The first cell labels of the first plurality of barcodes on the first solid support and the second cell labels of the second plurality of barcodes on the second solid support can differ by at least one nucleotide. A cell label, a barcode, or both can be, for example, about 5-20 nucleotides long. The synthetic particle can be, for example, a bead.

The bead can be, for example, a silica gel bead, a controlled pore glass bead, a magnetic bead, a Dynabead, a sephadex/sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, or any combination thereof.

In some embodiments, the bead can be a polymeric bead, for example a deformable bead or a gel bead, functionalized with barcodes or stochastic barcodes (such as gel beads from 10X Genomics (San Francisco, CA). In some implementation, a gel bead can comprise a polymer-based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the particle can be disruptable (e.g., dissolvable, degradable). For example, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some embodiments, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some embodiments, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte. In some embodiments, at least one barcode of the plurality of barcodes can be immobilized on the particle, partially immobilized on the particle, enclosed in the particle, partially enclosed in the particle, or any combination thereof.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), poly(lactic-co-glycolic acid) (PLGA).

Numerous chemical stimuli can be used to trigger the disruption, dissolution, or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity of the bead wall.

Biological stimuli may also be used to trigger disruption, dissolution, or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat can cause melting of a bead such that the bead wall disintegrates. In some embodiments, the heat can increase the internal pressure of the inner components of the bead such that the bead ruptures or explodes. In some embodiments, the heat can transform the bead into a shrunken dehydrated state. The heat may also act upon heat-sensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. In one example, incorporation of Fe₃O₄ nanoparticles into polyelectrolyte containing beads triggers rupture in the presence of an oscillating magnetic field stimulus.

A bead may also be disrupted, dissolved, or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus may also be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with SiO₂ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches result in a bead wall that may disintegrate or become more porous upon the application of a light trigger.

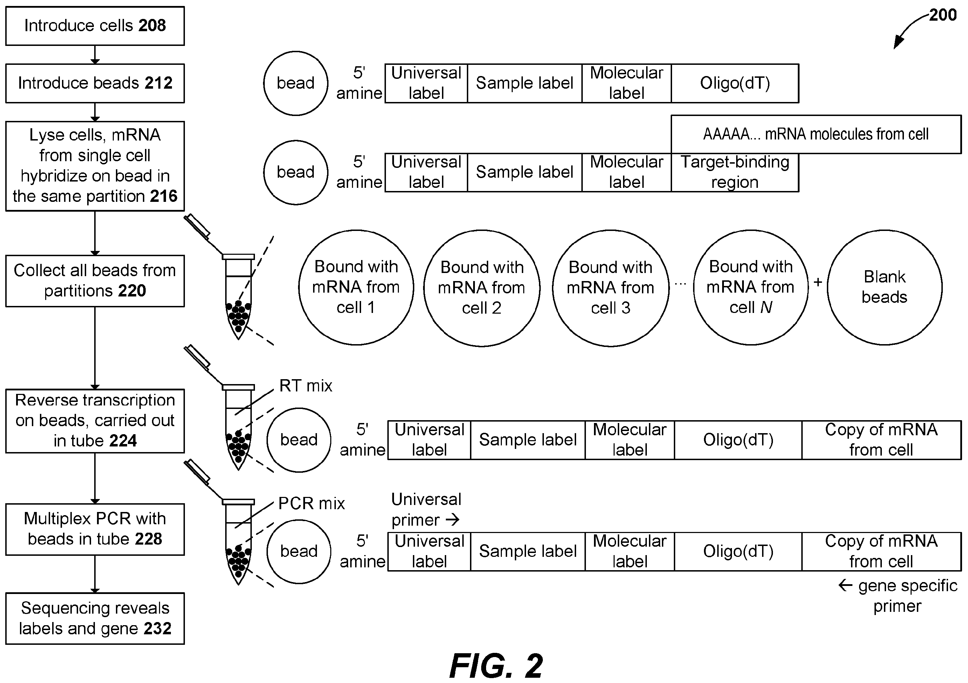

For example, in a non-limiting example of barcoding (e.g., stochastic barcoding) illustrated in FIG. 2, after introducing cells such as single cells onto a plurality of microwells of a microwell array at block 208, beads can be introduced onto the plurality of microwells of the microwell array at block 212. Each microwell can comprise one bead. The beads can comprise a plurality of barcodes. A barcode can comprise a 5' amine region attached to a bead. The barcode can comprise a universal label, a barcode sequence (e.g., a molecular label), a target-binding region, or any combination thereof.

The barcodes disclosed herein can be associated with (e.g., attached to) a solid support (e.g., a bead). The barcodes associated with a solid support can each comprise a barcode sequence selected from a group comprising at least 100 or 1000 barcode sequences with unique sequences. In some embodiments, different barcodes associated with a solid support can comprise barcode with different sequences. In some embodiments, a percentage of barcodes associated with a solid support comprises the same cell label. For example, the percentage can be, be about, be at least, or be at most, 60%, 70%, 80%, 85%, 90%, 95%, 97%, 99%, 100%, or a number or a range between any two of these values. In some embodiments, barcodes associated with a solid support can have the same cell label. The barcodes associated with different solid supports can have different cell labels selected from a group comprising at least 100 or 1000 cell labels with unique sequences.

The barcodes disclosed herein can be associated to (e.g., attached to) a solid support (e.g., a bead). In some embodiments, barcoding the plurality of targets in the sample can be performed with a solid support including a plurality of synthetic particles associated with the plurality of barcodes. In some embodiments, the solid support can include a plurality of synthetic particles associated with the plurality of barcodes. The spatial labels of the plurality of barcodes on different solid supports can differ by at least one nucleotide. The solid support can, for example, include the plurality of barcodes in two dimensions or three dimensions. The synthetic particles can be beads. The beads can be silica gel beads, controlled pore glass beads, magnetic beads, Dynabeads, Sephadex/Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The barcodes may not be associated with solid supports. The barcodes can be individual nucleotides. The barcodes can be associated with a substrate.

As used herein, the terms "tethered," "attached," and "immobilized," are used interchangeably, and can refer to covalent or non-covalent means for attaching barcodes to a solid support. Any of a variety of different solid supports can be used as solid supports for attaching pre-synthesized barcodes or for *in situ* solid-phase synthesis of barcode.

In some embodiments, the solid support is a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite (Fe₃O₄; magnetite) nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, Sepharose, agarose, hydrogel, polymer, cellulose, nylon, or any combination thereof.

In some embodiments, the bead (e.g., the bead to which the labels are attached) is a hydrogel bead. In some embodiments, the bead comprises hydrogel.

Some embodiments disclosed herein include one or more particles (for example, beads). Each of the particles can comprise a plurality of oligonucleotides (e.g., barcodes). Each of the plurality of oligonucleotides can comprise a barcode sequence (e.g., a molecular label sequence), a cell label, and a target-binding region (e.g., an oligo(dT) sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, a number or a range between any two of these values, or more. In some embodiments, the number of cell label sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. In some embodiments, no more than 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence. In some embodiment, the plurality of particles that include oligonucleotides with the same cell sequence can be at most 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more. In some embodiments, none of the plurality of the particles has the same cell label sequence.

The plurality of oligonucleotides on each particle can comprise different barcode sequences (e.g., molecular labels). In some embodiments, the number of barcode sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, or a number or a range between any two of these values. In some embodiments, the number of barcode sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. For example, at least 100 of the plurality of oligonucleotides comprise different barcode sequences. As another example, in a single particle, at least 100, 500, 1000, 5000, 10000, 15000, 20000, 50000, a number or a range between any two of these values, or more of the plurality of oligonucleotides comprise different barcode sequences. Some embodiments provide a plurality of the particles comprising barcodes. In some embodiments, the ratio of an occurrence (or a copy or a number) of a target to be labeled and the different barcode sequences can be at least 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

The size of the beads can vary. For example, the diameter of the bead can range from 0.1 micrometer to 50 micrometers. In some embodiments, the diameter of the bead can be, or be about, 0.1, 0.5, 1, 2, 3, 4, 5, 6, 7, 8, 9, 10, 20, 30, 40, or 50 micrometers, or a number or a range between any two of these values.

The diameter of the bead can be related to the diameter of the wells of the substrate. In some embodiments, the diameter of the bead can be, or be about, 10%, 20%, 30%, 40%, 50%, 60%, 70%, 80%, 90%, 100%, or a number or a range between any two of these values, longer or shorter than the diameter of the well. The diameter of the beads can be related to the diameter of a cell (e.g., a single cell entrapped by a well of the substrate). In some embodiments, the diameter of the bead can be at least, or be at most, 10%, 20%, 30%, 40%, 50%, 60%, 70%, 80%, 90%, or 100% longer or shorter than the diameter of the well. The diameter of the beads can be related to the diameter of a cell (e.g., a single cell entrapped by a well of the substrate). In some embodiments, the diameter of the bead can be, or be about, be at least, or be at most, 10%, 20%, 30%, 40%, 50%, 60%, 70%, 80%, 90%, 100%, 150%, 200%, 250%, 300%, or a number or a range between any two of these values, longer or shorter than the diameter of the cell.

A bead can be attached to and/or embedded in a substrate. A bead can be attached to and/or embedded in a gel, hydrogel, polymer and/or matrix. The spatial position of a bead within a substrate (e.g., gel, matrix, scaffold, or polymer) can be identified using the spatial label present on the barcode on the bead which can serve as a location address.

Examples of beads can include, but are not limited to, streptavidin beads, agarose beads, magnetic beads, Dynabeads^{®}, MACS^{®} microbeads, antibody conjugated beads (e.g., anti-immunoglobulin microbeads), protein A conjugated beads, protein G conjugated beads, protein A/G conjugated beads, protein L conjugated beads, oligo(dT) conjugated beads, silica beads, silica-like beads, anti-biotin microbeads, anti-fluorochrome microbeads, and BcMag^{™} Carboxyl-Terminated Magnetic Beads.

A bead can be associated with (e.g., impregnated with) quantum dots or fluorescent dyes to make it fluorescent in one fluorescence optical channel or multiple optical channels. A bead can be associated with iron oxide or chromium oxide to make it paramagnetic or ferromagnetic. Beads can be identifiable. For example, a bead can be imaged using a camera. A bead can have a detectable code associated with the bead. For example, a bead can comprise a barcode. A bead can change size, for example, due to swelling in an organic or inorganic solution. A bead can be hydrophobic. A bead can be hydrophilic. A bead can be biocompatible.

A solid support (e.g., a bead) can be visualized. The solid support can comprise a visualizing tag (e.g., fluorescent dye). A solid support (e.g., a bead) can be etched with an identifier (e.g., a number). The identifier can be visualized through imaging the beads.

A solid support can comprise an insoluble, semi-soluble, or insoluble material. A solid support can be referred to as "functionalized" when it includes a linker, a scaffold, a building block, or other reactive moiety attached thereto, whereas a solid support may be "nonfunctionalized" when it lacks such a reactive moiety attached thereto. The solid support can be employed free in solution, such as in a microtiter well format; in a flow-through format, such as in a column; or in a dipstick.

The solid support can comprise a membrane, paper, plastic, coated surface, flat surface, glass, slide, chip, or any combination thereof. A solid support can take the form of resins, gels, microspheres, or other geometric configurations. A solid support can comprise silica chips, microparticles, nanoparticles, plates, arrays, capillaries, flat supports such as glass fiber filters, glass surfaces, metal surfaces (steel, gold silver, aluminum, silicon and copper), glass supports, plastic supports, silicon supports, chips, filters, membranes, microwell plates, slides, plastic materials including multiwell plates or membranes (e.g., formed of polyethylene, polypropylene, polyamide, polyvinylidenedifluoride), and/or wafers, combs, pins or needles (e.g., arrays of pins suitable for combinatorial synthesis or analysis) or beads in an array of pits or nanoliter wells of flat surfaces such as wafers (e.g., silicon wafers), wafers with pits with or without filter bottoms.

The solid support can comprise a polymer matrix (e.g., gel, hydrogel). The polymer matrix may be able to permeate intracellular space (e.g., around organelles). The polymer matrix may able to be pumped throughout the circulatory system.

### Substrates and Microwell Array

As used herein, a substrate can refer to a type of solid support. A substrate can refer to a solid support that can comprise barcodes or stochastic barcodes of the disclosure. A substrate can, for example, comprise a plurality of microwells. For example, a substrate can be a well array comprising two or more microwells. In some embodiments, a microwell can comprise a small reaction chamber of defined volume. In some embodiments, a microwell can entrap one or more cells. In some embodiments, a microwell can entrap only one cell. In some embodiments, a microwell can entrap one or more solid supports. In some embodiments, a microwell can entrap only one solid support. In some embodiments, a microwell entraps a single cell and a single solid support (e.g., a bead). A microwell can comprise barcode reagents of the disclosure.

### Methods of Barcoding

The disclosure provides for methods for estimating the number of distinct targets at distinct locations in a physical sample (e.g., tissue, organ, tumor, cell). The methods can comprise placing barcodes (e.g., stochastic barcodes) in close proximity with the sample, lysing the sample, associating distinct targets with the barcodes, amplifying the targets and/or digitally counting the targets. The method can further comprise analyzing and/or visualizing the information obtained from the spatial labels on the barcodes. In some embodiments, a method comprises visualizing the plurality of targets in the sample. Mapping the plurality of targets onto the map of the sample can include generating a two-dimensional map or a three-dimensional map of the sample. The two-dimensional map and the three-dimensional map can be generated prior to or after barcoding (e.g., stochastically barcoding) the plurality of targets in the sample. Visualizing the plurality of targets in the sample can include mapping the plurality of targets onto a map of the sample. Mapping the plurality of targets onto the map of the sample can include generating a two-dimensional map or a three-dimensional map of the sample. The two-dimensional map and the three-dimensional map can be generated prior to or after barcoding the plurality of targets in the sample. in some embodiments, the two-dimensional map and the three-dimensional map can be generated before or after lysing the sample. Lysing the sample before or after generating the two-dimensional map or the three-dimensional map can include heating the sample, contacting the sample with a detergent, changing the pH of the sample, or any combination thereof.

In some embodiments, barcoding the plurality of targets comprises hybridizing a plurality of barcodes with a plurality of targets to create barcoded targets (e.g., stochastically barcoded targets). Barcoding the plurality of targets can comprise generating an indexed library of the barcoded targets. Generating an indexed library of the barcoded targets can be performed with a solid support comprising the plurality of barcodes (e.g., stochastic barcodes).

### Contacting a Sample and a Barcode

The disclosure provides for methods for contacting a sample (e.g., cells) to a substrate of the disclosure. A sample comprising, for example, a cell, organ, or tissue thin section, can be contacted to barcodes (e.g., stochastic barcodes). The cells can be contacted, for example, by gravity flow wherein the cells can settle and create a monolayer. The sample can be a tissue thin section. The thin section can be placed on the substrate. The sample can be onedimensional (e.g., forms a planar surface). The sample (e.g., cells) can be spread across the substrate, for example, by growing/culturing the cells on the substrate.

When barcodes are in close proximity to targets, the targets can hybridize to the barcode. The barcodes can be contacted at a non-depletable ratio such that each distinct target can associate with a distinct barcode of the disclosure. To ensure efficient association between the target and the barcode, the targets can be cross-linked to barcode.

### Cell Lysis

Following the distribution of cells and barcodes, the cells can be lysed to liberate the target molecules. Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, 10, or more. The pH of the lysis buffer can be at most about 1, 2, 3, 4, 5, 6, 7, 8, 9,10, or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about or at most about 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about or at most about 1, 5, 10, 15, 20, 25, or 30 mM or more. In some embodiments, the concentration of chelating agent in the lysis buffer is about 10 mM. The lysis buffer can comprise a reducing reagent (e.g., beta-mercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

### Attachment of Barcodes to Target Nucleic Acid Molecules

Following lysis of the cells and release of nucleic acid molecules therefrom, the nucleic acid molecules can randomly associate with the barcodes of the co-localized solid support. Association can comprise hybridization of a barcode's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule. For example, in a non-limiting example of barcoding illustrated in FIG. 2, at block 216, mRNA molecules can hybridize to barcodes on beads. For example, single-stranded nucleotide fragments can hybridize to the target-binding regions of barcodes.

Attachment can further comprise ligation of a barcode's target recognition region and a portion of the target nucleic acid molecule. For example, the target binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The barcode can then be ligated to any nucleic acid molecule comprising a sequence complementary to the restriction site overhang. A ligase (e.g., T4 DNA ligase) can be used to join the two fragments.

For example, in a non-limiting example of barcoding illustrated in FIG. 2, at block 220, the labeled targets from a plurality of cells (or a plurality of samples) (e.g., target-barcode molecules) can be subsequently pooled, for example, into a tube. The labeled targets can be pooled by, for example, retrieving the barcodes and/or the beads to which the target-barcode molecules are attached.

The retrieval of solid support-based collections of attached target-barcode molecules can be implemented by use of magnetic beads and an externally-applied magnetic field. Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

### Reverse Transcription

The disclosure provides for a method to create a target-barcode conjugate using reverse transcription (e.g., at block 224 of FIG. 2). The target-barcode conjugate can comprise the barcode and a complementary sequence of all or a portion of the target nucleic acid (i.e., a barcoded cDNA molecule, such as a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of the labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

Reverse transcription can occur repeatedly to produce multiple labeled-cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

### Amplification

One or more nucleic acid amplification reactions (e.g., at block 228 of FIG. 2) can be performed to create multiple copies of the labeled target nucleic acid molecules. Amplification can be performed in a multiplexed manner, wherein multiple target nucleic acid sequences are amplified simultaneously. The amplification reaction can be used to add sequencing adaptors to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeled amplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule. The single-stranded molecule can comprise DNA, RNA, or a combination thereof. The nucleic acids of the disclosure can comprise synthetic or altered nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can comprise less than 12-15 nucleotides. The one or more primers can anneal to at least a portion of the plurality of labeled targets (e.g., stochastically labeled targets). The one or more primers can anneal to the 3' end or 5' end of the plurality of labeled targets. The one or more primers can anneal to an internal region of the plurality of labeled targets. The intemal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers, at least 960 or more custom primers, or at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adaptors to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by PCR. PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, MDA, TMA, NASBA, SDA, real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a LCR, a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or RAM.

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids of the present invention can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adaptors can be added for sequencing using, for example, next generation sequencing (NGS). The sequencing results can be used to determine cell labels, molecular labels, and sequences of nucleotide fragments of the one or more copies of the targets, for example at block 232 of FIG. 2.

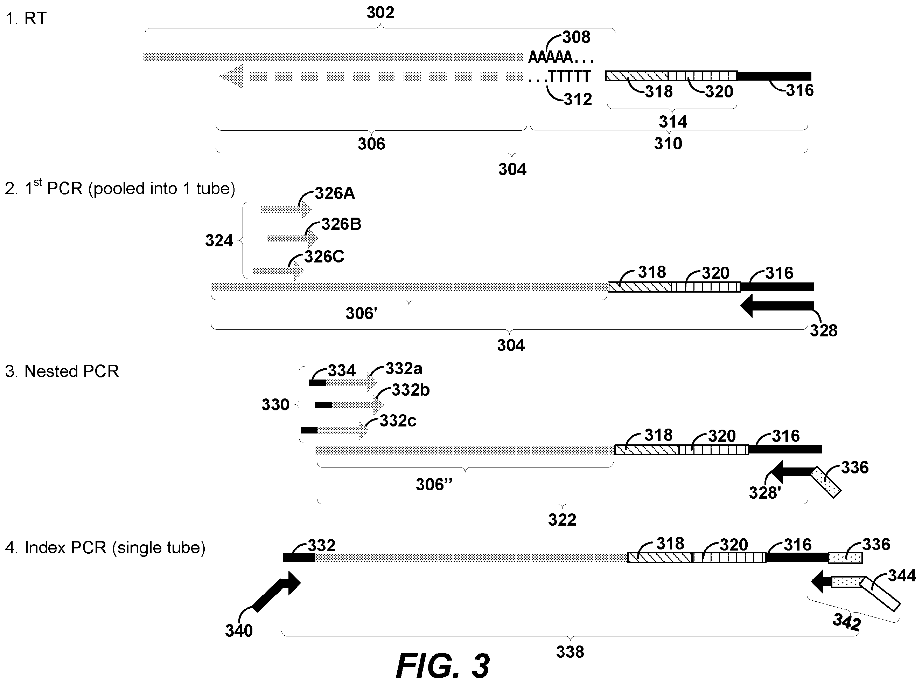

FIG. 3 is a schematic illustration showing a non-limiting exemplary process of generating an indexed library of the barcoded targets (e.g., stochastically barcoded targets), such as barcoded mRNAs or fragments thereof. As shown in step 1, the reverse transcription process can encode each mRNA molecule with a unique molecular label sequence, a cell label sequence, and a universal PCR site. In particular, RNA molecules 302 can be reverse transcribed to produce labeled cDNA molecules 304, including a cDNA region 306, by hybridization (e.g., stochastic hybridization) of a set of barcodes (e.g., stochastic barcodes) 310 to the poly(A) tail region 308 of the RNA molecules 302. Each of the barcodes 310 can comprise a target-binding region, for example a poly(dT) region 312, a label region 314 (e.g., a barcode sequence or a molecule), and a universal PCR region 316.

In some embodiments, the cell label sequence can include 3 to 20 nucleotides. In some embodiments, the molecular label sequence can include 3 to 20 nucleotides. In some embodiments, each of the plurality of stochastic barcodes further comprises one or more of a universal label and a cell label, wherein universal labels are the same for the plurality of stochastic barcodes on the solid support and cell labels are the same for the plurality of stochastic barcodes on the solid support. In some embodiments, the universal label can include 3 to 20 nucleotides. In some embodiments, the cell label comprises 3 to 20 nucleotides.

In some embodiments, the label region 314 can include a barcode sequence or a molecular label 318 and a cell label 320. In some embodiments, the label region 314 can include one or more of a universal label, a dimension label, and a cell label. The barcode sequence or molecular label 318 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The cell label 320 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The universal label can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. Universal labels can be the same for the plurality of stochastic barcodes on the solid support and cell labels are the same for the plurality of stochastic barcodes on the solid support. The dimension label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length.

In some embodiments, the label region 314 can comprise, comprise about, comprise at least, or comprise at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different labels, such as a barcode sequence or a molecular label 318 and a cell label 320. Each label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length. A set of barcodes or stochastic barcodes 310 can contain, contain about, contain at least, or can be at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹¹, 10¹⁴, 10¹⁵, 10²⁰, or a number or a range between any of these values, barcodes or stochastic barcodes 310. And the set of barcodes or stochastic barcodes 310 can, for example, each contain a unique label region 314. The labeled cDNA molecules 304 can be purified to remove excess barcodes or stochastic barcodes 310. Purification can comprise Ampure bead purification.

As shown in step 2, products from the reverse transcription process in step 1 can be pooled into 1 tube and PCR amplified with a 1^{st} PCR primer pool and a 1^{st} universal PCR primer. Pooling is possible because of the unique label region 314. In particular, the labeled cDNA molecules 304 can be amplified to produce nested PCR labeled amplicons 322. Amplification can comprise multiplex PCR amplification. Amplification can comprise a multiplex PCR amplification with 96 multiplex primers in a single reaction volume. In some embodiments, multiplex PCR amplification can utilize, utilize about, utilize at least, or utilize at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹¹, 10¹⁴, 10¹⁵, 10²⁰, or a number or a range between any of these values, multiplex primers in a single reaction volume. Amplification can comprise using a 1^{st} PCR primer pool 324 comprising custom primers 326A-C targeting specific genes and a universal primer 328. The custom primers 326 can hybridize to a region within the cDNA portion 306' of the labeled cDNA molecule 304. The universal primer 328 can hybridize to the universal PCR region 316 of the labeled cDNA molecule 304.

As shown in step 3 of FIG. 3, products from PCR amplification in step 2 can be amplified with a nested PCR primers pool and a 2^{nd} universal PCR primer. Nested PCR can minimize PCR amplification bias. In particular, the nested PCR labeled amplicons 322 can be further amplified by nested PCR. The nested PCR can comprise multiplex PCR with nested PCR primers pool 330 of nested PCR primers 332a-c and a 2^{nd} universal PCR primer 328' in a single reaction volume. The nested PCR primer pool 328 can contain, contain about, contain at least, or contain at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different nested PCR primers 330. The nested PCR primers 332 can contain an adaptor 334 and hybridize to a region within the cDNA portion 306" of the labeled amplicon 322. The universal primer 328' can contain an adaptor 336 and hybridize to the universal PCR region 316 of the labeled amplicon 322. Thus, step 3 produces adaptor-labeled amplicon 338. In some embodiments, nested PCR primers 332 and the 2^{nd} universal PCR primer 328' may not contain the adaptors 334 and 336. The adaptors 334 and 336 can instead be ligated to the products of nested PCR to produce adaptor-labeled amplicon 338.

As shown in step 4, PCR products from step 3 can be PCR amplified for sequencing using library amplification primers. In particular, the adaptors 334 and 336 can be used to conduct one or more additional assays on the adaptor-labeled amplicon 338. The adaptors 334 and 336 can be hybridized to primers 340 and 342. The one or more primers 340 and 342 can be PCR amplification primers. The one or more primers 340 and 342 can be sequencing primers. The one or more adaptors 334 and 336 can be used for further amplification of the adaptor-labeled amplicons 338. The one or more adaptors 334 and 336 can be used for sequencing the adaptor-labeled amplicon 338. The primer 342 can contain a plate index 344 so that amplicons generated using the same set of barcodes or stochastic barcodes 310 can be sequenced in one sequencing reaction using next generation sequencing (NGS).

### Template Switch Oligonucleotide (TSO) for mRNA 5' analysis

There are provided, in some embodiments, improved Template Switch Oligonucleotide (TSO) for Rhapsody mRNA 5' analysis. In some embodiments, the improvement is accomplished with an extension blocker sequence that improves specificity. The improved TSO design can be applied to any system where the polymerase lacks a 3' to 5' exonuclease or ssDNA endonuclease. The enzyme can be either a mesophilic, or psychrophilic or thermophilic polymerase, or a mixture of polymerases as long as the specific portion of the annealing sequence is stable or stabilized at the temperature of the process.

There are provided, in some embodiments, methods and compositions designed to prevent extension towards the bead from the 5'-capture oligonucleotide (the oligo on the bead that contains a capture bait sequence to capture the sequence made during Template Switching). In some embodiments, this improvement is also designed so that the template switching efficiency is not reduced. As a consequence of the improvements achieved with the methods and compositions provided herein, there can be a marked reduction in the presence and detection and sequencing of unwanted non-targeted products.

In some embodiments, there are unwanted products that are produced during VDJ PCR. For example, the principal unwanted sequenced products can be unaligned reads or aligned to other genomic regions. Being able to block the production of these forms of products would allow the PCR reaction resources to then be used to produce more of the intended amplification products and not include extra product purifications. Additionally, when some of the unwanted products end up being sequenced, they use costly sequence resources leading to unproductive sequencing reads.

In some embodiments, beads comprise 2 different types of oligonucleotides (e.g., oligonucleotide barcodes): one designed to capture 3'-ends of mRNA (poly dT-oligos) and a second one designed to capture 5'-ends of template switched mRNA (poly dT-oligos or non-poly dT-oligos). In the case of the non-poly dT-oligo 5'-end capture sequence, the design can also have a unique amplification primer sequence nearest the bead (often referred to as the 5'-universal primer or PB-Universal primer). When the PB-Universal primer and a specific primer(s) (e.g. for VDJ) are used for PCR of 5' products, every self-hybridized (or Pretzeled) cDNA can form the exponentially amplified specific product(s) as well as linearly amplified products. In some embodiments, unwanted products are produced by these linearly amplified products. There is a need for methods and compositions that can reduce or eliminate these unwanted products.

There are provided herein methods and compositions for VDJ analysis and for analysis of other 5' gene targets. There are provided, in some embodiments, Template Switch Oligonucleotides (TSO) whose complementary DNA sequence (which is formed and attached to the 3' end of the template switched cDNA) is partially complementary to the 5'-capture bait on the bead. In some embodiments, the non-complementary portion of the complement of the TSO acts as a block of extension. In some embodiments, the unaligned sequence serves as a blocker.

In some embodiments, Template Switch Efficiency equals the % of transcripts cDNA that get template switched divided by the total cDNA of that transcript that are reverse transcribed to the 5'-end of the transcript. Surprising and importantly, the lengthening the TSOs as described herein was not found to decrease Template Switch Efficiency. In some embodiments provided herein, longer Template Switch Oligonucleotide (TSO) sequences can increase the measured Template Switch Efficiency.

In some embodiments, the methods and compositions disclosed herein reduce unwanted products that lead to non-VDJ products from VDJ PCR reactions. These unwanted products, in some embodiments, need to be blocked from forming or be separated after they form.

In some embodiments, blocking is preferred to separation, especially if the block is implemented simply, because fewer resources are wasted on products that will be separated and discarded. To block these unwanted amplicons, a blocking oligo sequence can be added to the reaction mix. For example, the systems, methods, compositions, and kits for 5'-based gene expression profiling provided herein can, in some embodiments, be employed in concert with the blocker oligonucleotides described in U.S. Patent Application Number 17/163,177, filed on January 29, 2021, entitled "MESOPHILIC DNA POLYMERASE EXTENSION BLOCKERS", the content of which is incorporated herein by reference in its entirety. As an alternative to blocking oligo sequence being added to the reaction mix, it can, as demonstrated herein, be incorporated into the TSO sequence when the bead capture sequence is only a portion of the TSO sequence.

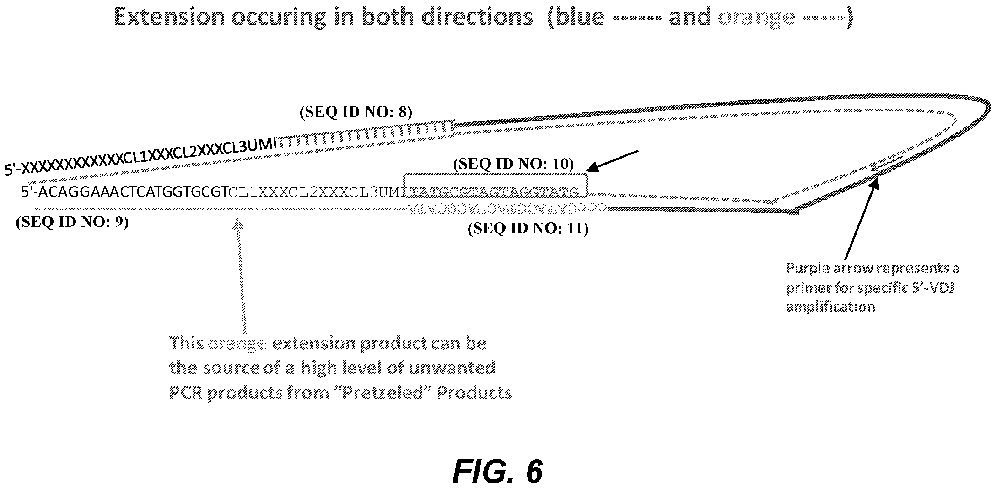

In some embodiments, when a TSO is used for template switching, its complementary sequence is copied onto the cDNA strand. In some embodiments, the mRNA and TSO are denatured off after reverse transcription and template switching. And then this cDNA with the complement to the TSO can then partially hybridize to the bait that is present on the bead (termed 'pretzeling' herein). (See FIGS. 6-10).

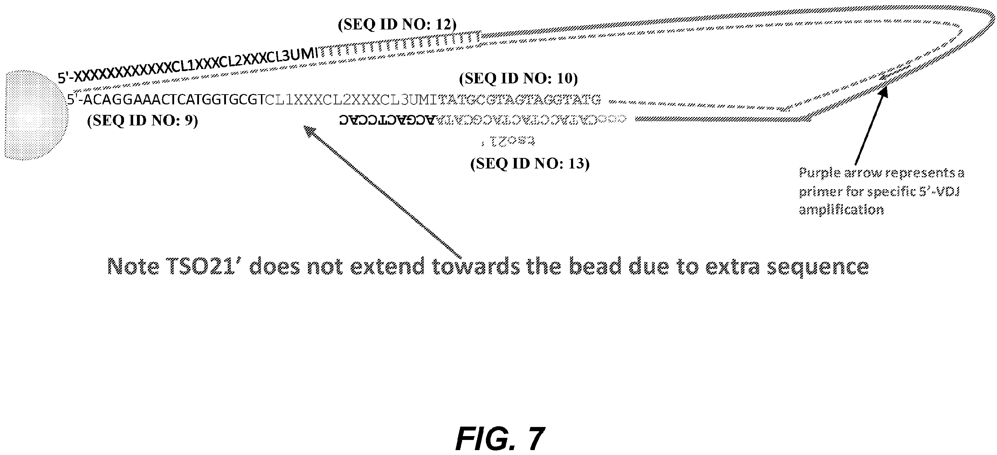

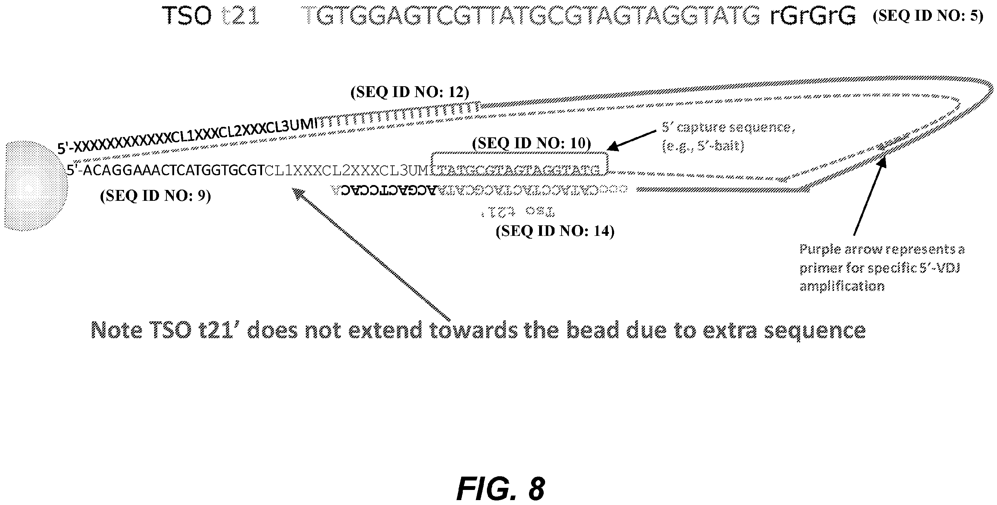

FIG. 7 depicts a TSO-complement (TSO 21') that can yield a non-complementary sequence which will act as an extension blocker for the vast majority of oligos attached to the bead. In some embodiments, the UMI on the beads contain 65,536 possible combinations with 8 N's in the UMI, and thus, an exact match of all 8 N's will only occur 1 out of 65,536 UMIs. In some embodiments, the Cell Label 3, CL3, sequence has 96 designated sequences, of which only 4 of the 96 have the 2 bases that would be complementary to the last 2 bases of the "blocking" part of the TSO 21'. Thus, in some embodiments, essentially all the pretzeling products with TSO 21' will be blocked from extension. There is provided, in some embodiments, a modified design of TSO 21 with one extra 5' base, that can lead to a blocker with a 3' end that is not complementary to any of the 96 CL3 sequences used in current Rhapsody beads. Such a modified sequence is TSO t21 (shown in FIG. 8).

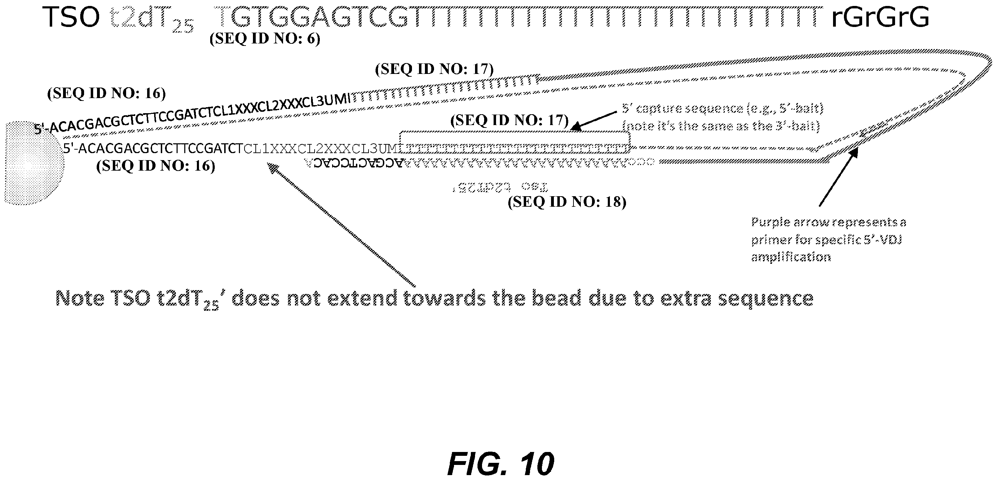

FIG. 10 depicts an alternative extension blocking sequence for use when the bead used only has one type of capture sequence (poly dT₂₅ in this example). In some embodiments the method employs a bead with only this one capture sequence. In some embodiments, the TSO disclosed herein help reduce unwanted products. In some such embodiments, only one type of bead "universal" primer exists, and amplifying off the 5' end of the gene following TS and pretzeling, the universal and a 5' specific primer can be used. This can, in some embodiments, still allow linear amplification of products on the 3' end from the "universal" primer. The TSO provided herein can reduce the exponential amplification of "universal" to "universal" products, which can be the most plentiful product made.

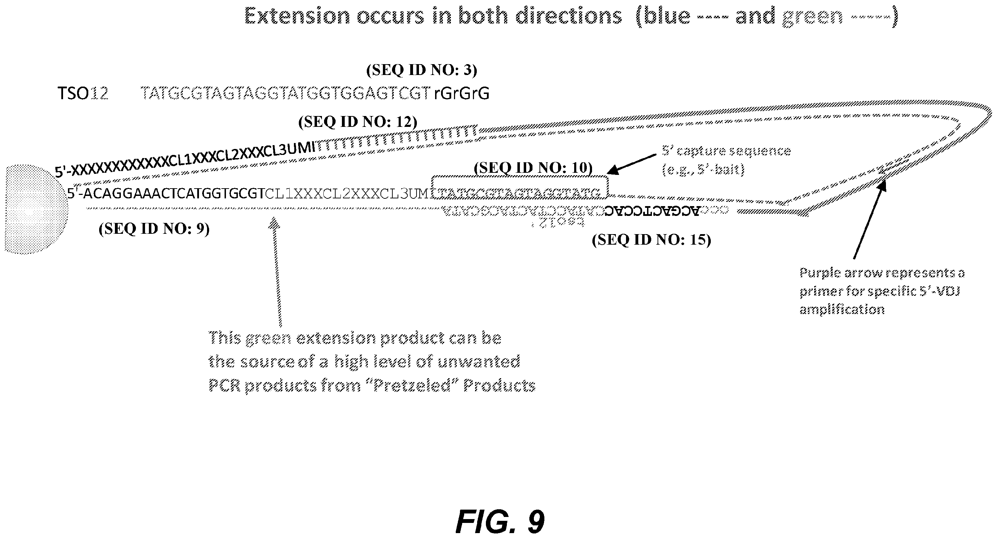

FIG. 9 depicts a longer TSO which leads to a complement that is partially complementary to the capture bait sequence, however, it does not form a blocking entity.

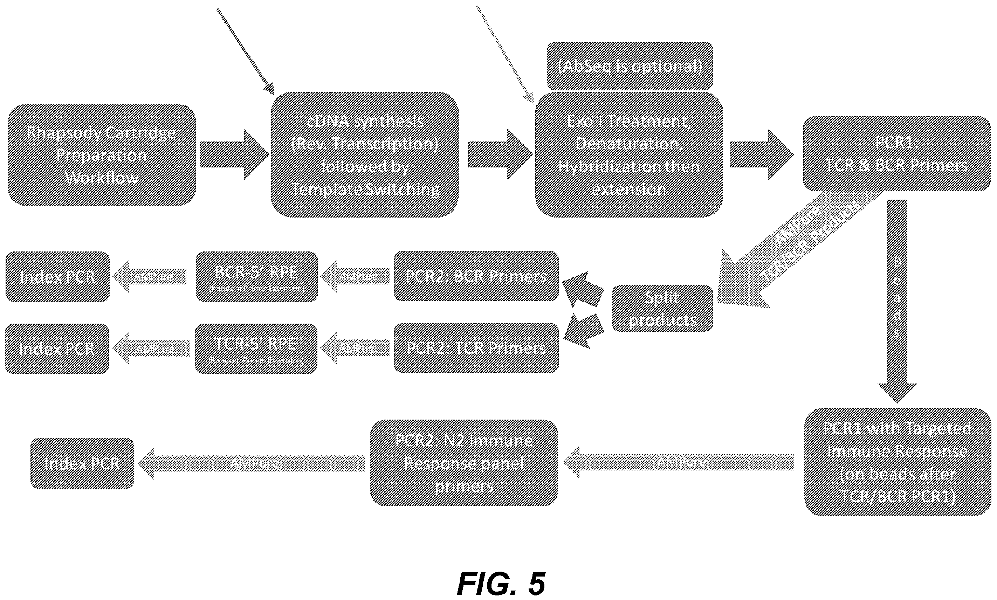

The compositions and methods provided herein can, in some embodiments, improve workflow (e.g., time and effort - less time than that need for additional separation clean-ups) as compared to current protocols and increase specificity of sequencing results (e.g., fewer unwanted products and more of the targeted products). Disclosed herein, in some embodiments, are blocking sequences derived from the complement of the oligonucleotide that is being template switched. In some embodiments, the Template Switch Oligo (TSO) is necessary to accomplish template switching, so this is not an addition of another reagent. FIG. 5 shows non-limiting exemplary workflows for the determination of the sequences of a nucleic acid target (e.g., the V(D)J region of an immune receptor) using the compositions and methods provided herein.

There are provided, in some embodiments, methods and compositions for library preparation (e.g., VDJ library preparation). In some embodiments, the methods and compositions comprise improved template switch oligonucleotides (TSO). In some embodiments, the workflow comprises WTA analysis and/or AbSeq analysis.

FIG. 5 shows a non-limiting exemplary workflows for the determination of the sequences of a nucleic acid target (e.g., the V(D)J region of an immune receptor) using the compositions and methods provided herein. Thin arrows point out to particular portions of the workflow that, in some embodiments, are improved with the use of the TSO described herein (e.g., improved interactions). In some embodiments, VDJ Library preparation follows the workflow outlined in FIG. 5, with use of targeted primers.

There are provided, in some embodiments, template switch oligonucleotides (TSO). In some embodiments, the TSO, such as, for example, TSO1 (TATGCGTAGTAGGTATG rGrGrG; SEQ ID NO: 1) and TSO2 (GTGGAGTCGTGATTATA rGrGrG; SEQ ID NO: 2), were designed by bioinformatics to be unique and non-interfering with primer panels disclosed herein, as well as the transcriptome, cell labels, etc.

TSOs with partially homologous sequences to 5' capture sequence (e.g., 5'-bait) of oligonucleotide barcodes provided herein (e.g., target-binding region, bait sequence) are provided in some embodiments, such as, for example, TSO12 (TATGCGTAGTAGGTATGGTGGAGTCGT rGrGrG; SEQ ID NO: 3), TSO21 (GTGGAGTCGTTATGCGTAGTAGGTATG rGrGrG; SEQ ID NO: 4), TSO t21 (TGTGGAGTCGTTATGCGTAGTAGGTATG rGrGrG; SEQ ID NO: 5), and CBO97 (AAGCAGTGGTATCAACGCAGAGTACAT rGrGrG; SEQ ID NO: 7). Some embodiments of the compositions and methods disclosed herein employ TSO21 or TSO t21 in concert with Rhapsody beads disclosed herein. In some embodiments, TSO t21 performs better than TSO21. TSO21 and TSO t21 are TSO whose DNA complement are partially complementary to the capture bait on the bead and whose non-complementary portions can act as an extension block.

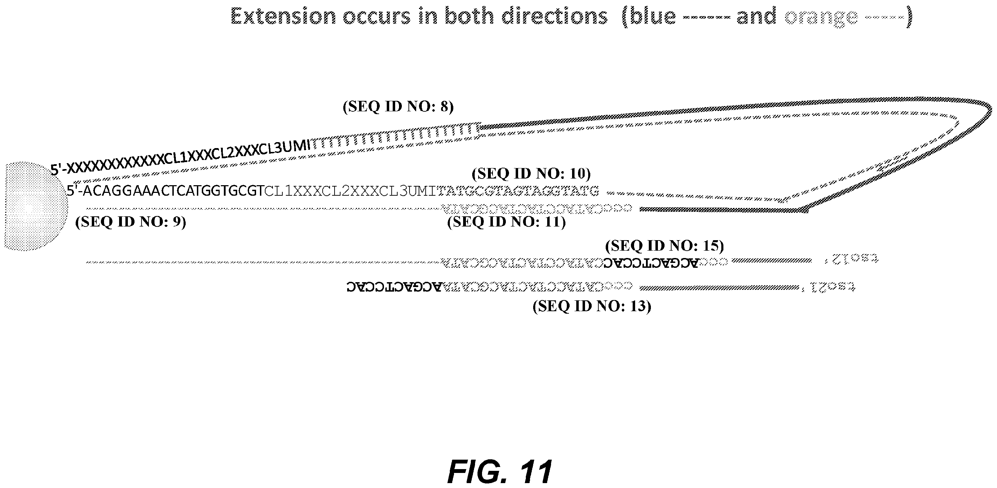

FIG. 6 and FIG. 11 depict a non-limiting schematic illustration of a library preparation method employing the template switch oligonucleotide TSO1 (TATGCGTAGTAGGTATG rGrGrG; SEQ ID NO: 1). The box comprises the 5' capture sequence (e.g., 5'-bait). Extension is shown occurring in two directions (blue and orange dotted lines). In some embodiments, the orange extension product (e.g., the extension of the barcoded nucleic acid molecule) can be the source of a high level of unwanted PCR products from the extension reaction (e.g., "pretzeled" products). FIG. 6 and FIG. 11 depict the pretzeled TSO1 product. The purple arrow depicts a specific primer that can be used for specific 5'-VDJ amplification.

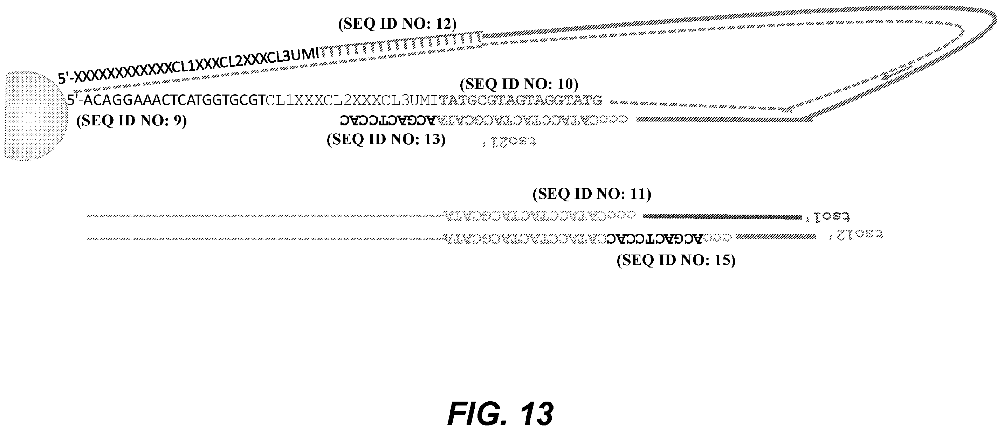

FIG. 7 and FIG. 13 depict a non-limiting schematic illustration of a library preparation method employing the template switch oligonucleotide TSO21 (GTGGAGTCGTTATGCGTAGTAGGTATG rGrGrG; SEQ ID NO: 4). FIG. 7 and FIG. 13 depict the pretzeled TSO21 product. The purple arrow depicts a specific primer that can be used for specific 5'-VDJ amplification. As seen in FIG. 7 and FIG. 13, TSO21' does not extend towards the bead, in some embodiments, due to the extra sequence (e.g., the blocking sequence). In some embodiments, the extra sequence acts as an extension blocker.

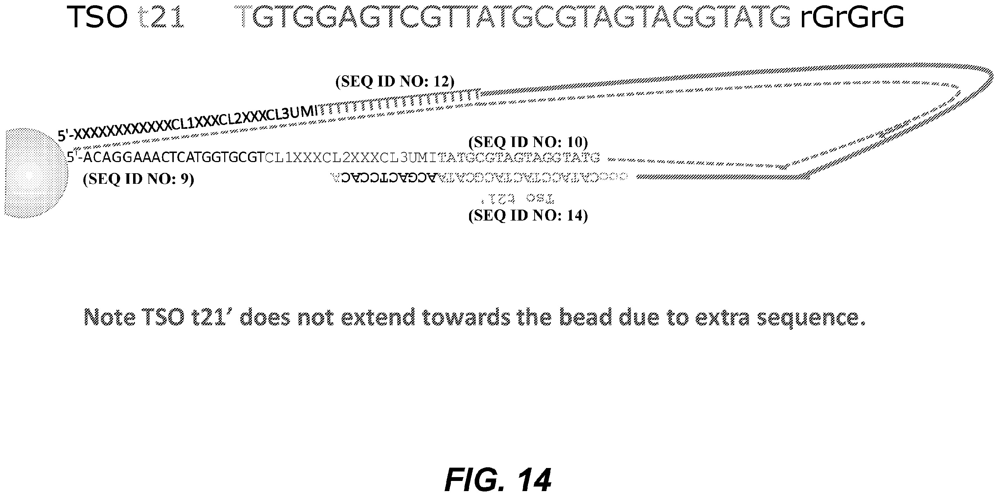

FIG. 8 and FIG. 14 depict a non-limiting schematic illustration of a library preparation method employing the template switch oligonucleotide TSO t21 (TGTGGAGTCGTTATGCGTAGTAGGTATG rGrGrG; SEQ ID NO: 5). TSO t21 is a modified version of TSO21 comprising an extra nucleotide (e.g., a blocker nucleotide). The purple arrow depicts a specific primer that can be used for specific 5'-VDJ amplification. The box comprises the 5' capture sequence (e.g., 5'-bait). As seen in FIG. 8 and FIG. 14, TSO t21' does not extend towards the bead, in some embodiments, due to the extra sequence (e.g., the blocking sequence). In some embodiments, the extra sequence acts as an extension blocker.

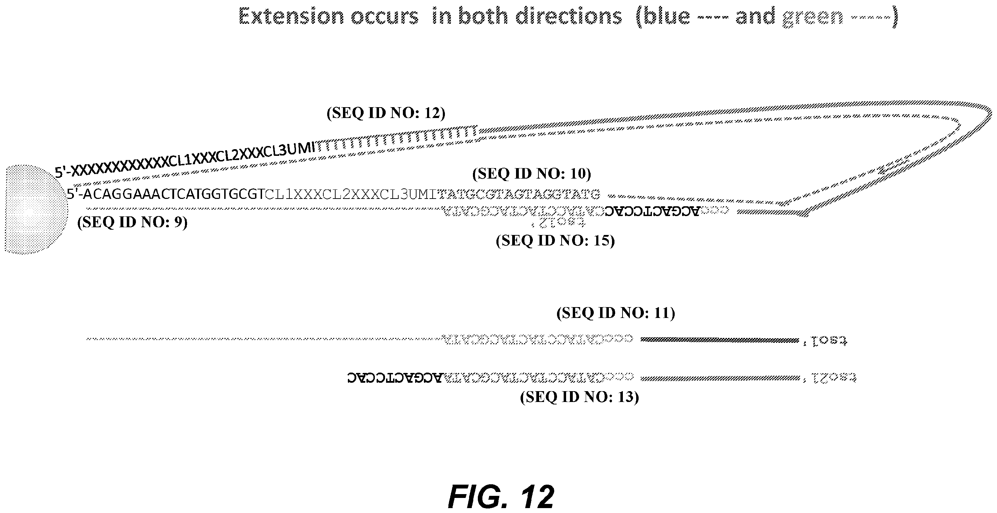

FIG. 9 and FIG. 12 depict a non-limiting schematic illustration of a library preparation method employing the template switch oligonucleotide TSO12 (TATGCGTAGTAGGTATGGTGGAGTCGT rGrGrG; SEQ ID NO: 3). The box comprises the 5' capture sequence (e.g., 5'-bait). Extension is shown occurring in two directions (blue and green dotted lines). In some embodiments, the green extension product (e.g., the extension of the barcoded nucleic acid molecule) can be the source of a high level of unwanted PCR products from the extension reaction (e.g., "pretzeled" products). FIG. 9 and FIG. 12 depict the pretzeled TSO12 product. The purple arrow depicts a specific primer that can be used for specific 5'-VDJ amplification. TSO12 is an example of a TSO that is longer in length but does not lead to a block of extension.

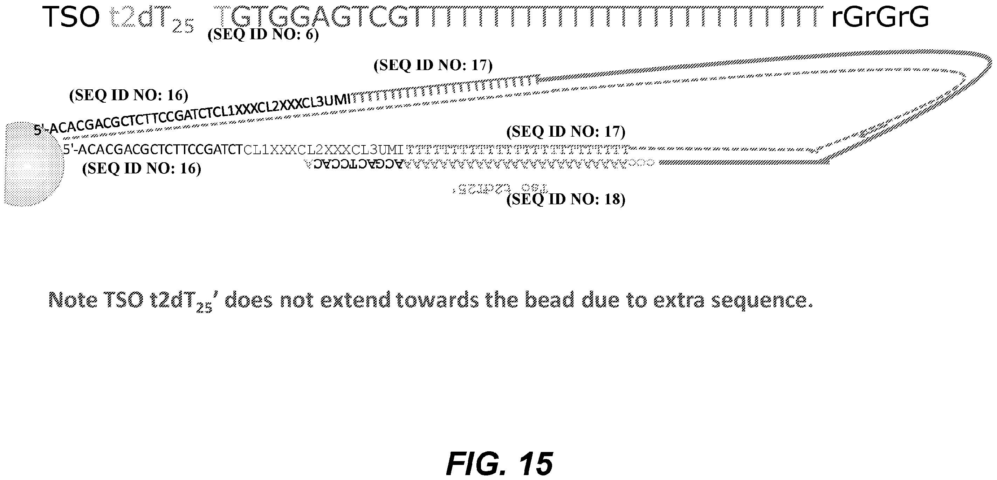

There are provided, in some embodiments, TSO for use with single capture sequence beads (e.g., beads with only a single type of capture sequence on the beads). For example, FIG. 10 and FIG. 15 depict a non-limiting schematic illustration of a library preparation method employing the template switch oligonucleotide TSO t2dT₂₅ (TGTGGAGTCGTTTTTTTTTTTTTTTTTTTTTTTTTT rGrGrG; SEQ ID NO: 6). The box comprises the 5' capture sequence (e.g., 5'-bait). The purple arrow depicts a specific primer that can be used for specific 5'-VDJ amplification. As seen in FIG. 10 and FIG. 15, TSO t2dT25' does not extend towards the bead, in some embodiments, due to the extra sequence (e.g., the blocking sequence). In some embodiments, the extra sequence acts as an extension blocker.

In some embodiments, the blocking sequence is not sufficiently complementary to the oligonucleotide barcode (e.g., the portion of the oligonucleotide barcode 5' of the target-binding region and/or bait sequence). As used herein, the term "sufficiently complementary" can refer to a contiguous nucleic acid base sequence that is capable of hybridizing to another base sequence by hydrogen bonding between a series of complementary bases. Complementary base sequences can be complementary at each position in the oligomer sequence by using standard base pairing (e.g., G:C, A:T or A:U) or can contain one or more residues that are not complementary (including abasic positions), but in which the entire complementary base sequence is capable of specifically hybridizing with another base sequence in appropriate hybridization conditions. Contiguous bases can be at least about 80%, at least about 85%, at least about 90%, at least about 95%, at least about 99%, or 100% complementary to a sequence to which an oligomer is intended to hybridize. Substantially complementary sequences can refer to sequences ranging in percent identity from 100, 99, 98, 97, 96, 95, 94, 93, 92, 91, 90, 89, 88, 87, 86, 85, 84, 83, 82, 81, 80, 75, 70 or less, or any number in between, compared to the reference sequence. A skilled artisan can readily choose appropriate hybridization conditions which can be predicted based on base sequence composition, or be determined by using routine testing (*see e.g.*, Green and Sambrook, Molecular Cloning, A Laboratory Manual, 4th ed. (Cold Spring Harbor Laboratory Press, Cold Spring Harbor, N.Y., 2012)).

The methods and systems described herein can be used with methods and systems using antibodies associated with (e.g., attached to or conjugated with) oligonucleotides (also referred to herein as AbOs or AbOligos). Some embodiments of using AbOs to determine protein expression profiles in single cells and tracking sample origins have been described in US2018/0088112, and US2018/0346970; the content of each is incorporated by reference herein in its entirety. In some embodiments, the method disclosed herein allows V(D)J profiling of T cells and B cells, 3' targeted, 5' targeted, 3' whole transcriptome amplification (WTA), 5' WTA, protein expression profiling with AbO, and/or sample multiplexing on a single experiment. Methods for determining the sequences of a nucleic acid target (e.g., the V(D)J region of an immune receptor) using 5' barcoding and/or 3' barcoding are described in US2020/0109437; the content of which is incorporated herein by reference in its entirety. Systems, methods, compositions, and kits for molecular barcoding on the 5'-end of a nucleic acid target have been described in, for example, US2019/0338278, the content of which is incorporated herein by reference in its entirety. The systems, methods, compositions, and kits for 5'-based gene expression profiling provided herein can, in some embodiments, be employed in concert with the methods to obtain full-length V(D)J information (e.g., by Illumina sequencing on the Rhapsody system) using a combined 5' barcoding and random priming approach described in US20210139970; the content of which is incorporated herein by reference in its entirety. The systems, methods, compositions, and kits for 5'-based gene expression profiling provided herein can, in some embodiments, be employed in concert with random priming and extension (RPE)-based whole transcriptome analysis methods and compositions have been described in U.S. Patent Application No. 16/677,012; the content of which is incorporated herein by reference in its entirety. The systems, methods, compositions, and kits for 5'-based gene expression profiling provided herein can, in some embodiments, be employed in concert with the blocker oligonucleotides described in US20210238661, the content of which is incorporated herein by reference in its entirety. Some embodiments of the compositions and methods disclosed herein comprise a first plurality of oligonucleotide barcodes and a second plurality of oligonucleotide barcodes, which have described in U.S. Patent Application Number 17/336,055, filed on June 1, 2021, entitled "OLIGONUCLEOTIDES AND BEADS FOR 5 PRIME GENE EXPRESSION ASSAY," and content of which is incorporated herein by reference in its entirety. In some embodiments, nucleic acid targets (e.g., mRNAs) are initially barcoded on the 3' end with the first plurality of oligonucleotide barcodes and subsequently barcoded on the 5' end following a template switching reaction and intermolecular hybridization with the first plurality of oligonucleotide barcodes and extension. Methods for manufacturing oligonucleotide barcodes and barcoding particles has been described in, for example, US2015/0299784, WO2015/031691, and Fu et al, PNAS U.S.A. 2011 May 31;108(22):9026-31, the content of these publications is incorporated hereby in its entirety.

### Methods of Barcoding the 5' Ends of Nucleic Acid Targets

High-throughput single-cell RNA-sequencing has transformed the understanding of complex and heterogenous biological samples. However, most methods enable only 3' analysis of the mRNA transcript information, which may limit analysis of splice variants, alternative transcription start sites and highly variable loci due to rearrangement such as the VDJ junction of T cell and B cell receptors and antibodies. Methods for determining the sequences of a nucleic acid target (e.g., the V(D)J region of an immune receptor) using 5' barcoding and/or 3' barcoding are described in U.S. Patent Application Number 16/588,405, filed on September 30, 2019; the content of which is incorporated herein by reference in its entirety. Systems, methods, compositions, and kits for molecular barcoding on the 5'-end of a nucleic acid target have been described in U.S. Patent Application No. 16/588,405, published as U.S. Patent Application Publication No. 2019/0338278, the content of which is incorporated herein by reference in its entirety.

Disclosed herein includes systems, methods, compositions, and kits for attachment of barcodes (e.g., stochastic barcodes) with molecular labels (or molecular indices) to the 5'-ends of nucleic acid targets being barcoded or labeled (e.g., deoxyribonucleic acid molecules, and ribonucleic acid molecules). The 5'-based transcript counting methods disclosed herein can complement, or supplement, for example, 3'-based transcript counting methods (e.g., Rhapsody^{™} assay (Becton, Dickinson and Company (Franklin Lakes, NJ)), Chromium^{™} Single Cell 3' Solution (10X Genomics (San Francisco, CA))). The barcoded nucleic acid targets can be used for sequence identification, transcript counting, alternative splicing analysis, mutation screening, and/or full length sequencing in a high throughput manner. Transcript counting on the 5'-end (5' relative to the target nucleic acid targets being labeled) can reveal alternative splicing isoforms and variants (including, but not limited to, splice variants, single nucleotide polymorphisms (SNPs), insertions, deletions, substitutions.) on, or closer to, the 5'-ends of nucleic acid molecules. In some embodiments, the method can involve intramolecular hybridization.

The methods of the disclosure can be used for identifying VDJ regions of B cell receptors (BCR), T cell receptors (TCR), and antibodies. VDJ recombination, also known as somatic recombination, is a mechanism of genetic recombination in the early stages of immunoglobulin (Ig) (e.g., BCR) and T cell receptor (TCR) production of the immune system. VDJ recombination can nearly randomly combine Variable (V), Diverse (D) and Joining (J) gene segments. Because of its randomness in choosing different genes, it is able to diversely encode proteins to match antigens from bacteria, viruses, parasites, dysfunctional cells such as tumor cells and pollens.

The VDJ region can comprise a large 3 Mb locus comprising variable (V) genes, diversity (D) genes and joining (J) genes. These are the segments that can participate in VDJ recombination. There can be constant genes which may not undergo VDJ recombination. The first event in the VDJ recombination of this locus can be that one of the D genes rearranges to one of the J genes. Following this, one of the V genes can be appended to this DJ rearrangement to form the functional VDJ rearranged gene that then codes for the variable segment of the heavy chain protein. Both of these steps can be catalyzed by recombinase enzymes, which can delete out the intervening DNA.

This recombination process takes place in a stepwise fashion in progenitor B cells to produce the diversity required for the antibody repertoire. Each B cell may only produce one antibody (e.g., BCR). This specificity can be achieved by allelic exclusion such that functional rearrangement of one allele signals to prevent further recombination of the second allele.

In some embodiments, the sample comprises an immune cell. An immune cell can include, for example, T cell, B cell, lymphoid stem cell, myeloid progenitor cell, lymphocyte, granulocyte, B-cell progenitor, T cell progenitor, Natural Killer cell, Tc cell, Th cell, plasma cell, memory cell, neutrophil, eosinophil, basophil, mast cell, monocyte, dendritic cell and/or macrophage, or any combination thereof.

A T cell can be a T cell clone, which can refer to T cells derived from a single T cell or those having identical TCRs. A T cell can be part of a T cell line which can include T cell clones and mixed populations of T cells with different TCRs all of which may recognize the same target (e.g., antigen, tumor, virus). T cells can be obtained from a number of sources, including peripheral blood mononuclear cells, bone marrow, lymph node tissue, spleen tissue, and tumors. T cells can be obtained from a unit of blood collected from a subject, such as using the Ficoll separation. Cells from the circulating blood of an individual can be obtained by apheresis or leukapheresis. The apheresis product can comprise lymphocytes, including T cells, monocytes, granulocytes, B cells, other nucleated white blood cells, red blood cells, and platelets. The cells can be washed and resuspended in media to isolate the cell of interest.

T cells can be isolated from peripheral blood lymphocytes by lysing the red blood cells and depleting the monocytes, for example, by centrifugation through a PERCOLL^{™} gradient. A specific subpopulation of T cells, such as CD28+, CD4+, CDC, CD45RA+, and CD45RO+ T cells, can be further isolated by positive or negative selection techniques. For example, T cells can be isolated by incubation with anti-CD3/anti-CD28 (i.e., 3×28)-conjugated beads, such as DYNABEADS^{®} M-450 CD3/CD28 T, or XCYTE DYNABEADS^{™} for a time period sufficient for positive selection of the desired T cells. Immune cells (e.g., T cells and B cells) can be antigen specific (e.g., specific for a tumor).

In some embodiments, the cell can be an antigen-presenting cell (APC), such as a B cell, an activated B cell from a lymph node, a lymphoblastoid cell, a resting B-cell, or a neoplastic B cell, e.g. from a lymphoma. An APC can refer to a B-cell or a follicular dendritic cell expressing at least one of the BCRC proteins on its surface.

The methods of the disclosure can be used to trace the molecular phenotype of single T cells. Different subtypes of T cells can be distinguished by expression of different molecular markers. T cells express a unique T cell receptor (TCR) from a diverse repertoire of TCRs. In most T cells, the TCR can be composed of a heterodimer of a α and a β chain; each functional chain can be a product of somatic DNA recombination events during T cell development, allowing the expression of over a million different TCRs in a single individual. TCRs can be used to define the identity of individual T cells, allowing for lineage tracing for T cell clonal expansion during an immune response. The immunological methods of the disclosure can be used in a variety of ways, including but not limited to, identifying unique TCRα and TCRβ chain pairing in single T cells, quantifying TCR and marker expression at the single cell level, identifying TCR diversity in an individual, characterizing the TCR repertoire expressed in different T cell populations, determining functionality of the alpha and beta chain alleles of the TCR, and identifying clonal expansion of T cells during immune response.

### T-cell receptor chain pairing

T-cell receptors (TCRs) are recognition molecules present on the surface of T lymphocytes. The T-cell receptors found on the surface of T-cells can be comprised of two glycoprotein subunits which are referred to as the alpha and beta chains. Both chains can comprise a molecular weight of about 40 kDa and possess a variable and a constant domain. The genes which encode the alpha and beta chains can be organized in libraries of V, D and J regions from which the genes are formed by genetic rearrangement. TCRs can recognize antigen which is presented by an antigen presenting cell as a part of a complex with a specific self-molecule encoded by a histocompatibility gene. The most potent histocompatibility genes are known as the major histocompatibility complex (MHC). The complex which is recognized by T-cell receptors, therefore, consists of and MHC/peptide ligand.

In some embodiments, the methods, devices, and systems of the disclosure can be used for T cell receptor sequencing and pairing. The methods, devices, and systems of the disclosure can be used for sequencing T-cell receptor alpha and beta chains, pairing alpha and beta chains, and/or determining the functional copy of T-cell receptor alpha chains. A single cell can be contained in a single partition (e.g., well) with a single solid support (e.g., bead). The cell can be lysed. The bead can comprise a stochastic label that can bind to a specific location within an alpha and/or beta chain of a TCR. The TCR alpha and beta molecules associated with solid support can be subjected to the molecular biology methods of the disclosure, including reverse transcription, amplification, and sequencing. TCR alpha and beta chains that comprise the same cellular label can be considered to be from the same single cell, thereby pairing alpha and beta chains of the TCR.

### Heavy and light chain pairing in antibody repertoires

The methods devices and systems of the disclosure can be used for heavy and light chain pairing of BCR receptors and antibodies. The methods of the present disclosure allow for the repertoire of immune receptors and antibodies in an individual organism or population of cells to be determined. The methods of the present disclosure can aid in determining pairs of polypeptide chains that make up immune receptors. B cells and T cells each express immune receptors; B cells express immunoglobulins and BCRs, and T cells express T cell receptors (TCRs). Both types of immune receptors can comprise two polypeptide chains. Immunoglobulins can comprise variable heavy (VH) and variable light (VL) chains. There can be two types of TCRs: one consisting of an alpha and a beta chain, and one consisting of a delta and a gamma chain. Polypeptides in an immune receptor can comprise constant region and a variable region. Variable regions can result from recombination and end joint rearrangement of gene fragments on the chromosome of a B or T cell. In B cells additional diversification of variable regions can occur by somatic hypermutation.

The immune system has a large repertoire of receptors, and any given receptor pair expressed by a lymphocyte can be encoded by a pair of separate, unique transcripts. Knowing the sequences of pairs of immune receptor chains expressed in a single cell can be used to ascertain the immune repertoire of a given individual or population of cells.

In some embodiments, the methods, devices, and systems of the disclosure can be used for antibody sequencing and pairing. The methods, devices, and systems of the disclosure can be used for sequencing antibody heavy and light chains (e.g., in B cells), and/or pairing the heavy and light chains. A single cell can be contained in a single partition (e.g., well) with a single solid support (e.g., bead). The cell can be lysed.

The bead can comprise a stochastic label that can bind to a specific location within a heavy and/or light chain of an antibody (e.g., in a B cell). The heavy and light chain molecules associated with solid support can be subjected to the molecular biology methods of the disclosure, including reverse transcription, amplification, and sequencing. Antibody heavy and light chains that comprise the same cellular label can be considered to be from the same single cell, thereby pairing heavy and light chains of the antibody.

Disclosed herein include methods for labeling nucleic acid targets in a sample. In some embodiments, the method comprises: contacting copies of a nucleic acid target with a first plurality of oligonucleotide barcodes, wherein each oligonucleotide barcode comprises a first universal sequence, a molecular label, and a target-binding region capable of hybridizing to the nucleic acid target; extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region; hybridizing the complement of the target-binding region of each barcoded nucleic acid molecule with the target-binding region of an oligonucleotide barcode of the first plurality of oligonucleotide barcodes; and extending the 3' ends of oligonucleotide barcodes hybridized to the complement of the target-binding region of the barcoded nucleic acid molecule to generate a first plurality of extended barcoded nucleic acid molecules each comprising a complement of the first molecular label and a second molecular label. The method can comprise: determining the copy number of the nucleic acid target in the sample based on the number of second molecular labels with distinct sequences associated with the first plurality of extended barcoded nucleic acid molecules, or products thereof.

Disclosed herein include methods for determining the numbers of nucleic acid targets in a sample. In some embodiments, the method comprises: contacting copies of a nucleic acid target with a first plurality of oligonucleotide barcodes, wherein each oligonucleotide barcode comprises a first universal sequence, a molecular label, and a target-binding region capable of hybridizing to the nucleic acid target; extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region; hybridizing the complement of the target-binding region of each barcoded nucleic acid molecule with the target-binding region of an oligonucleotide barcode of the first plurality of oligonucleotide barcodes; extending the 3' ends of the oligonucleotide barcodes hybridized to the complement of the target-binding region of the barcoded nucleic acid molecule to generate a first plurality of extended barcoded nucleic acid molecules each comprising a complement of the first molecular label and a second molecular label; and determining the copy number of the nucleic acid target in the sample based on the number of second molecular labels with distinct sequences associated with the first plurality of extended barcoded nucleic acid molecules, or products thereof.

The method can comprise: amplifying the first plurality of extended barcoded nucleic acid molecules to generate a first plurality of single-labeled nucleic acid molecules each comprising the second molecular label, wherein determining the copy number of the nucleic acid target in the sample comprises: determining the copy number of the nucleic acid target in the sample based on the number of second molecular labels with distinct sequences associated with the first plurality of single-labeled nucleic acid molecules.

Disclosed herein include methods for labeling nucleic acid targets in a sample. In some embodiments, the method comprises: contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target; extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target; contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence; and extending: the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence. The method can comprise: determining the copy number of the nucleic acid target in the sample based on: the number of first molecular labels with distinct sequences, second molecular labels with distinct sequences, or a combination thereof, associated with the second plurality of extended barcoded nucleic acid molecules, or products thereof.

Disclosed herein include methods for the copy number of a nucleic acid target in a sample. In some embodiments, the method comprises: contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target; extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target; contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence; extending: the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence; and determining the copy number of the nucleic acid target in the sample based on: the number of first molecular labels with distinct sequences, second molecular labels with distinct sequences, or a combination thereof, associated with the second plurality of extended barcoded nucleic acid molecules, or products thereof.

In some embodiments, determining the copy number of the nucleic acid target comprises determining the copy number of each of a plurality of nucleic acid targets in the sample based on the number of first molecular labels with distinct sequences, second molecular labels with distinct sequences, or a combination thereof, associated with extended barcoded nucleic acid molecules of the second plurality of extended barcoded nucleic acid molecules comprising a sequence of the each of the plurality of nucleic acid targets. In some embodiments, the first universal sequence of each oligonucleotide barcode of the first plurality of oligonucleotide barcodes is 5' of the first molecular label and the target-binding region. In some embodiments, the second universal sequence of each oligonucleotide barcode of the second plurality of oligonucleotide barcodes is 5' of the second molecular label and the bait sequence. In some embodiments, the bait sequence comprises at least 6 nucleotides. In some embodiments, the bait sequence comprises a GC content of about 20% to about 80%.

The method can comprise: amplifying the second plurality of extended barcoded nucleic acid molecules using an amplification primer and a primer comprising the second universal sequence, or a portion thereof, thereby generating a second plurality of single-labeled nucleic acid molecules comprising the sequence of the nucleic acid target, or a portion thereof, wherein determining the copy number of the nucleic acid target in the sample comprises: determining the copy number of the nucleic acid target in the sample based on the number of second molecular labels with distinct sequences associated with the second plurality of single-labeled nucleic acid molecules, or products thereof.

The method can comprise: amplifying the second plurality of extended barcoded nucleic acid molecules using an amplification primer and a primer comprising the first universal sequence, or a portion thereof, thereby generating a third plurality of single-labeled nucleic acid molecules comprising the sequence of the nucleic acid target, or a portion thereof, wherein determining the copy number of the nucleic acid target in the sample comprises: determining the copy number of the nucleic acid target in the sample based on the number of first molecular labels with distinct sequences associated with the third plurality of single-labeled nucleic acid molecules, or products thereof.

In some embodiments, the oligonucleotide barcodes of the first and/or second pluralities of oligonucleotide barcodes comprise a cell label, optionally the cell label is located 5' of the molecular label. In some embodiments, at least a portion of the oligonucleotide barcode the first and/or second pluralities of oligonucleotide barcodes comprises at least one variable sequence, optionally at least 10 of the first and/or second pluralities of oligonucleotide barcodes comprise different sequences at the invariable sequence, optionally at least a portion of the cell label and/or molecular label comprises a variable sequence. In some embodiments, the blocking sequence is located at the 3' end of the first and/or second pluralities of barcoded nucleic acid molecules. In some embodiments, the blocking sequence is configured to be non-complementary to: (i) the region of the oligonucleotide barcode of the first plurality of oligonucleotide barcodes 5' of the target-binding region; and/or (ii) the region of the oligonucleotide barcode of the second plurality of oligonucleotide barcodes 5' of the bait sequence. In some embodiments, the blocking sequence is less than about 50% complementary to: (i) the region of at least 50% of oligonucleotide barcodes of the first plurality of oligonucleotide barcodes 5' of the target-binding region; and/or (ii) the region of at least 50% of the oligonucleotide barcodes of the second plurality of oligonucleotide barcodes 5' of the bait sequence. In some embodiments, the blocking sequence is configured to prevent extension of the 3' ends of the first and/or second pluralities of barcoded nucleic acid molecules. In some embodiments, the blocking sequence reduces the generation of extended barcoded nucleic acid molecules comprising a complement of the first universal sequence.

In some embodiments, in the absence of the blocking sequence, (i) the 3' ends of the second plurality of barcoded nucleic acid molecules hybridized to the bait sequence of the second plurality of oligonucleotide barcodes; and/or (ii) the 3' ends of the first plurality of barcoded nucleic acid molecules hybridized to the target-binding region of the first plurality of oligonucleotide barcodes; are capable of being extended to generate a third plurality of extended barcoded nucleic acid molecules. In some embodiments, the generation of the third plurality of extended barcoded nucleic acid molecules is reduced by at least 10%, by at least 25%, by at least 50%, by at least 80%. by at least 90%, by at least 95%, or by at least 99%, as compared to a comparable method wherein the TSO does not comprise a complement of a blocking sequence.

In some embodiments, at least a portion of an oligonucleotide barcode of the first and/or second pluralities of oligonucleotide barcodes comprises an invariable sequence, optionally each of the first and/or second pluralities of oligonucleotide barcodes comprise the same sequence at the invariable sequence. In some embodiments, the invariable sequence is between about 1-15 nucleotides in length, optionally the invariable sequence is 1 nucleotide in length. In some embodiments, the invariable sequence is located in the cell label, optionally the cell label comprises a first portion of the cell label, a first linker, a second portion of the cell label, a second linker, and a third portion of the cell label, further optionally the invariable sequence is located in the third portion of the cell label. In some embodiments, the blocking sequence comprises a blocker nucleotide, wherein the blocker nucleotide is located at the 3' end of the blocking sequence. In some embodiments, the blocker nucleotide is configured to be non-complementary to the invariable sequence. In some embodiments, the distance between the invariable sequence and the bait sequence is equidistant to the distance between the complement of the bait sequence and the blocker nucleotide. In some embodiments, the distance between the invariable sequence and the target-binding region is equidistant to the distance between the complement of the target-binding region and the blocker nucleotide. In some embodiments, the TSO comprises a sequence selected from SEQ ID NOs: 1-7, or a sequence that exhibits at least about 50% identity to a sequence selected from SEQ ID NOs: 1-7. In some embodiments, the blocking sequence comprises at least 4 contiguous bases of any one of SEQ ID NOs: 1-7. In some embodiments, the complement of the blocking sequence is 5' of the bait sequence in the TSO. In some embodiments, the complement of the blocking sequence is 5' of the target-binding region, or portion thereof, in the TSO.

In some embodiments, Template Switch Efficiency is reduced less than 40%, less than 20%, less than 10%, or less than 5%, as compared to a comparable method wherein the TSO does not comprise a complement of a blocking sequence. In some embodiments, the method further comprises the addition of blocker oligonucleotides before or during one or more extension steps. In some embodiments, the method does not comprise the addition of blocker oligonucleotides. In some embodiments, the complement of the blocking sequence comprises a reverse complementary sequence of the blocking sequence, and/or a complementary sequence of the blocking sequence.

In some embodiments, hybridizing the complement of the target-binding region of a barcoded nucleic acid molecule with the target-binding region of an oligonucleotide barcode of the first plurality of oligonucleotide barcodes comprises intermolecular hybridization of the complement of the target-binding region of a barcoded nucleic acid molecule with the target-binding region of an oligonucleotide barcode of the first plurality of oligonucleotide barcodes. The method can comprise: denaturing the first plurality of barcoded nucleic acid molecules prior to hybridizing the complement of the target-binding region of each barcoded nucleic acid molecule with the target-binding region of an oligonucleotide barcode of the first plurality of oligonucleotide barcodes. The method can comprise: denaturing the first and/or second plurality of extended barcoded nucleic acid molecules prior to amplifying the first and/or second plurality of extended barcoded nucleic acid molecules.

In some embodiments, determining the copy number of the nucleic acid target comprises determining the copy number of each of the plurality of nucleic acid targets in the sample based on the number of second molecular labels with distinct sequences associated with single-labeled nucleic acid molecules of the first plurality of single-labeled nucleic acid molecules comprising a sequence of the each of the plurality of nucleic acid targets. In some embodiments, the sequence of the each of the plurality of nucleic acid targets comprises a subsequence of the each of the plurality of nucleic acid targets. In some embodiments, the sequence of the nucleic acid target in the first plurality of barcoded nucleic acid molecules and/or second plurality of barcoded nucleic acid molecules comprises a subsequence of the nucleic acid target.

In some embodiments, the complement of the target-binding region is complementary to a portion of the target-binding region. In some embodiments, the target-binding region comprises a gene-specific sequence, and/or a poly(dT) sequence. In some embodiments, the second molecular label is a different from the first molecular label, and wherein the second molecular label is not a complement of the first molecular label. In some embodiments, the first and/or second plurality of extended barcoded nucleic acid molecules each comprise the sequence of the nucleic acid target. In some embodiments, the nucleic acid target comprises mRNA, and wherein the first and/or second plurality of extended barcoded nucleic acid molecules each comprise the sequence of the sense strand of the nucleic acid target. In some embodiments, the reverse transcriptase is capable of terminal transferase activity. In some embodiments, the template switch oligonucleotide comprises one or more 3' ribonucleotides, optionally three 3' ribonucleotides, and further optionally the 3' ribonucleotides comprise guanine. In some embodiments, the reverse transcriptase comprises a viral reverse transcriptase, optionally the viral reverse transcriptase is a murine leukemia virus (MLV) reverse transcriptase or a Moloney murine leukemia virus (MMLV) reverse transcriptase.

In some embodiments, the sample comprises a single cell, optionally an immune cell, and further optionally a B cell or a T cell. In some embodiments, the sample comprises a plurality of cells, a plurality of single cells, a tissue, a tumor sample, or any combination thereof. In some embodiments, the single cell comprises a circulating tumor cell. In some embodiments, the first universal sequence is 5' of the molecular label and the target-binding region. In some embodiments, amplifying the first plurality of extended barcoded nucleic acid molecules to generate a first plurality of single-labeled nucleic acid molecules comprises using a primer capable of hybridizing to the first universal sequence, and an amplification primer.

In some embodiments, the amplification primer is a target-specific primer, and optionally the target-specific primer specifically hybridizes to an immune receptor, a constant region of an immune receptor, a variable region of an immune receptor, a diversity region of an immune receptor, and/or the junction of a variable region and diversity region of an immune receptor. In some embodiments, the immune receptor is a T cell receptor (TCR) and/or a B cell receptor (BCR) receptor, and optionally the TCR comprises TCR alpha chain, TCR beta chain, TCR gamma chain, TCR delta chain, or any combination thereof; and the BCR receptor comprises BCR heavy chain and/or BCR light chain. In some embodiments, the amplification primer specifically binds the extended barcoded nucleic acid molecules each comprising a complement of the first molecular label and a second molecular label. In some embodiments, the amplification primer does not bind extended barcoded nucleic acid molecules comprising the first molecular label. In some embodiments, the amplification primer comprises the complement of the nucleic acid target. In some embodiments, the nucleic acid target comprises mRNA, and wherein the amplification primer comprises the sequence of the anti-sense strand of the nucleic acid target. In some embodiments, extending the 3' ends of the oligonucleotide barcodes comprises extending the 3' ends of the oligonucleotide barcodes using a mesophilic DNA polymerase, a thermophilic DNA polymerase, a psychrophilic DNA polymerase, or any combination thereof. In some embodiments, extending the 3' ends of the oligonucleotide barcodes comprises extending the 3' ends of the oligonucleotide barcodes using a DNA polymerase lacking at least one of 5' to 3' exonuclease activity and 3' to 5' exonuclease activity, and optionally the DNA polymerase comprises a Klenow Fragment.

The method can comprise: obtaining sequence information of the first and/or second plurality of extended barcoded nucleic acid molecules, or products thereof. The method can comprise: obtaining sequence information of one or more of the first, second, and third pluralities of single-labeled nucleic acid molecules, or products thereof, In some embodiments, obtaining the sequence information comprises attaching sequencing adaptors to the first and/or second plurality of extended barcoded nucleic acid molecules, or products thereof. In some embodiments, obtaining the sequence information comprises attaching sequencing adaptors to the first, second, and/or third pluralities of single-labeled nucleic acid molecules, or products thereof. In some embodiments, obtaining sequence information comprises: obtaining sequencing data comprising a plurality of sequencing reads of one or more of the first and/or second plurality of extended barcoded nucleic acid molecules, or products thereof, and/or the first, second, and third pluralities of single-labeled nucleic acid molecules, or products thereof, wherein each of the plurality of sequencing reads comprise (1) a cell label sequence, (2) a molecular label sequence, and/or (3) a subsequence of the nucleic acid target. In some embodiments, less than 40%, less than 20%, less than 10%, or less than 5%, of the total sequencing reads are derived from the third plurality of extended barcoded nucleic acid molecules, or products thereof. In some embodiments, the number of sequencing reads derived from the third plurality of extended barcoded nucleic acid molecules, or products thereof, is reduced at least about 2-fold as compared to a comparable method wherein the TSO does not comprise a complement of a blocking sequence.

In some embodiments, obtaining the sequence information comprises obtaining the sequence information of the BCR light chain and the BCR heavy chain of a single cell. In some embodiments, the sequence information of the BCR light chain and the BCR heavy chain comprises the sequence of the complementarity determining region 1 (CDR1), the CDR2, the CDR3, or any combination thereof, of the BCR light chain and/or the BCR heavy chain. The method can comprise: pairing the BCR light chain and the BCR heavy chain of the single cell based on the obtained sequence information. In some embodiments, the sample comprises a plurality of single cells, the method comprising pairing the BCR light chain and the BCR heavy chain of at least 50% of said single cells based on the obtained sequence information.

In some embodiments, obtaining the sequence information comprises obtaining the sequence information of the TCR alpha chain and the TCR beta chain of a single cell. In some embodiments, the sequence information of the TCR alpha chain and the TCR beta chain comprises the sequence of the complementarity determining region 1 (CDR1), the CDR2, the CDR3, or any combination thereof, of the TCR alpha chain and/or the TCR beta chain. The method can comprise: pairing the TCR alpha chain and the TCR beta chain of the single cell based on the obtained sequence information. In some embodiments, the sample comprises a plurality of single cells, the method comprising pairing the TCR alpha chain and the TCR beta chain of at least 50% of said single cells based on the obtained sequence information.

Obtaining the sequence information can comprise obtaining the sequence information of the TCR gamma chain and the TCR delta chain of a single cell. In some embodiments, the sequence information of the TCR gamma chain and the TCR delta chain comprises the sequence of the complementarity determining region 1 (CDR1), the CDR2, the CDR3, or any combination thereof, of the TCR gamma chain and/or the TCR delta chain. The method can comprise: pairing the TCR gamma chain and the TCR delta chain of the single cell based on the obtained sequence information. In some embodiments, the sample comprises a plurality of single cells, the method comprising pairing the TCR gamma chain and the TCR delta chain of at least 50% of said single cells based on the obtained sequence information.

In some embodiments, the complement of the target-binding region comprises the reverse complementary sequence of the target-binding region and/or the complementary sequence of the target-binding region. In some embodiments, the complement of the molecular label comprises a reverse complementary sequence of the molecular label, and/or a complementary sequence of the molecular label. In some embodiments, the first and/or second plurality of barcoded nucleic acid molecules comprises barcoded deoxyribonucleic acid (DNA) molecules and/or barcoded ribonucleic acid (RNA) molecules. In some embodiments, the target-binding region comprises an oligo dT sequence, a random sequence, a target-specific sequence, or a combination thereof. In some embodiments, the target-binding region comprises a poly(dT) region, and wherein the nucleic acid target comprises a poly(dA) region. In some embodiments, the sample comprises a plurality of cells, a plurality of single cells, a tissue, a tumor sample, or any combination thereof. In some embodiments, the nucleic acid target comprises a nucleic acid molecule, and optionally the nucleic acid molecule comprises ribonucleic acid (RNA), messenger RNA (mRNA), microRNA, small interfering RNA (siRNA), RNA degradation product, RNA comprising a poly(A) tail, or any combination thereof. In some embodiments, the mRNA encodes an immune receptor. In some embodiments, the nucleic acid target comprises a cellular component binding reagent. In some embodiments, the nucleic acid molecule is associated with the cellular component binding reagent. The method can comprise: dissociating the nucleic acid molecule and the cellular component binding reagent.

In some embodiments, at least 10 of the first plurality of oligonucleotide barcodes comprise different molecular label sequences. In some embodiments, each molecular label of the plurality of oligonucleotide barcodes comprises at least 6 nucleotides. In some embodiments, the first plurality of oligonucleotide barcodes are associated with a solid support, and optionally the first plurality of oligonucleotide barcodes associated with the same solid support each comprise an identical sample label, and further optionally each sample label of the first plurality of oligonucleotide barcodes comprises at least 6 nucleotides. In some embodiments, the first plurality of oligonucleotide barcodes each comprise a cell label, and optionally each cell label of the first plurality of oligonucleotide barcodes comprises at least 6 nucleotides. In some embodiments, oligonucleotide barcodes associated with the same solid support comprise the same cell label. In some embodiments, oligonucleotide barcodes associated with different solid supports comprise different cell labels. In some embodiments, the first and/or second plurality of extended barcoded nucleic acid molecules each comprises a cell label and a complement of the cell label, and optionally the complement of the cell label comprises a reverse complementary sequence of the cell label and/or a complementary sequence of the cell label.

The method can comprise: extending the first plurality of oligonucleotide barcodes hybridized to the copies of the nucleic acid target in the presence of one or more of ethylene glycol, polyethylene glycol, 1,2- propanediol, dimethyl sulfoxide (DMSO), glycerol, formamide, 7-deaza-GTP, acetamide, tetramethylammonium chloride salt, betaine, or any combination thereof. In some embodiments, at least 10 of the first and second pluralities of oligonucleotide barcodes comprise different molecular label sequences, optionally each molecular label of the first and second pluralities of oligonucleotide barcodes comprises at least 6 nucleotides. In some embodiments, the first and second pluralities of oligonucleotide barcodes are associated with a solid support. In some embodiments, the first and second pluralities of oligonucleotide barcodes associated with the same solid support each comprise an identical sample label, optionally each sample label of the first and second pluralities of oligonucleotide barcodes comprises at least 6 nucleotides. In some embodiments, the first and second pluralities of oligonucleotide barcodes each comprise a cell label, optionally each cell label of the first and second pluralities of oligonucleotide barcodes comprises at least 6 nucleotides. In some embodiments, (a) oligonucleotide barcodes of the first and second pluralities of oligonucleotide barcodes associated with the same solid support comprise the same cell label; and/or (b) oligonucleotide barcodes of the first and second pluralities of oligonucleotide barcodes associated with different solid supports comprise different cell labels.

In some embodiments, the extended barcoded nucleic acid molecules of the first and second pluralities of extended barcoded nucleic acid molecules each comprises a cell label and a complement of the cell label, optionally the complement of the cell label comprises a reverse complementary sequence of the cell label, or a complementary sequence of the cell label.. In some embodiments, at least one oligonucleotide barcode of the first and second pluralities of oligonucleotide barcodes is immobilized or partially immobilized on the synthetic particle, or at least one oligonucleotide barcode of the first and second pluralities of oligonucleotide barcodes is enclosed or partially enclosed in the synthetic particle. In some embodiments, the solid support comprises a synthetic particle or a planar surface.

In some embodiments, the sample comprises a single cell, the method comprising associating a synthetic particle comprising the plurality of the oligonucleotide barcodes with the single cell in the sample. The method can comprise: lysing the single cell after associating the synthetic particle with the single cell, and optionally lysing the single cell comprises heating the sample, contacting the sample with a detergent, changing the pH of the sample, or any combination thereof. In some embodiments, the synthetic particle and the single cell are in the same well. In some embodiments, the synthetic particle and the single cell are in the same droplet. In some embodiments, at least one of the first plurality of oligonucleotide barcodes is immobilized or partially immobilized on the synthetic particle, or the at least one of the first plurality of oligonucleotide barcodes is enclosed or partially enclosed in the synthetic particle.

In some embodiments, the synthetic particle is disruptable. In some embodiments, the synthetic particle comprises a bead, and optionally the bead comprises a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof; a material selected from polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof; or a disruptable hydrogel particle.

In some embodiments, each oligonucleotide barcode of the first and second pluralities of oligonucleotide barcodes comprises a linker functional group, the synthetic particle comprises a solid support functional group, and the support functional group and the linker functional group are associated with each other, and optionally the linker functional group and the support functional group are individually selected from C6, biotin, streptavidin, primary amine(s), aldehyde(s), ketone(s), and any combination thereof. In some embodiments, each of the first plurality of oligonucleotide barcodes comprises a linker functional group, the synthetic particle comprises a solid support functional group, and the support functional group and the linker functional group are associated with each other, and optionally the linker functional group and the support functional group are individually selected from C6, biotin, streptavidin, primary amine(s), aldehyde(s), ketone(s), and any combination thereof.

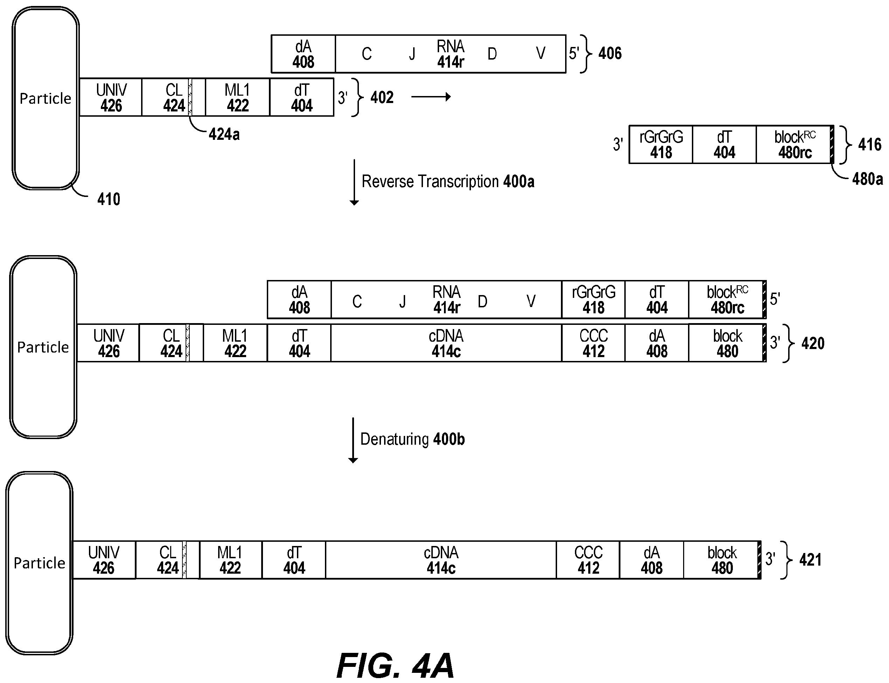

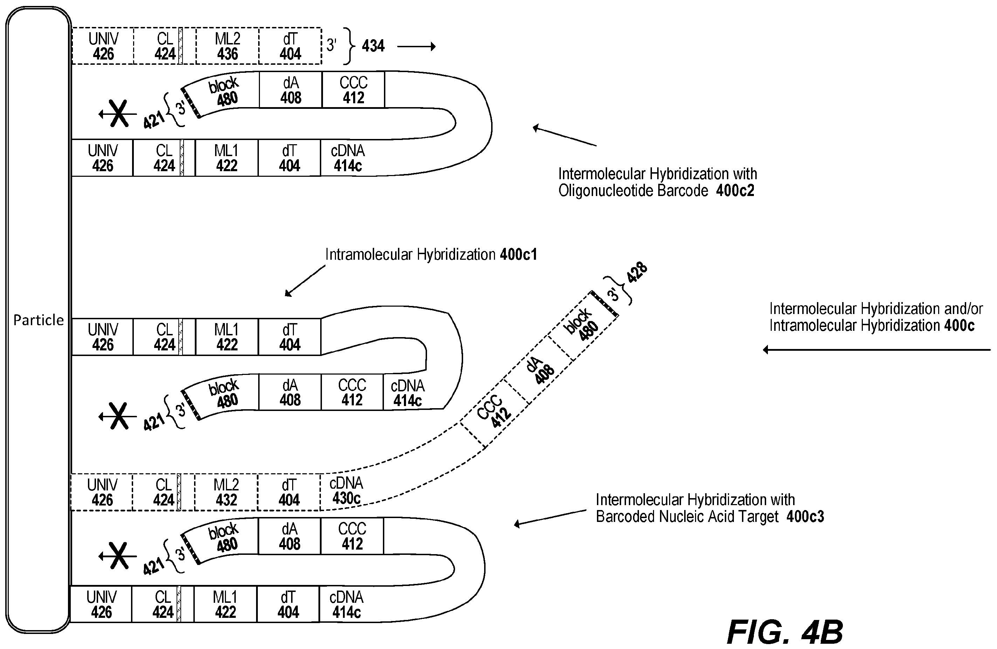

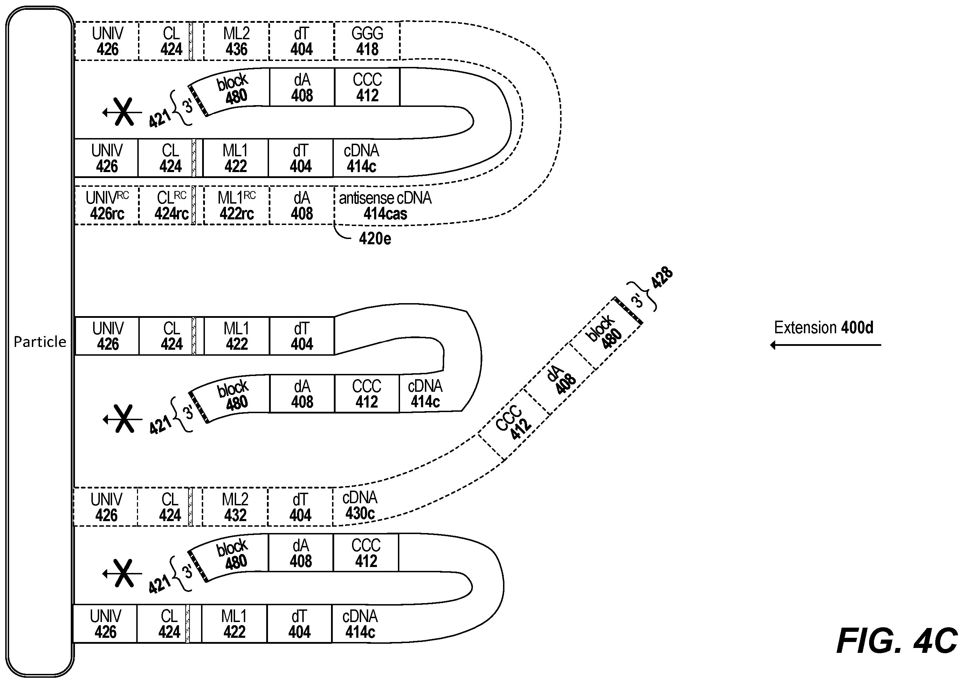

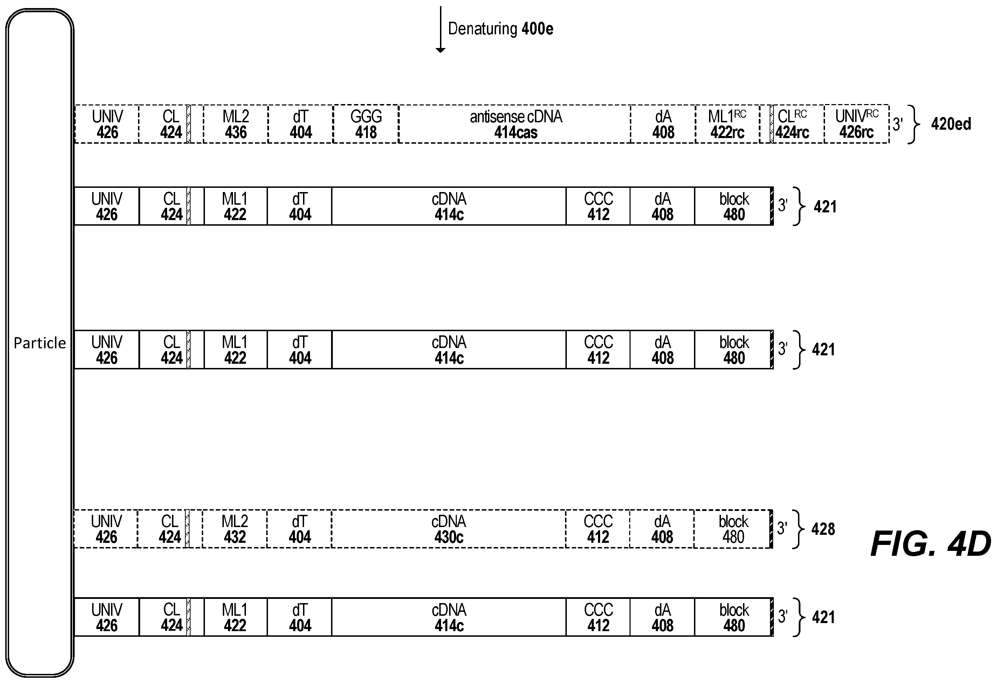

FIGS. 4A-4E show schematic illustrations of non-limiting exemplary workflows of determining the sequences of a nucleic acid target (e.g., the V(D)J region of an immune receptor) using 5' barcoding and the TSO disclosed herein. A barcode (e.g., a stochastic barcode, an oligonucleotide barcode **402)** can comprise a target binding region (e.g., a poly(dT) **404)** that can bind to nucleic acid targets (e.g., poly-adenylated RNA transcripts **406)** via a poly(dA) tail **408,** or other nucleic acid targets, for labeling or barcoding (e.g., unique labeling). The target-binding region can comprise a gene-specific sequence, an oligo(dT) sequence, a random multimer, or any combination thereof. In some embodiments the barcode is associated with a solid support (e.g., a particle **410).** A plurality of barcodes **402** can be associated with particle **410.** In some embodiments, the particle is a bead. The bead can be a polymeric bead, for example a deformable bead or a gel bead, functionalized with barcodes or stochastic barcodes (such as gel beads from 10X Genomics (San Francisco, CA)). In some implementation, a gel bead can comprise a polymer-based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

FIG. 4A depicts a non-limiting exemplary embodiment of reverse transcription reaction **400a.** During reverse transcription **400a,** upon reaching the end of the oligonucleotide barcode **402,** the terminal transferase activity of an enzyme (e.g., a reverse transcriptase, such as a Moloney murine leukemia virus (MMLV)) adds a few additional nucleotides (e.g., deoxycytidine, CCC **412)** to the 3' end of the newly synthesized cDNA sequence strand **414c** (the antisense sequence of RNA sequence **414r).** These CCC bases **412** can function as an anchoring site of the template switch oligonucleotide (e.g., template switching oligonucleotide) **416,** which comprises a sequence complementary to the tailed sequence (e.g., rGrGrG **418).** The template switch oligonucleotide **416** can comprise a complement of a blocking sequence 480rc and at least part of the target binding region **404.** Upon base pairing between the rGrGrG **418** and the appended deoxycytidine stretch **412,** the enzyme "switches" template strands, from oligonucleotide barcode **402** to the template switch oligonucleotide **416,** and continues replication to the 5' end of the template switch oligonucleotide **416.** Thus, the resulting first strand labelled cDNA (e.g., barcoded nucleic acid molecule **420)** contains a reverse complement sequence of the template switch oligonucleotide **416** and thus can comprise the complement (e.g., reverse complement) of the target binding region (e.g., poly(dA) **408)** and the blocking sequence **480.** The blocking sequence can comprise a blocking nucleotide **480a.** The barcoded nucleic acid molecule **420** can comprise cDNA **414c** (the reverse complementary sequence of RNA sequence **414r).** The reaction can be performed in the presence of one or more additives configured to reduce secondary structure (e.g., ethylene glycol). The barcoded nucleic acid molecule **420** can also comprise a number of labels. The oligonucleotide barcode **402** can include first molecular label (ML1) **422** and a sample label (e.g, partition label, cell label (CL) **424)** for labeling the transcripts **406** and tracking sample origins of the RNA transcripts **406** (or nucleic acid targets, such as for example, antibody oligonucleotides, whether associated with antibodies or have dissociated from antibodies), respectively, along with one or more additional sequences flanking the first molecular label **422** /cell label **424** region of each barcode **402** for subsequent reactions, such as, for example, a first universal sequence **426** (e.g., Read 1 sequence). The cell label can comprise an invariable sequence the blocking nucleotide is incapable of hybridizing to. The repertoire of sequences of the molecular labels in the oligonucleotide barcodes per sample can be sufficiently large for stochastic labeling of RNA transcripts. In some embodiments, the sample label is a partition label. In some embodiments, the sample label is a cell label. The barcoded nucleic acid molecule **420** can undergo a denaturing step **400b** (e.g., denaturing), thereby generating single-stranded barcoded nucleic acid molecule **421.**

In some embodiments, the first molecular label is hybridized to the second molecular label after extending the 3'-ends of the plurality of barcoded nucleic acid molecules. In some embodiments, the extended barcoded nucleic acid molecules each comprise the first molecular label, the second molecular label, the target-binding region, and the complement of the target-binding region. In some embodiments, the complement of the target-binding region is complementary to a portion of the target-binding region. In some embodiments, the target-binding region comprises a gene-specific sequence. In some embodiments, the target-binding region comprises a poly(dT) sequence.

The term "template switching" can refer to the ability of a reverse transcriptase to switch from an initial nucleic acid sequence template to the 3' end of a new nucleic acid sequence template having little or no complementarity to the 3' end of the nucleic acid synthesized from the initial template. An example of template switching is the ability of a reverse transcriptase to switch from an initial nucleic acid sequence template/primer substrate to the 3' end of a new nucleic acid sequence template having little or no complementary to the 3' end of the nucleic acid primer strand. Template switching allows, e.g., a DNA copy to be prepared using a reverse transcriptase that switches from an initial nucleic acid sequence template to the 3' end of a new nucleic acid sequence template having little or no complementarity to the 3' end of the DNA synthesized from the initial template, thereby allowing the synthesis of a continuous product DNA that directly links an adaptor sequence to a target oligonucleotide sequence without ligation. Template switching can comprise ligation of adaptor, homopolymer tailing (e.g., polyadenylation), random primer, or an oligonucleotide that the polymerase can associate with. In any of the above-mentioned embodiments, template switching may be used to introduce a target-binding region or the complement thereof.

In some embodiments, the reverse transcriptase is capable of terminal transferase activity. In some embodiments, the template switch oligonucleotide comprises one or more 3' ribonucleotides. In some embodiments, the template switch oligonucleotide comprises three 3' ribonucleotides. In some embodiments, the 3' ribonucleotides comprise guanine. In some embodiments, the reverse transcriptase comprises a viral reverse transcriptase. In some embodiments, the viral reverse transcriptase is a murine leukemia virus (MLV) reverse transcriptase. In some embodiments, the viral reverse transcriptase is a Moloney murine leukemia virus (MMLV) reverse transcriptase.

The complement of a target-binding region can comprise the reverse complementary sequence of the target-binding region or can comprise the complementary sequence of the target-binding region. The complement of a molecular label can comprise a reverse complementary sequence of the molecular label or can comprise a complementary sequence of the molecular label. In some embodiments, the plurality of barcoded nucleic acid molecules can comprise barcoded deoxyribonucleic acid (DNA) molecules and/or barcoded ribonucleic acid (RNA) molecules. In some embodiments, the nucleic acid target comprises a nucleic acid molecule (e.g, ribonucleic acid (RNA), messenger RNA (mRNA), microRNA, small interfering RNA (siRNA), RNA degradation product, RNA comprising a poly(A) tail, or any combination thereof). In some embodiments, the mRNA encodes an immune receptor. The nucleic acid target can comprise a cellular component binding reagent. In some embodiments, the nucleic acid molecule is associated with the cellular component binding reagent. The method can comprise dissociating the nucleic acid molecule and the cellular component binding reagent. In some embodiments, at least 10 of the plurality of oligonucleotide barcodes comprise different molecular label sequences. Each molecular label of the plurality of oligonucleotide barcodes can comprise at least 6 nucleotides.

In some embodiments, the plurality of oligonucleotide barcodes are associated with a solid support. The plurality of oligonucleotide barcodes associated with the same solid support can each comprise an identical sample label. Each sample label of the plurality of oligonucleotide barcodes can comprise at least 6 nucleotides. The plurality of oligonucleotide barcodes can each comprise a cell label. Each cell label of the plurality of oligonucleotide barcodes can comprise at least 6 nucleotides. Oligonucleotide barcodes associated with the same solid support can comprise the same cell label. Oligonucleotide barcodes associated with different solid supports can comprise different cell labels. The plurality of extended barcoded nucleic acid molecules can each comprise a cell label and a complement of the cell label. The complement of the cell label can comprise a reverse complementary sequence of the cell label or a complementary sequence of the cell label. The method can comprise extending the plurality of oligonucleotide barcodes hybridized to the copies of the nucleic acid target in the presence of one or more of ethylene glycol, polyethylene glycol, 1,2-propanediol, dimethyl sulfoxide (DMSO), glycerol, formamide, 7-deaza-GTP, acetamide, tetramethylammonium chloride salt, betaine, or any combination thereof. In some embodiments, the solid support can comprise a synthetic particle. In some embodiments, the solid support can comprise a planar surface.

The sample can comprise a single cell, and the method can comprise associating a synthetic particle comprising the plurality of the oligonucleotide barcodes with the single cell in the sample. The method can comprise lysing the single cell after associating the synthetic particle with the single cell. Lysing the single cell can comprise heating the sample, contacting the sample with a detergent, changing the pH of the sample, or any combination thereof. In some embodiments, the synthetic particle and the single cell are in the same well. In some embodiments, the synthetic particle and the single cell are in the same droplet. In some embodiments, at least one of the plurality of oligonucleotide barcodes is immobilized on the synthetic particle. In some embodiments, at least one of the plurality of oligonucleotide barcodes is partially immobilized on the synthetic particle. At least one of the plurality of oligonucleotide barcodes can be enclosed in the synthetic particle. In some embodiments, at least one of the plurality of oligonucleotide barcodes is partially enclosed in the synthetic particle. In some embodiments, the synthetic particle is disruptable. The synthetic particle can comprise a bead. The bead can comprise a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof. The synthetic particle can comprise a material selected from polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof. In some embodiments, the synthetic particle can comprise a disruptable hydrogel particle. Each of the plurality of oligonucleotide barcodes can comprise a linker functional group, the synthetic particle can comprise a solid support functional group, and/or the support functional group and the linker functional group can be associated with each other. In some embodiments, the linker functional group and the support functional group are individually selected from C6, biotin, streptavidin, primary amine(s), aldehyde(s), ketone(s), and any combination thereof.

The workflow can comprise intramolecular and/or inter molecular hybridization **400c** of the single-stranded barcoded nucleic acid molecule **421.** In some embodiments, multiple types of hybridization reaction(s) **400c** (e.g., **400c1, 400c2,** and/or **400c3)** can occur. The workflow can comprise an extension reaction **400d** following the hybridization reaction(s) **400c** (e.g., **400c1, 400c2,** and/or **400c3).** In some embodiments, the hybridization and/or extension reactions are performed in the presence of a high salt buffer and/or PEG. In some embodiments, extension is performed using a DNA polymerase lacking at least one of 5' to 3' exonuclease activity and 3' to 5' exonuclease activity (e.g., a Klenow Fragment). The DNA polymerase lacking at least one of 5' to 3' exonuclease activity and 3' to 5' exonuclease activity can comprise a mesophilic DNA polymerase, a thermophilic DNA polymerase, a psychrophilic DNA polymerase, or any combination thereof.

Some embodiments of the methods and compositions provided herein improve reduce or prevent undesired products produced during 3' and/or 5' expression profiling of the V(D)J region of immune receptors (e.g., extended barcoded nucleic acid molecules comprising a complement of the first universal sequence). The workflow can proceed as shown in FIG. 4A-4E. The blocking sequence can configured to prevent extension of the 3' ends of the barcoded nucleic acid molecules as shown (e.g., due to lack of hybridizing to the portion of the oligonucleotide barcode 5' of the dT 404).

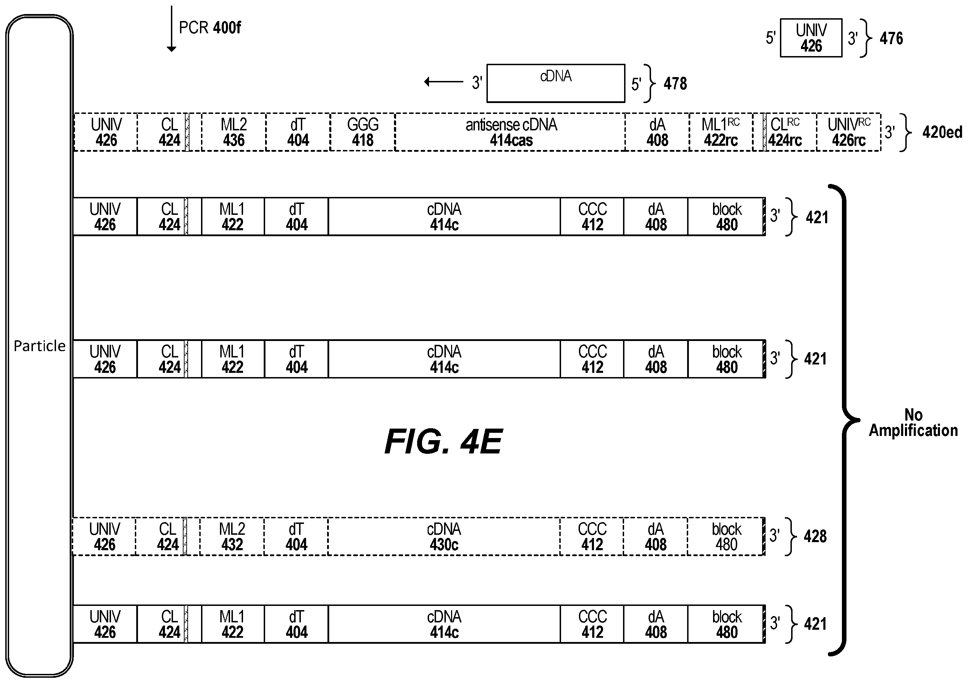

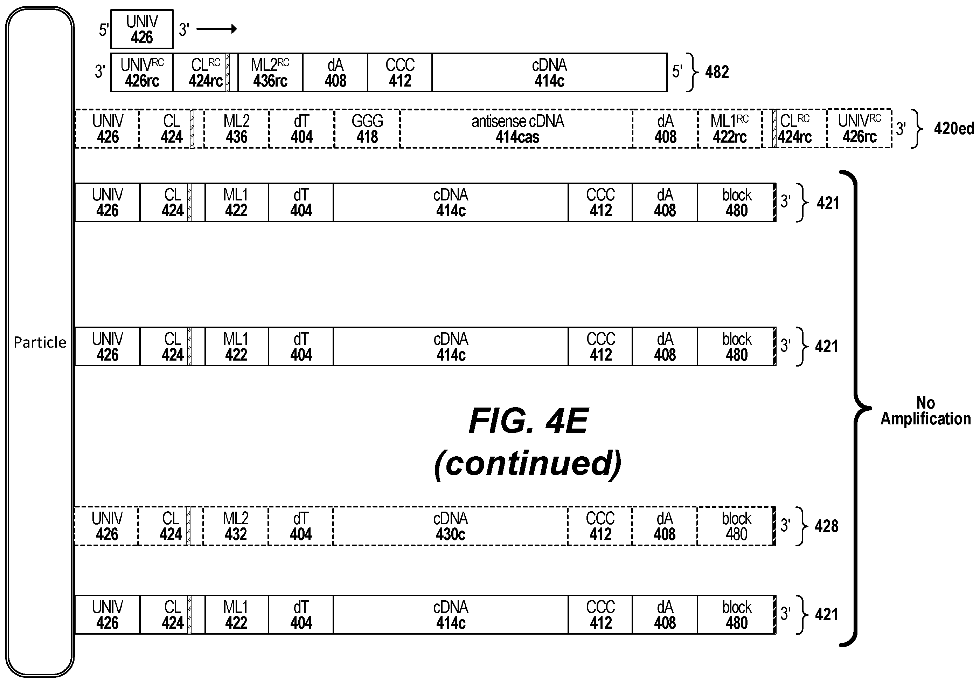

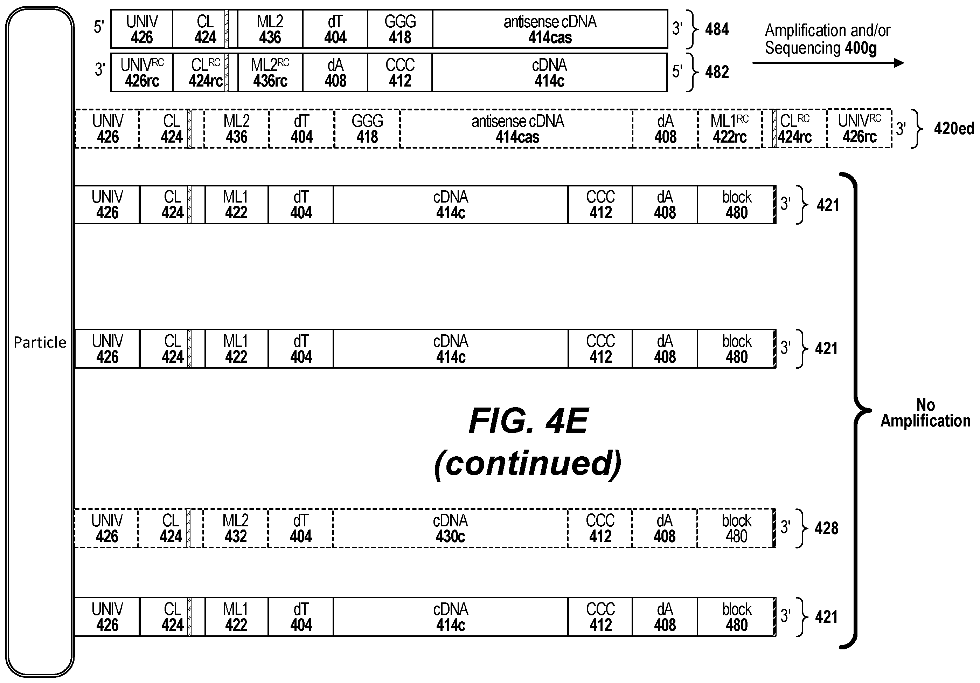

Single-stranded extended barcoded nucleic acid molecule **420ed** can serve as a template for one or more amplification reactions (e.g., PCR), such as, for example, the non-limiting exemplary amplification scheme depicted in FIG. 4E. The amplification(s) can comprise target-specific (e.g., gene-specific) cDNA amplification. For example, single-stranded extended barcoded nucleic acid molecule **420ed** can undergo a first round of amplification ("PCR1") **400f** employing a universal oligonucleotide primer **476** comprising a sequence of the first universal sequence **426** and a target-specific primer **478.** The target-specific primer **478** can comprise a second universal sequence (e.g., Read 2 sequence, a universal PCR handle). The target-specific primer **478** can comprise the complement of the nucleic acid target. The target-specific primer **478** can bind the constant region, variable region, diversity region, and/or junction region of an immune receptor. In embodiments where the nucleic acid target comprises mRNA, and target-specific primer **478** can comprise the sequence of the anti-sense strand of the nucleic acid target (e.g., cDNA). The target-specific primer **478** can be capable of specifically binding single-stranded extended barcoded nucleic acid molecule **420ed** and being extended to generate product **482.** Product **482** can comprise a reverse complement of the first universal sequence **426rc.** The target-specific primer **478** can be capable of specifically binding product **482** and being extended to generate product **484.** The PCR1 product can comprise the second molecular label **436** and the cell label **424.** The double-stranded DNA molecule comprising **482 and 484** (e.g., the PCR1 product) can subject to additional downstream reactions **400g** (priming and extension reactions, amplification reactions, and/or sequencing reactions as disclosed herein. For example, in some embodiments, the workflow comprises a second round of amplification ("PCR2") employing a universal oligonucleotide primer and a nested target-specific primer. The universal oligonucleotide primer and/or the nested target-specific primer can add sequencing adapter(s) to the PCR2 product via overhang(s) in the primer(s). The workflow can comprise library amplification ("Index PCR") of the PCR2 products. Index PCR can add sequencing adapters (e.g., P5 and P7) and sample index (e.g., i5, i7) via overhangs in sequencing library amplification primers. Index PCR amplicons can be sequenced and subjected to downstream methods of the disclosure. Sequencing using 150 bp x 2 sequencing can reveal the cell label, unique molecular label and/or gene (or a partial sequence of the gene) on read 1, the gene (or a partial sequence of the gene) on read 2, and the sample index on index 1 read and/or index 2 read. PCR1, PCR2, and/or PCR3 can comprise 1-30 cycles (e.g., 15 cycles). In some embodiments the workflow comprises multiplex PCR employing a panel of target-specific PCR1 primers and/or a panel of target-specific PCR2 primers. In some embodiments, the targets comprise BCRs, TCRs, and/or immune-related transcripts. In some embodiments, 3' and/or 5' expression profiling of the V(D)J region of an immune receptor can be performed. In some embodiments, both phenotypic markers and immune receptor V(D)J sequence(s) of T cells and/or B cells in single cell platforms can be investigated. In some embodiments, both the 3' and 5' information of their transcripts can be captured in a single experiment. The method disclosed herein can allow V(D)J detection of both T cells and B cells (e.g., hypermutation).

The TSO provided here can, in some embodiments, be employed in concert with the methods to obtain full-length V(D)J information (e.g., by Illumina sequencing on the Rhapsody system) using a combined 5' barcoding and random priming approach described in U.S. Patent Application Number 17/091,639, filed on November 6, 2020 entitled "USING RANDOM PRIMING TO OBTAIN FULL-LENGTH V(D)J INFORMATION FOR IMMUNE REPERTOIRE SEQUENCING", the content of which is incorporated herein by reference in its entirety. There are provided, in some embodiments, methods of employing 5' barcoding, random priming and extension, and the disclosed TSO to obtain full-length V(D)J information.

### Immune Repertoire Profiling

There are provided, in some embodiments, methods of 3' and/or 5' expression profiling of the V(D)J region of immune receptors. In some embodiments, the sample comprises a single cell. In some embodiments, the sample comprises a plurality of cells, a plurality of single cells, a tissue, a tumor sample, or any combination thereof. A single cell can comprise an immune cell. In some embodiments, the immune cell is a B cell or a T cell. In some embodiments, a single cell can comprise a circulating tumor cell. In some embodiments, each oligonucleotide barcode can comprise a first universal sequence. In some embodiments, the plurality of extended barcoded nucleic acid molecules comprises a first universal sequence and do not comprise a complement of the first universal sequence. In some embodiments, amplifying the plurality of extended barcoded nucleic acid molecules to generate a plurality of single-labeled nucleic acid molecules comprises using a primer capable of hybridizing to the first universal sequence, and an amplification primer.

In some embodiments, the amplification primer is a target-specific primer. In some such embodiments, the target-specific primer specifically hybridizes to an immune receptor. For example, the target-specific primer can specifically hybridize to a constant region of an immune receptor, a variable region of an immune receptor, a diversity region of an immune receptor, the junction of a variable region and diversity region of an immune receptor, or any combination thereof. The immune receptor can be a T cell receptor (TCR) and/or a B cell receptor (BCR) receptor. The TCR can comprise TCR alpha chain, TCR beta chain, TCR gamma chain, TCR delta chain, or any combination thereof. The BCR can comprise BCR heavy chain and/or BCR light chain.

The method can comprise obtaining sequence information of the plurality of extended barcoded nucleic acid molecules, or products thereof. Obtaining the sequence information can comprise attaching sequencing adaptors to the plurality of extended barcoded nucleic acid molecules, or products thereof. Obtaining the sequence information can comprise attaching sequencing adaptors to the plurality of single-labeled nucleic acid molecules, or products thereof.

Obtaining the sequence information can comprise obtaining the sequence information of the BCR light chain and the BCR heavy chain of a single cell. The sequence information of the BCR light chain and the BCR heavy chain can comprise the sequence of the complementarity determining region 1 (CDR1), the CDR2, the CDR3, or any combination thereof, of the BCR light chain and/or the BCR heavy chain. The method can comprise pairing the BCR light chain and the BCR heavy chain of the single cell based on the obtained sequence information. The sample can comprise a plurality of single cells, and the method can comprise pairing the BCR light chain and the BCR heavy chain of at least 50% of the single cells based on the obtained sequence information. In some embodiments, the percentage of single cells of a sample wherein the BCR light chain and the BCR heavy chain are paired according the methods provided herein can be, or be about, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values. In some embodiments, the percentage of single cells of a sample wherein the BCR light chain and the BCR heavy chain are paired according the methods provided herein can be at least, or at most, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

Obtaining the sequence information can comprise obtaining the sequence information of the TCR alpha chain and the TCR beta chain of a single cell. In some embodiments, the sequence information of the TCR alpha chain and the TCR beta chain can comprise the sequence of the complementarity determining region 1 (CDR1), the CDR2, the CDR3, or any combination thereof, of the TCR alpha chain and/or the TCR beta chain. In some embodiments, the method can comprise pairing the TCR alpha chain and the TCR beta chain of the single cell based on the obtained sequence information. In some embodiments, the sample can comprise a plurality of single cells, and the method can comprise pairing the TCR alpha chain and the TCR beta chain of at least 50% of the single cells based on the obtained sequence information. In some embodiments, the percentage of single cells of a sample wherein the TCR alpha chain and the TCR beta chain are paired according the methods provided herein can be, or be about, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values. In some embodiments, the percentage of single cells of a sample wherein the TCR alpha chain and the TCR beta chain are paired according the methods provided herein can be at least, or at most, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%.

Obtaining the sequence information can comprise obtaining the sequence information of the TCR gamma chain and the TCR delta chain of a single cell. The sequence information of the TCR gamma chain and the TCR delta chain can comprise the sequence of the complementarity determining region 1 (CDR1), the CDR2, the CDR3, or any combination thereof, of the TCR gamma chain and/or the TCR delta chain. The method can comprise pairing the TCR gamma chain and the TCR delta chain of the single cell based on the obtained sequence information. The sample can comprise a plurality of single cells, and the method can comprise pairing the TCR gamma chain and the TCR delta chain of at least 50% of the single cells based on the obtained sequence information. In some embodiments, the percentage of single cells of a sample wherein the TCR delta chain and the TCR gamma chain are paired according the methods provided herein can be, be about, be at least, or at most, 0.000000001%, 0.00000001%, 0.0000001%, 0.000001%, 0.00001%, 0.0001%, 0.001%, 0.01%, 0.1%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values.

### Whole Transcriptome Analysis (WTA) with Random Priming and Extension

Disclosed herein include methods for generating a whole transcriptome analysis (WTA) library from cDNA product (e.g., cDNA product of the BD Rhapsody Single Cell Analysis System) for sequencing on compatible sequencers (e.g., Illumina^{®} sequencers) with the disclosed TSO. In some embodiments, standard BD Rhapsody RNA workflow first captures the RNA transcriptome via 3' capture and cDNA synthesis on the BD Rhapsody Cell Capture Beads (hereafter referred to as 'Beads'), followed by 2 multiplexed, nested PCR amplification to enrich for RNA targets of interest (targeted assay). In some embodiments of the method, targeted RNA analysis generally yields higher sensitivity for low expressing targets, while WTA RNA analysis provides a larger breadth of interrogated genes. Successful amplification of desired targets in some embodiments, may need a thorough understanding of each RNA's 3' end and its use of polyadenylation sites in model system of interest. The WTA method, for example using with BD Rhapsody, allows obtaining of several levels of information, including 1) identify interesting RNA targets in their model system to identify a panel of genes to design; 2) characterize the 3' ends of the transcriptome in their model system as an input to a new generation of PCR panel design for BD Rhapsody, and 3) allows users to do discovery biology by assaying a wider breath of genes compared to the standard BD Rhapsody targeted approach. In some embodiments, the methods comprise random priming and extension reactions using the first and/or second pluralities of extended barcoded nucleic acid molecules, or products thereof, as templates. The compositions and methods disclosed herein can, in some embodiments, be employed in concert with the RPE-based whole transcriptome analysis methods and compositions described in U.S. Patent Application Number 17/163,177, filed on January 29, 2021, entitled "MESOPHILIC DNA POLYMERASE EXTENSION BLOCKERS", the content of which is incorporated herein by reference in its entirety. RPE-based whole transcriptome analysis methods and compositions have been described in U.S. Patent Application No. 16/677,012; the content of which is incorporated herein by reference in its entirety. The RPE-based WTA methods disclosed herein can yield increased sensitivity of low abundance transcripts, detection of new transcripts, detection of new cell types, and/or reduced sequencing costs. Some embodiments of the RPE-based WTA methods provided herein yield increased sensitivity of low abundance transcripts, detection of new transcripts, detection of new cell types, and/or reduced sequencing costs as compared to alternative non-RPE-based WTA methods (e.g., ligation-based or fragmentation-based WTA methods) and/or RPE-based WTA methods.

### Kits

Disclosed herein include kits. In some embodiments, the kit comprises: a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences; a reverse transcriptase; a template switch oligonucleotide (TSO) comprising (i) a complement of a blocking sequence and (ii) the target-binding region, or a portion thereof; and/or a DNA polymerase lacking at least one of 5' to 3' exonuclease activity and 3' to 5' exonuclease activity.

Disclosed herein include kits. In some embodiments, the kit comprises: a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences; a second plurality of oligonucleotide barcodes, wherein each oligonucleotide barcode of the second plurality of oligonucleotide barcodes comprises a second universal sequence, a second molecular label, and a bait sequence, wherein at least 10 of the second plurality of oligonucleotide barcodes comprise different molecular label sequences; a template switch oligonucleotide (TSO) comprising (i) a complement of a blocking sequence and (ii) a bait sequence; a DNA polymerase lacking at least one of 5' to 3' exonuclease activity and 3' to 5' exonuclease activity; and/or a reverse transcriptase.

In some embodiments, the DNA polymerase lacking at least one of 5' to 3' exonuclease activity and 3' to 5' exonuclease activity comprises a mesophilic DNA polymerase, a thermophilic DNA polymerase, a psychrophilic DNA polymerase, or any combination thereof. In some embodiments, the DNA polymerase comprises a Klenow Fragment. In some embodiments, the reverse transcriptase comprises a viral reverse transcriptase. In some embodiments, the viral reverse transcriptase is a murine leukemia virus (MLV) reverse transcriptase. In some embodiments, the viral reverse transcriptase is a Moloney murine leukemia virus (MMLV) reverse transcriptase. In some embodiments, the template switch oligonucleotide comprises one or more 3' ribonucleotides, for example three 3' ribonucleotides. In some embodiments, the 3' ribonucleotides comprise guanine. In some embodiments, the kit comprises one or more of ethylene glycol, polyethylene glycol, 1,2- propanediol, dimethyl sulfoxide (DMSO), glycerol, formamide, 7-deaza-GTP, acetamide, tetramethylammonium chloride salt, betaine, or any combination thereof. In some embodiments, the kit comprises a buffer., In some embodiments, the kit comprises a cartridge. In some embodiments, the kit comprises one or more reagents for a reverse transcription reaction. In some embodiments, the kit comprises one or more reagents for an amplification reaction.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

One skilled in the art will appreciate that, for this and other processes and methods disclosed herein, the functions performed in the processes and methods can be implemented in differing order. Furthermore, the outlined steps and operations are only provided as examples, and some of the steps and operations can be optional, combined into fewer steps and operations, or expanded into additional steps and operations without detracting from the essence of the disclosed embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

### The invention also provides the following numbered embodiments:

1. A method for labeling nucleic acid targets in a sample, comprising:
   contacting copies of a nucleic acid target with a first plurality of oligonucleotide barcodes, wherein each oligonucleotide barcode comprises a first universal sequence, a molecular label, and a target-binding region capable of hybridizing to the nucleic acid target;
   extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region;
   hybridizing the complement of the target-binding region of each barcoded nucleic acid molecule with the target-binding region of an oligonucleotide barcode of the first plurality of oligonucleotide barcodes; and
   extending the 3' ends of oligonucleotide barcodes hybridized to the complement of the target-binding region of the barcoded nucleic acid molecule to generate a first plurality of extended barcoded nucleic acid molecules each comprising a complement of the first molecular label and a second molecular label.
2. The method of embodiment 1, comprising: determining the copy number of the nucleic acid target in the sample based on the number of second molecular labels with distinct sequences associated with the first plurality of extended barcoded nucleic acid molecules, or products thereof.
3. A method for determining the numbers of nucleic acid targets in a sample, comprising:
   contacting copies of a nucleic acid target with a first plurality of oligonucleotide barcodes, wherein each oligonucleotide barcode comprises a first universal sequence, a molecular label, and a target-binding region capable of hybridizing to the nucleic acid target;
   extending the plurality of oligonucleotide barcodes hybridized to the copies of the nucleic acid target in the presence of a reverse transcriptase and a template switch oligonucleotide (TSO) comprising (i) a complement of a blocking sequence and (ii) the target-binding region, or a portion thereof, to generate a first plurality of barcoded nucleic acid molecules each comprising a sequence complementary to at least a portion of the nucleic acid target, a first molecular label, the target-binding region, the blocking sequence, and a complement of the target-binding region;
   hybridizing the complement of the target-binding region of each barcoded nucleic acid molecule with the target-binding region of an oligonucleotide barcode of the first plurality of oligonucleotide barcodes;
   extending the 3' ends of the oligonucleotide barcodes hybridized to the complement of the target-binding region of the barcoded nucleic acid molecule to generate a first plurality of extended barcoded nucleic acid molecules each comprising a complement of the first molecular label and a second molecular label; and
   determining the copy number of the nucleic acid target in the sample based on the number of second molecular labels with distinct sequences associated with the first plurality of extended barcoded nucleic acid molecules, or products thereof.
4. The method of any one of embodiments 2-3,
   comprising amplifying the first plurality of extended barcoded nucleic acid molecules to generate a first plurality of single-labeled nucleic acid molecules each comprising the second molecular label,
   wherein determining the copy number of the nucleic acid target in the sample comprises: determining the copy number of the nucleic acid target in the sample based on the number of second molecular labels with distinct sequences associated with the first plurality of single-labeled nucleic acid molecules.
5. A method for labeling nucleic acid targets in a sample, comprising:
   contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target;
   extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target;
   contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence; and
   extending:
      the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence.
6. The method of embodiment 5, further comprising determining the copy number of the nucleic acid target in the sample based on:
   the number of first molecular labels with distinct sequences, second molecular labels with distinct sequences, or a combination thereof, associated with the second plurality of extended barcoded nucleic acid molecules, or products thereof.
7. A method for determining the copy number of a nucleic acid target in a sample, comprising:
   contacting copies of a nucleic acid target with a first plurality of oligonucleotide barcodes, wherein each oligonucleotide barcode of the first plurality of oligonucleotide barcodes comprises a first universal sequence, a first molecular label, and a target-binding region capable of hybridizing to the nucleic acid target;
   extending the first plurality of oligonucleotide barcodes hybridized to the copies of the nucleic acid target in the presence of a reverse transcriptase and a template switch oligonucleotide (TSO) comprising (i) a complement of a blocking sequence and (ii) a bait sequence to generate a second plurality of barcoded nucleic acid molecules each comprising the first universal sequence, the first molecular label, a complement of the bait sequence, the blocking sequence, and a sequence complementary to at least a portion of the nucleic acid target;
   contacting the barcoded nucleic acid molecules with a second plurality of oligonucleotide barcodes, wherein each oligonucleotide barcode of the second plurality of oligonucleotide barcodes comprises a second universal sequence, a second molecular label, and the bait sequence;
   extending:
      the 3' ends of oligonucleotide barcodes of the second plurality of oligonucleotide barcodes hybridized to the complement of the bait sequence of the barcoded nucleic acid molecules to generate a second plurality of extended barcoded nucleic acid molecules each comprising a second molecular label, a second universal sequence, a complement of the first molecular label and a complement of the first universal sequence; and
   determining the copy number of the nucleic acid target in the sample based on:
      the number of first molecular labels with distinct sequences, second molecular labels with distinct sequences, or a combination thereof, associated with the second plurality of extended barcoded nucleic acid molecules, or products thereof.
8. The method of any one of embodiments 6-7,
   wherein determining the copy number of the nucleic acid target comprises determining the copy number of each of a plurality of nucleic acid targets in the sample based on the number of first molecular labels with distinct sequences, second molecular labels with distinct sequences, or a combination thereof, associated with extended barcoded nucleic acid molecules of the second plurality of extended barcoded nucleic acid molecules comprising a sequence of the each of the plurality of nucleic acid targets.
9. The method of any one of embodiments 1-8, wherein the first universal sequence of each oligonucleotide barcode of the first plurality of oligonucleotide barcodes is 5' of the first molecular label and the target-binding region.
10. The method of any one of embodiments 1-9, wherein the second universal sequence of each oligonucleotide barcode of the second plurality of oligonucleotide barcodes is 5' of the second molecular label and the bait sequence.
11. The method of any one of embodiments 1-10, wherein the bait sequence comprises at least 6 nucleotides.
12. The method of any one of embodiments 1-11, wherein the bait sequence comprises a GC content of about 20% to about 80%.
13. The method of any one of embodiments 1-12,
   comprising amplifying the second plurality of extended barcoded nucleic acid molecules using an amplification primer and a primer comprising the second universal sequence, or a portion thereof, thereby generating a second plurality of single-labeled nucleic acid molecules comprising the sequence of the nucleic acid target, or a portion thereof,
   wherein determining the copy number of the nucleic acid target in the sample comprises: determining the copy number of the nucleic acid target in the sample based on the number of second molecular labels with distinct sequences associated with the second plurality of single-labeled nucleic acid molecules, or products thereof.
14. The method of any one of embodiments 1-13,
   comprising amplifying the second plurality of extended barcoded nucleic acid molecules using an amplification primer and a primer comprising the first universal sequence, or a portion thereof, thereby generating a third plurality of single-labeled nucleic acid molecules comprising the sequence of the nucleic acid target, or a portion thereof,
   wherein determining the copy number of the nucleic acid target in the sample comprises: determining the copy number of the nucleic acid target in the sample based on the number of first molecular labels with distinct sequences associated with the third plurality of single-labeled nucleic acid molecules, or products thereof.
15. The method of any one of embodiments 1-14, wherein the oligonucleotide barcodes of the first and/or second pluralities of oligonucleotide barcodes comprise a cell label, optionally the cell label is located 5' of the molecular label.
16. The method of any one of embodiments 1-15, wherein at least a portion of the oligonucleotide barcode the first and/or second pluralities of oligonucleotide barcodes comprises at least one variable sequence, optionally at least 10 of the first and/or second pluralities of oligonucleotide barcodes comprise different sequences at the invariable sequence, optionally at least a portion of the cell label and/or molecular label comprises a variable sequence.
17. The method of any one of embodiments 1-16, wherein the blocking sequence:
   is located at the 3' end of the first and/or second pluralities of barcoded nucleic acid molecules;
   is configured to be non-complementary to: (i) the region of the oligonucleotide barcode of the first plurality of oligonucleotide barcodes 5' of the target-binding region; and/or (ii) the region of the oligonucleotide barcode of the second plurality of oligonucleotide barcodes 5' of the bait sequence;
   is less than about 50% complementary to: (i) the region of at least 50% of oligonucleotide barcodes of the first plurality of oligonucleotide barcodes 5' of the target-binding region; and/or (ii) the region of at least 50% of the oligonucleotide barcodes of the second plurality of oligonucleotide barcodes 5' of the bait sequence;
   is configured to prevent extension of the 3' ends of the first and/or second pluralities of barcoded nucleic acid molecules;
   reduces the generation of extended barcoded nucleic acid molecules comprising a complement of the first universal sequence;
   comprises a blocker nucleotide, wherein the blocker nucleotide is located at the 3' end of the blocking sequence; and/or
   comprises at least 4 contiguous bases of any one of SEQ ID NOs: 1-7.
18. The method of any one of embodiments 1-17, wherein,
   in the absence of the blocking sequence,
      (i) the 3' ends of the second plurality of barcoded nucleic acid molecules hybridized to the bait sequence of the second plurality of oligonucleotide barcodes; and/or
      (ii) the 3' ends of the first plurality of barcoded nucleic acid molecules hybridized to the target-binding region of the first plurality of oligonucleotide barcodes;
   are capable of being extended to generate a third plurality of extended barcoded nucleic acid molecules.
19. The method of any one of embodiments 1-18, wherein the generation of the third plurality of extended barcoded nucleic acid molecules is reduced by at least 10%, by at least 25%, by at least 50%, by at least 80%. by at least 90%, by at least 95%, or by at least 99%, as compared to a comparable method wherein the TSO does not comprise a complement of a blocking sequence.
20. The method of any one of embodiments 1-19, wherein at least a portion of an oligonucleotide barcode of the first and/or second pluralities of oligonucleotide barcodes comprises an invariable sequence, optionally each of the first and/or second pluralities of oligonucleotide barcodes comprise the same sequence at the invariable sequence.
21. The method of any one of embodiments 1-20, wherein the invariable sequence is between about 1-15 nucleotides in length, optionally the invariable sequence is 1 nucleotide in length.
22. The method of any one of embodiments 1-21, wherein the invariable sequence is located in the cell label, optionally the cell label comprises a first portion of the cell label, a first linker, a second portion of the cell label, a second linker, and a third portion of the cell label, further optionally the invariable sequence is located in the third portion of the cell label.
23. The method of any one of embodiments 1-22, wherein the blocker nucleotide is configured to be non-complementary to the invariable sequence.
24. The method of any one of embodiments 1-23, wherein the distance between the invariable sequence and the bait sequence is equidistant to the distance between the complement of the bait sequence and the blocker nucleotide.
25. The method of any one of embodiments 1-24, wherein the distance between the invariable sequence and the target-binding region is equidistant to the distance between the complement of the target-binding region and the blocker nucleotide.
26. The method of any one of embodiments 1-25, wherein the TSO comprises a sequence selected from the group consisting of SEQ ID NOs: 1-7, or a sequence that exhibits at least about 50% identity to a sequence selected from the group consisting of SEQ ID NOs: 1-7.
27. The method of any one of embodiments 1-26, wherein the complement of the blocking sequence is 5' of the bait sequence in the TSO.
28. The method of any one of embodiments 1-27, wherein the complement of the blocking sequence is 5' of the target-binding region, or portion thereof, in the TSO.
29. The method of any one of embodiments 1-28, wherein Template Switch Efficiency is reduced less than 40%, less than 20%, less than 10%, or less than 5%, as compared to a comparable method wherein the TSO does not comprise a complement of a blocking sequence.
30. The method of any one of embodiments 1-29, wherein the method further comprises the addition of blocker oligonucleotides before or during one or more extension steps.
31. The method of any one of embodiments 1-30, wherein the method does not comprise the addition of blocker oligonucleotides.
32. The method of any one of embodiments 1-31, wherein the complement of the blocking sequence comprises a reverse complementary sequence of the blocking sequence, and/or a complementary sequence of the blocking sequence.
33. The method of any one of embodiments 1-32, wherein hybridizing the complement of the target-binding region of a barcoded nucleic acid molecule with the target-binding region of an oligonucleotide barcode of the first plurality of oligonucleotide barcodes comprises intermolecular hybridization of the complement of the target-binding region of a barcoded nucleic acid molecule with the target-binding region of an oligonucleotide barcode of the first plurality of oligonucleotide barcodes.
34. The method of any one of embodiments 1-33, the method comprising denaturing the first plurality of barcoded nucleic acid molecules prior to hybridizing the complement of the target-binding region of each barcoded nucleic acid molecule with the target-binding region of an oligonucleotide barcode of the first plurality of oligonucleotide barcodes.
35. The method of any one of embodiments 1-34, the method comprising denaturing the first and/or second plurality of extended barcoded nucleic acid molecules prior to amplifying the first and/or second plurality of extended barcoded nucleic acid molecules.
36. The method of any one of embodiments 1-35,
   wherein determining the copy number of the nucleic acid target comprises determining the copy number of each of the plurality of nucleic acid targets in the sample based on the number of second molecular labels with distinct sequences associated with single-labeled nucleic acid molecules of the first plurality of single-labeled nucleic acid molecules comprising a sequence of the each of the plurality of nucleic acid targets.
37. The method of embodiment 36, wherein the sequence of the each of the plurality of nucleic acid targets comprises a subsequence of the each of the plurality of nucleic acid targets.
38. The method of any one of embodiments 1-37, wherein the sequence of the nucleic acid target in the first plurality of barcoded nucleic acid molecules and/or second plurality of barcoded nucleic acid molecules comprises a subsequence of the nucleic acid target.
39. The method of any one of embodiments 1-38, wherein the complement of the target-binding region is complementary to a portion of the target-binding region.
40. The method of any one of embodiments 1-39, wherein the target-binding region comprises a gene-specific sequence, and/or a poly(dT) sequence.
41. The method of any one of embodiments 1-40, wherein the second molecular label is a different from the first molecular label, and wherein the second molecular label is not a complement of the first molecular label.
42. The method of any one of embodiments 1-41, wherein the first and/or second plurality of extended barcoded nucleic acid molecules each comprise the sequence of the nucleic acid target.
43. The method of any one of embodiments 1-42, wherein the nucleic acid target comprises mRNA, and wherein the first and/or second plurality of extended barcoded nucleic acid molecules each comprise the sequence of the sense strand of the nucleic acid target.
44. The method of any one of embodiments 1-43,
   wherein the reverse transcriptase is capable of terminal transferase activity;
   wherein the template switch oligonucleotide comprises one or more 3' ribonucleotides, optionally three 3' ribonucleotides, and further optionally the 3' ribonucleotides comprise guanine; and/or
   wherein the reverse transcriptase comprises a viral reverse transcriptase, optionally the viral reverse transcriptase is a murine leukemia virus (MLV) reverse transcriptase or a Moloney murine leukemia virus (MMLV) reverse transcriptase.
45. The method of any one of embodiments 1-44,
   wherein the sample comprises a single cell, optionally an immune cell, and further optionally a B cell or a T cell; and/or
   wherein the sample comprises a plurality of cells, a plurality of single cells, a tissue, a tumor sample, or any combination thereof,
   optionally wherein single cell comprises a circulating tumor cell.
46. The method of any one of embodiments 1-45, wherein the first universal sequence is 5' of the molecular label and the target-binding region.
47. The method of any one of embodiments 1-46, wherein amplifying the first plurality of extended barcoded nucleic acid molecules to generate a first plurality of single-labeled nucleic acid molecules comprises using a primer capable of hybridizing to the first universal sequence, and an amplification primer.
48. The method of any one of embodiments 1-47, wherein the amplification primer is a target-specific primer, and optionally the target-specific primer specifically hybridizes to an immune receptor, a constant region of an immune receptor, a variable region of an immune receptor, a diversity region of an immune receptor, and/or the junction of a variable region and diversity region of an immune receptor.
49. The method of embodiment 48, wherein the immune receptor is a T cell receptor (TCR) and/or a B cell receptor (BCR) receptor, and optionally
   the TCR comprises TCR alpha chain, TCR beta chain, TCR gamma chain, TCR delta chain, or any combination thereof; and
   the BCR receptor comprises BCR heavy chain and/or BCR light chain.
50. The method of any one of embodiments 47-49, wherein the amplification primer specifically binds the extended barcoded nucleic acid molecules each comprising a complement of the first molecular label and a second molecular label.
51. The method of any one of embodiments 47-50, wherein the amplification primer does not bind extended barcoded nucleic acid molecules comprising the first molecular label.
52. The method of any one of embodiments 47-51, wherein the amplification primer comprises the complement of the nucleic acid target.
53. The method of any one of embodiments 47-52, wherein the nucleic acid target comprises mRNA, and wherein the amplification primer comprises the sequence of the anti-sense strand of the nucleic acid target.
54. The method of any one of embodiments 1-53, wherein extending the 3' ends of the oligonucleotide barcodes comprises extending the 3' ends of the oligonucleotide barcodes using a mesophilic DNA polymerase, a thermophilic DNA polymerase, a psychrophilic DNA polymerase, or any combination thereof.
55. The method of any one of embodiments 1-54, wherein extending the 3' ends of the oligonucleotide barcodes comprises extending the 3' ends of the oligonucleotide barcodes using a DNA polymerase lacking at least one of 5' to 3' exonuclease activity and 3' to 5' exonuclease activity, and optionally the DNA polymerase comprises a Klenow Fragment.
56. The method of any one of embodiments 1-55, comprising obtaining sequence information of the first and/or second plurality of extended barcoded nucleic acid molecules, or products thereof.
57. The method of any one of embodiments 1-56, comprising obtaining sequence information of one or more of the first, second, and third pluralities of single-labeled nucleic acid molecules, or products thereof,
58. The method of any one of embodiments 56-57, wherein obtaining the sequence information comprises attaching sequencing adaptors to the first and/or second plurality of extended barcoded nucleic acid molecules, or products thereof.
59. The method of any one of embodiments 56-58, wherein obtaining the sequence information comprises attaching sequencing adaptors to the first, second, and/or third pluralities of single-labeled nucleic acid molecules, or products thereof.
60. The method of any one of embodiments 57-59, wherein obtaining sequence information comprises:
   obtaining sequencing data comprising a plurality of sequencing reads of one or more of the first and/or second plurality of extended barcoded nucleic acid molecules, or products thereof, and/or the first, second, and third pluralities of single-labeled nucleic acid molecules, or products thereof,
   wherein each of the plurality of sequencing reads comprise (1) a cell label sequence, (2) a molecular label sequence, and/or (3) a subsequence of the nucleic acid target.
61. The method of embodiment 60, wherein less than 40%, less than 20%, less than 10%, or less than 5%, of the total sequencing reads are derived from the third plurality of extended barcoded nucleic acid molecules, or products thereof.
62. The method of any one of embodiments 60-61, wherein the number of sequencing reads derived from the third plurality of extended barcoded nucleic acid molecules, or products thereof, is reduced at least about 2-fold as compared to a comparable method wherein the TSO does not comprise a complement of a blocking sequence.
63. The method of any one of embodiments 56-62, wherein obtaining the sequence information comprises obtaining the sequence information of the BCR light chain and the BCR heavy chain of a single cell,
   optionally the sequence information of the BCR light chain and the BCR heavy chain comprises the sequence of the complementarity determining region 1 (CDR1), the CDR2, the CDR3, or any combination thereof, of the BCR light chain and/or the BCR heavy chain,
   optionally the method comprises pairing the BCR light chain and the BCR heavy chain of the single cell based on the obtained sequence information, and
   optionally the sample comprises a plurality of single cells, the method comprising pairing the BCR light chain and the BCR heavy chain of at least 50% of said single cells based on the obtained sequence information.
64. The method of any one of embodiments 56-63, wherein obtaining the sequence information comprises obtaining the sequence information of the TCR alpha chain and the TCR beta chain of a single cell,
   optionally the sequence information of the TCR alpha chain and the TCR beta chain comprises the sequence of the complementarity determining region 1 (CDR1), the CDR2, the CDR3, or any combination thereof, of the TCR alpha chain and/or the TCR beta chain,
   optionally the method comprises pairing the TCR alpha chain and the TCR beta chain of the single cell based on the obtained sequence information, and
   optionally the sample comprises a plurality of single cells, the method comprising pairing the TCR alpha chain and the TCR beta chain of at least 50% of said single cells based on the obtained sequence information.
65. The method of any one of embodiments 56-64, wherein obtaining the sequence information comprises obtaining the sequence information of the TCR gamma chain and the TCR delta chain of a single cell,
   optionally the sequence information of the TCR gamma chain and the TCR delta chain comprises the sequence of the complementarity determining region 1 (CDR1), the CDR2, the CDR3, or any combination thereof, of the TCR gamma chain and/or the TCR delta chain,
   optionally the method comprises pairing the TCR gamma chain and the TCR delta chain of the single cell based on the obtained sequence information, and
   optionally the sample comprises a plurality of single cells, the method comprising pairing the TCR gamma chain and the TCR delta chain of at least 50% of said single cells based on the obtained sequence information.
66. The method of any one of embodiments 1-65, wherein the complement of the target-binding region comprises the reverse complementary sequence of the target-binding region and/or the complementary sequence of the target-binding region.
67. The method of any one of embodiments 1-66, wherein the complement of the molecular label comprises a reverse complementary sequence of the molecular label, and/or a complementary sequence of the molecular label.
68. The method of any one of embodiments 1-67, wherein the first and/or second plurality of barcoded nucleic acid molecules comprises barcoded deoxyribonucleic acid (DNA) molecules and/or barcoded ribonucleic acid (RNA) molecules.
69. The method of any one of embodiments 1-68, wherein the target-binding region comprises an oligo dT sequence, a random sequence, a target-specific sequence, or a combination thereof.
70. The method of any one of embodiments 1-69, wherein the target-binding region comprises a poly(dT) region, and wherein the nucleic acid target comprises a poly(dA) region.
71. The method of any one of embodiments 1-70, wherein the sample comprises a plurality of cells, a plurality of single cells, a tissue, a tumor sample, or any combination thereof.
72. The method of any one of embodiments 1-71, wherein the nucleic acid target comprises
   a nucleic acid molecule, optionally the nucleic acid molecule comprises ribonucleic acid (RNA), messenger RNA (mRNA), microRNA, small interfering RNA (siRNA), RNA degradation product, RNA comprising a poly(A) tail, or any combination thereof, optionally the mRNA encodes an immune receptor; and/or
   a cellular component binding reagent, optionally the nucleic acid molecule is associated with the cellular component binding reagent, optionally the method comprises dissociating the nucleic acid molecule and the cellular component binding reagent.
73. The method of any one of embodiments 1-72, wherein at least 10 of the first plurality of oligonucleotide barcodes comprise different molecular label sequences.
74. The method of any one of embodiments 1-73, wherein each molecular label of the plurality of oligonucleotide barcodes comprises at least 6 nucleotides.
75. The method of any one of embodiments 1-74, wherein the first plurality of oligonucleotide barcodes are associated with a solid support, and optionally the first plurality of oligonucleotide barcodes associated with the same solid support each comprise an identical sample label, and further optionally each sample label of the first plurality of oligonucleotide barcodes comprises at least 6 nucleotides.
76. The method of any one of embodiments 1-75,
   wherein the first plurality of oligonucleotide barcodes each comprise a cell label, and optionally each cell label of the first plurality of oligonucleotide barcodes comprises at least 6 nucleotides;
   wherein oligonucleotide barcodes associated with the same solid support comprise the same cell label; and/or
   wherein oligonucleotide barcodes associated with different solid supports comprise different cell labels.
77. The method of embodiment 76, wherein the first and/or second plurality of extended barcoded nucleic acid molecules each comprises a cell label and a complement of the cell label, and optionally the complement of the cell label comprises a reverse complementary sequence of the cell label and/or a complementary sequence of the cell label.
78. The method of any one of embodiments 1-77, comprising extending the first plurality of oligonucleotide barcodes hybridized to the copies of the nucleic acid target in the presence of one or more of ethylene glycol, polyethylene glycol, 1,2- propanediol, dimethyl sulfoxide (DMSO), glycerol, formamide, 7-deaza-GTP, acetamide, tetramethylammonium chloride salt, betaine, or any combination thereof.
79. The method of any one of embodiments 1-78,
   wherein at least 10 of the first and second pluralities of oligonucleotide barcodes comprise different molecular label sequences, optionally each molecular label of the first and second pluralities of oligonucleotide barcodes comprises at least 6 nucleotides;
   wherein the first and second pluralities of oligonucleotide barcodes are associated with a solid support;
   wherein the first and second pluralities of oligonucleotide barcodes associated with the same solid support each comprise an identical sample label, optionally each sample label of the first and second pluralities of oligonucleotide barcodes comprises at least 6 nucleotides;
   wherein the first and second pluralities of oligonucleotide barcodes each comprise a cell label, optionally each cell label of the first and second pluralities of oligonucleotide barcodes comprises at least 6 nucleotides;
   wherein (a) oligonucleotide barcodes of the first and second pluralities of oligonucleotide barcodes associated with the same solid support comprise the same cell label; and/or (b) oligonucleotide barcodes of the first and second pluralities of oligonucleotide barcodes associated with different solid supports comprise different cell labels; and/or
   wherein the extended barcoded nucleic acid molecules of the first and second pluralities of extended barcoded nucleic acid molecules each comprises a cell label and a complement of the cell label, optionally the complement of the cell label comprises a reverse complementary sequence of the cell label, or a complementary sequence of the cell label.
80. The method of any one of embodiments 1-79,
   wherein at least one oligonucleotide barcode of the first and second pluralities of oligonucleotide barcodes is immobilized or partially immobilized on the synthetic particle, or at least one oligonucleotide barcode of the first and second pluralities of oligonucleotide barcodes is enclosed or partially enclosed in the synthetic particle;
   wherein the solid support comprises a synthetic particle or a planar surface;
   wherein the sample comprises a single cell, the method comprising associating a synthetic particle comprising the plurality of the oligonucleotide barcodes with the single cell in the sample;
   wherein the method comprises lysing the single cell after associating the synthetic particle with the single cell, and optionally lysing the single cell comprises heating the sample, contacting the sample with a detergent, changing the pH of the sample, or any combination thereof;
   wherein the synthetic particle and the single cell are in the same well;
   wherein the synthetic particle and the single cell are in the same droplet;
   wherein at least one of the first plurality of oligonucleotide barcodes is immobilized or partially immobilized on the synthetic particle, or the at least one of the first plurality of oligonucleotide barcodes is enclosed or partially enclosed in the synthetic particle; and/or
   wherein the synthetic particle is disruptable.
81. The method of any one of embodiments 1-80, wherein the synthetic particle comprises a bead, and optionally the bead comprises
   a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof;
   a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof; or
   a disruptable hydrogel particle.
82. The method of any one of embodiments 1-81,
   wherein each oligonucleotide barcode of the first and second pluralities of oligonucleotide barcodes comprises a linker functional group,
   wherein the synthetic particle comprises a solid support functional group, and
   wherein the support functional group and the linker functional group are associated with each other, and optionally the linker functional group and the support functional group are individually selected from the group consisting of C6, biotin, streptavidin, primary amine(s), aldehyde(s), ketone(s), and any combination thereof.
83. The method of any one of embodiments 1-82,
   wherein each of the first plurality of oligonucleotide barcodes comprises a linker functional group,
   wherein the synthetic particle comprises a solid support functional group, and
   wherein the support functional group and the linker functional group are associated with each other,
   and optionally the linker functional group and the support functional group are individually selected from the group consisting of C6, biotin, streptavidin, primary amine(s), aldehyde(s), ketone(s), and any combination thereof.
84. A kit comprising:
   a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences;
   a reverse transcriptase;
   a template switch oligonucleotide (TSO) comprising (i) a complement of a blocking sequence and (ii) the target-binding region, or a portion thereof; and/or
   a DNA polymerase lacking at least one of 5' to 3' exonuclease activity and 3' to 5' exonuclease activity.
85. A kit comprising:
   a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences;
   a second plurality of oligonucleotide barcodes, wherein each oligonucleotide barcode of the second plurality of oligonucleotide barcodes comprises a second universal sequence, a second molecular label, and a bait sequence, wherein at least 10 of the second plurality of oligonucleotide barcodes comprise different molecular label sequences;
   a template switch oligonucleotide (TSO) comprising (i) a complement of a blocking sequence and (ii) a bait sequence;
   a DNA polymerase lacking at least one of 5' to 3' exonuclease activity and 3' to 5' exonuclease activity; and/or
   a reverse transcriptase.
86. The kit of any one of embodiments 84-85, wherein the oligonucleotide barcodes of the first and/or second pluralities of oligonucleotide barcodes comprise a cell label, optionally the cell label is located 5' of the molecular label.
87. The kit of any one of embodiments 84-86, wherein at least a portion of the oligonucleotide barcode the first and/or second pluralities of oligonucleotide barcodes comprises at least one variable sequence, optionally at least 10 of the first and/or second pluralities of oligonucleotide barcodes comprise different sequences at the invariable sequence, optionally at least a portion of the cell label and/or molecular label comprises a variable sequence.
88. The kit of any one of embodiments 84-87, wherein the blocking sequence:
   is located at the 3' end of the first and/or second pluralities of barcoded nucleic acid molecules;
   is configured to be non-complementary to: (i) the region of the oligonucleotide barcode of the first plurality of oligonucleotide barcodes 5' of the target-binding region; and/or (ii) the region of the oligonucleotide barcode of the second plurality of oligonucleotide barcodes 5' of the bait sequence;
   is less than about 50% complementary to: (i) the region of at least 50% of oligonucleotide barcodes of the first plurality of oligonucleotide barcodes 5' of the target-binding region; and/or (ii) the region of at least 50% of the oligonucleotide barcodes of the second plurality of oligonucleotide barcodes 5' of the bait sequence;
   comprises a blocker nucleotide, wherein the blocker nucleotide is located at the 3' end of the blocking sequence; and/or
   comprises at least 4 contiguous bases of any one of SEQ ID NOs: 1-7.
89. The kit of any one of embodiments 84-88, wherein at least a portion of an oligonucleotide barcode of the first and/or second pluralities of oligonucleotide barcodes comprises an invariable sequence, optionally each of the first and/or second pluralities of oligonucleotide barcodes comprise the same sequence at the invariable sequence.
90. The kit of any one of embodiments 84-89, wherein the invariable sequence:
   is between about 1-15 nucleotides in length, optionally the invariable sequence is 1 nucleotide in length; and/or
   is located in the cell label, optionally the cell label comprises a first portion of the cell label, a first linker, a second portion of the cell label, a second linker, and a third portion of the cell label, further optionally the invariable sequence is located in the third portion of the cell label.
91. The kit of any one of embodiments 84-90,
   wherein the blocker nucleotide is configured to be non-complementary to the invariable sequence;
   wherein the distance between the invariable sequence and the bait sequence is equidistant to the distance between the complement of the bait sequence and the blocker nucleotide;
   wherein the distance between the invariable sequence and the target-binding region is equidistant to the distance between the complement of the target-binding region and the blocker nucleotide;
   wherein the TSO comprises a sequence selected from the group consisting of SEQ ID NOs: 1-7, or a sequence that exhibits at least about 50% identity to a sequence selected from the group consisting of SEQ ID NOs: 1-7;
   wherein the complement of the blocking sequence is 5' of the bait sequence in the TSO;
   wherein the complement of the blocking sequence is 5' of the target-binding region, or portion thereof, in the TSO; and/or
   wherein the complement of the blocking sequence comprises a reverse complementary sequence of the blocking sequence, and/or a complementary sequence of the blocking sequence.
92. The kit of any one of embodiments 84-91,
   wherein the DNA polymerase lacking at least one of 5' to 3' exonuclease activity and 3' to 5' exonuclease activity comprises a mesophilic DNA polymerase, a thermophilic DNA polymerase, a psychrophilic DNA polymerase, or any combination thereof, optionally the DNA polymerase comprises a Klenow Fragment;
   wherein the reverse transcriptase comprises a viral reverse transcriptase, optionally a murine leukemia virus (MLV) reverse transcriptase or a Moloney murine leukemia virus (MMLV) reverse transcriptase; and/or
   wherein the template switch oligonucleotide comprises one or more 3' ribonucleotides, optionally three 3' ribonucleotides, and further optionally the 3' ribonucleotides comprise guanine.
93. The kit of any one of embodiments 84-92, comprising:
   one or more of ethylene glycol, polyethylene glycol, 1,2- propanediol, dimethyl sulfoxide (DMSO), glycerol, formamide, 7-deaza-GTP, acetamide, tetramethylammonium chloride salt, betaine, or any combination thereof; and/or
   a buffer, a cartridge, one or more reagents for a reverse transcription reaction, one or more reagents for an amplification reaction, or a combination thereof.
94. The kit of any one of embodiments 84-93,
   wherein the target-binding region comprises a gene-specific sequence, an oligo(dT) sequence, a random multimer, or any combination thereof;
   wherein at least 10 of the first and second pluralities of oligonucleotide barcodes comprise different molecular label sequences, optionally each molecular label of the first and second pluralities of oligonucleotide barcodes comprises at least 6 nucleotides;
   wherein the first and second pluralities of oligonucleotide barcodes are associated with a solid support;
   wherein the first and second pluralities of oligonucleotide barcodes associated with the same solid support each comprise an identical sample label, optionally each sample label of the first and second pluralities of oligonucleotide barcodes comprises at least 6 nucleotides;
   wherein the first and second pluralities of oligonucleotide barcodes each comprise a cell label, optionally each cell label of the first and second pluralities of oligonucleotide barcodes comprises at least 6 nucleotides;
   wherein (a) oligonucleotide barcodes of the first and second pluralities of oligonucleotide barcodes associated with the same solid support comprise the same cell label; and/or (b) oligonucleotide barcodes of the first and second pluralities of oligonucleotide barcodes associated with different solid supports comprise different cell labels; and/or
   wherein the extended barcoded nucleic acid molecules of the first and second pluralities of extended barcoded nucleic acid molecules each comprises a cell label and a complement of the cell label, optionally the complement of the cell label comprises a reverse complementary sequence of the cell label, or a complementary sequence of the cell label.
95. The kit of any one of embodiments 84-94,
   wherein at least one oligonucleotide barcode of the first and second pluralities of oligonucleotide barcodes is immobilized or partially immobilized on the synthetic particle, or at least one oligonucleotide barcode of the first and second pluralities of oligonucleotide barcodes is enclosed or partially enclosed in the synthetic particle;
   wherein the oligonucleotide barcode comprises an identical sample label and/or an identical cell label; optionally wherein each sample label, cell label, and/or molecular label of the first plurality of oligonucleotide barcodes comprise at least 6 nucleotides;
   wherein at least one of the first plurality of oligonucleotide barcodes is immobilized or partially immobilized on the synthetic particle; and/or the at least one of the first plurality of oligonucleotide barcodes is enclosed or partially enclosed in the synthetic particle; and/or
   wherein the synthetic particle is disruptable.
96. The kit of any one of embodiments 84-95, wherein the synthetic particle comprises a bead, and optionally the bead comprises
   a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof;
   a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof; or
   a disruptable hydrogel particle.
97. The kit of any one of embodiments 84-96,
   wherein each oligonucleotide barcode of the first and second pluralities of oligonucleotide barcodes comprises a linker functional group,
   wherein the synthetic particle comprises a solid support functional group, and
   wherein the support functional group and the linker functional group are associated with each other, and optionally the linker functional group and the support functional group are individually selected from the group consisting of C6, biotin, streptavidin, primary amine(s), aldehyde(s), ketone(s), and any combination thereof.
98. The kit of any one of embodiments 84-97,
   wherein each of the first plurality of oligonucleotide barcodes comprises a linker functional group,
   wherein the synthetic particle comprises a solid support functional group, and
   wherein the support functional group and the linker functional group are associated with each other; and optionally the linker functional group and the support functional group are individually selected from the group consisting of C6, biotin, streptavidin, primary amine(s), aldehyde(s), ketone(s), and any combination thereof.

## Claims

1. A kit comprising:
a first plurality of oligonucleotide barcodes, wherein each of the first plurality of oligonucleotide barcodes comprises a first universal sequence, a cell label, a molecular label, and a target-binding region, and wherein at least 10 of the first plurality of oligonucleotide barcodes comprise different molecular label sequences; and
a second plurality of oligonucleotide barcodes, wherein each oligonucleotide barcode of the second plurality of oligonucleotide barcodes comprises a second universal sequence, a second molecular label, and a bait sequence, wherein at least 10 of the second plurality of oligonucleotide barcodes comprise different molecular label sequences,
wherein the first and second pluralities of oligonucleotide barcodes are associated with a solid support.

2. The kit of claim 1, further comprising:
a template switch oligonucleotide (TSO) comprising (i) a complement of a blocking sequence and (ii) a bait sequence;
a DNA polymerase lacking at least one of 5' to 3' exonuclease activity and 3' to 5' exonuclease activity; and/or
a reverse transcriptase.

3. The kit of any one of claims 1-2, wherein the oligonucleotide barcodes of the first and/or second pluralities of oligonucleotide barcodes comprise a cell label, optionally the cell label is located 5' of the molecular label.

4. The kit of any one of claims 1-3, wherein at least a portion of the oligonucleotide barcode the first and/or second pluralities of oligonucleotide barcodes comprises at least one variable sequence, optionally at least 10 of the first and/or second pluralities of oligonucleotide barcodes comprise different sequences at an invariable sequence, optionally at least a portion of the cell label and/or molecular label comprises a variable sequence.

5. The kit of any one of claims 2-4, wherein the blocking sequence:
is located at the 3' end of the first and/or second pluralities of barcoded nucleic acid molecules;
is configured to be non-complementary to: (i) the region of the oligonucleotide barcode of the first plurality of oligonucleotide barcodes 5' of the target-binding region; and/or (ii) the region of the oligonucleotide barcode of the second plurality of oligonucleotide barcodes 5' of the bait sequence;
is less than about 50% complementary to: (i) the region of at least 50% of oligonucleotide barcodes of the first plurality of oligonucleotide barcodes 5' of the target-binding region; and/or (ii) the region of at least 50% of the oligonucleotide barcodes of the second plurality of oligonucleotide barcodes 5' of the bait sequence;
comprises a blocker nucleotide, wherein the blocker nucleotide is located at the 3' end of the blocking sequence; and/or
comprises at least 4 contiguous bases of any one of SEQ ID NOs: 1-7.

6. The kit of any one of claims 1-5, wherein at least a portion of an oligonucleotide barcode of the first and/or second pluralities of oligonucleotide barcodes comprises an invariable sequence, optionally each of the first and/or second pluralities of oligonucleotide barcodes comprise the same sequence at the invariable sequence.

7. The kit of any one of claims 4-6, wherein the invariable sequence:
is between about 1-15 nucleotides in length, optionally the invariable sequence is 1 nucleotide in length; and/or
is located in the cell label, optionally the cell label comprises a first portion of the cell label, a first linker, a second portion of the cell label, a second linker, and a third portion of the cell label, further optionally the invariable sequence is located in the third portion of the cell label.

8. The kit of any one of claims 5-7,
wherein the blocker nucleotide is configured to be non-complementary to the invariable sequence;
wherein the distance between the invariable sequence and the bait sequence is equidistant to the distance between the complement of the bait sequence and the blocker nucleotide;
wherein the distance between the invariable sequence and the target-binding region is equidistant to the distance between the complement of the target-binding region and the blocker nucleotide;
wherein the TSO comprises a sequence selected from the group consisting of SEQ ID NOs: 1-7, or a sequence that exhibits at least about 50% identity to a sequence selected from the group consisting of SEQ ID NOs: 1-7;
wherein the complement of the blocking sequence is 5' of the bait sequence in the TSO;
wherein the complement of the blocking sequence is 5' of the target-binding region, or portion thereof, in the TSO; and/or
wherein the complement of the blocking sequence comprises a reverse complementary sequence of the blocking sequence, and/or a complementary sequence of the blocking sequence.

9. The kit of any one of claims 2-8,
wherein the DNA polymerase lacking at least one of 5' to 3' exonuclease activity and 3' to 5' exonuclease activity comprises a mesophilic DNA polymerase, a thermophilic DNA polymerase, a psychrophilic DNA polymerase, or any combination thereof, optionally the DNA polymerase comprises a Klenow Fragment;
wherein the reverse transcriptase comprises a viral reverse transcriptase, optionally a murine leukemia virus (MLV) reverse transcriptase or a Moloney murine leukemia virus (MMLV) reverse transcriptase; and/or
wherein the template switch oligonucleotide comprises one or more 3' ribonucleotides, optionally three 3' ribonucleotides, and further optionally the 3' ribonucleotides comprise guanine.

10. The kit of any one of claims 1-9, comprising:
one or more of ethylene glycol, polyethylene glycol, 1,2- propanediol, dimethyl sulfoxide (DMSO), glycerol, formamide, 7-deaza-GTP, acetamide, tetramethylammonium chloride salt, betaine, or any combination thereof; and/or
a buffer, a cartridge, one or more reagents for a reverse transcription reaction, one or more reagents for an amplification reaction, or a combination thereof.

11. The kit of any one of claims 1-10,
wherein the target-binding region comprises a gene-specific sequence, an oligo(dT) sequence, a random multimer, or any combination thereof;
wherein at least 10 of the first and second pluralities of oligonucleotide barcodes comprise different molecular label sequences, optionally each molecular label of the first and second pluralities of oligonucleotide barcodes comprises at least 6 nucleotides;
wherein the first and second pluralities of oligonucleotide barcodes associated with the same solid support each comprise an identical sample label, optionally each sample label of the first and second pluralities of oligonucleotide barcodes comprises at least 6 nucleotides;
wherein the first and second pluralities of oligonucleotide barcodes each comprise a cell label, optionally each cell label of the first and second pluralities of oligonucleotide barcodes comprises at least 6 nucleotides; and/or
wherein (a) oligonucleotide barcodes of the first and second pluralities of oligonucleotide barcodes associated with the same solid support comprise the same cell label; and/or (b) oligonucleotide barcodes of the first and second pluralities of oligonucleotide barcodes associated with different solid supports comprise different cell labels.

12. The kit of any one of claims 1-11,
wherein at least one oligonucleotide barcode of the first and second pluralities of oligonucleotide barcodes is immobilized or partially immobilized on a synthetic particle, or at least one oligonucleotide barcode of the first and second pluralities of oligonucleotide barcodes is enclosed or partially enclosed in a synthetic particle;
wherein the oligonucleotide barcode comprises an identical sample label and/or an identical cell label; optionally wherein each sample label, cell label, and/or molecular label of the first plurality of oligonucleotide barcodes comprise at least 6 nucleotides; and/or
wherein at least one of the first plurality of oligonucleotide barcodes is immobilized or partially immobilized on a synthetic particle; and/or the at least one of the first plurality of oligonucleotide barcodes is enclosed or partially enclosed in a synthetic particle.

13. The kit of claim 12, wherein the synthetic particle comprises a bead, and optionally the bead comprises
a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof;
a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof; or
a disruptable hydrogel particle.

14. The kit of any one of claims 12-13,
wherein each oligonucleotide barcode of the first and second pluralities of oligonucleotide barcodes comprises a linker functional group,
wherein the synthetic particle comprises a solid support functional group, and
wherein the support functional group and the linker functional group are associated with each other, and optionally the linker functional group and the support functional group are individually selected from the group consisting of C6, biotin, streptavidin, primary amine(s), aldehyde(s), ketone(s), and any combination thereof.

15. The kit of any one of claims 12-14,
wherein each of the first plurality of oligonucleotide barcodes comprises a linker functional group,
wherein the synthetic particle comprises a solid support functional group, and
wherein the support functional group and the linker functional group are associated with each other; and optionally the linker functional group and the support functional group are individually selected from the group consisting of C6, biotin, streptavidin, primary amine(s), aldehyde(s), ketone(s), and any combination thereof.
